(19) Europäisches Patentamt
European Patent Office
Office européen des brevets

(11) **EP 4 610 249 A1**

(12) **EUROPEAN PATENT APPLICATION**
published in accordance with Art. 153(4) EPC

(43) Date of publication:
**03.09.2025 Bulletin 2025/36**

(21) Application number: **24784859.1**

(22) Date of filing: **01.04.2024**

(51) International Patent Classification (IPC):
***C07C 235/78*** *(2006.01)* ***C07D 251/34*** *(2006.01)*
***C07D 403/14*** *(2006.01)* ***C08F 2/50*** *(2006.01)*
***C08F 4/00*** *(2006.01)* ***C08F 20/60*** *(2006.01)*
***C08G 65/10*** *(2006.01)* ***C09D 7/63*** *(2018.01)*
***C09D 11/38*** *(2014.01)* ***C09D 201/00*** *(2006.01)*
***C09J 11/06*** *(2006.01)* ***C09J 201/00*** *(2006.01)*
***G03F 7/031*** *(2006.01)*

(52) Cooperative Patent Classification (CPC):
**C07C 235/78; C07D 251/34; C07D 403/14; C08F 2/50; C08F 4/00; C08F 20/60; C08G 65/10; C09D 7/63; C09D 11/38; C09D 201/00; C09J 11/06; C09J 201/00; G03F 7/031**

(86) International application number:
**PCT/JP2024/013398**

(87) International publication number:
**WO 2024/210074 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.04.2023 JP 2023061790**
**05.04.2023 JP 2023061794**

(71) Applicant: **KJ Chemicals Corporation**
**Tokyo 103-0023 (JP)**

(72) Inventors:
- **TAKEDA, Yoshimi**
**Yatsushiro-shi Kumamoto 866-0081 (JP)**
- **TAKEKAWA, Yurie**
**Yatsushiro-shi Kumamoto 866-0081 (JP)**
- **GOTO, Teruya**
**Yatsushiro-shi Kumamoto 866-0081 (JP)**
- **KIYOSADA, Toshitsugu**
**Yatsushiro-shi Kumamoto 866-0081 (JP)**

(74) Representative: **ABG Intellectual Property Law, S.L.**
**Avenida de Burgos, 16D**
**Edificio Euromor**
**28036 Madrid (ES)**

(54) **BENZOYLFORMIC ACID AMIDE DERIVATIVE**

(57) [Problem] The present invention addresses the problem of providing a benzoylformic acid amide derivative. The benzoylformic acid amide derivative has good photopolymerization initiation properties and photosensitization effect with respect to long-wavelength ultraviolet light, and a cured product obtained from a curable composition that contains the benzoylformic acid amide derivative has an extremely low content of a low molecular weight component, and has high yellowing resistance, durability and safety.

[Solution] The present invention provides a benzoylformic acid amide derivative.

EP 4 610 249 A1

## Description

### Technical Field

The present invention relates to a novel benzoylformic acid amide derivative. The benzoylformic acid amide derivative can be used as a photopolymerization initiator and a photosensitizer. The present invention further relates to an active energy ray-curable composition, an active energy ray-curable ink composition, an ink-jet ink, a three-dimensional modeling ink, a pressure-sensitive adhesive composition, an adhesive composition, a sealant composition, a photosensitive composition, a nail cosmetic composition, a dental material composition, a coating agent composition, and an aqueous composition each containing the benzoylformic acid amide derivative.

### Background Art

Photopolymerization and photocuring with active energy rays including ultraviolet light (UV) generally mean that photopolymerization initiator-containing compositions are irradiated with UV to generate active species such as radicals or ions, resulting in the occurrence of polymerization reaction to solidify (cure) liquid compositions for a short time. Such a technique is currently utilized in a variety of fields and used for paint, coating agents, pressure-sensitive adhesives, adhesives, elastomer-based materials, ink-jet inks, sealing materials, sealants, dental health materials, and optical materials. From the viewpoint that curing is possible in any location and shape, utilization for nail cosmetics such as gel nails and utilization for three-dimensional photomodeling materials in 3D printers are expanded.

Photopolymerization initiators which generate radicals by active energy rays can be classified into intramolecular cleavage-type photopolymerization initiators and hydrogen abstraction-type photopolymerization initiators. The former type is to generate radicals by intramolecular cleavage and the latter type is to generate radicals by abstraction of hydrogen from hydrogen donors. Intramolecular cleavage-type photopolymerization initiators cause initiator-derived decomposed products remaining in cured products, and therefore cause the problems of deterioration in durability, odor generation, coloring over time and the like in such cured products and have the problem of low safety. Hydrogen abstraction-type photopolymerization initiators, although most thereof are low in efficiency of photopolymerization initiation, have attracted rising attention in recent years because initiator-derived decomposed products are not caused.

Long-wavelength UV-LED lamps and LED lamps are becoming more widely used due to their high safety. While photopolymerization initiators and photosensitizers to be applied to these light sources have been actively developed, problems are that the photopolymerization initiation effect and the photosensitive effect are low and cured products obtained are easily yellowed.

### Summary of Invention

### Technical Problem

A first object of the present invention is to provide a benzoylformic acid amide derivative. A second object thereof is to provide a benzoylformic acid amide derivative as a highly safe photopolymerization initiator, which is high in photopolymerization initiation ability with active energy rays, in particular, light beams at 360 to 420 nm radiated from an LED lamp and which allows no decomposed product of the benzoylformic acid amide derivative to be present in the resulting cured product. A third object thereof is to provide a highly curable, active energy ray-curable composition containing the benzoylformic acid amide derivative as a photopolymerization initiator. A fourth object thereof is to provide highly safe ink composition, ink-jet ink composition, three-dimensional modeling ink composition, pressure-sensitive adhesive composition, adhesive composition, sealant composition, photosensitive composition, nail cosmetic composition, dental material composition, coating agent composition, aqueous composition, hydrogel composition, and intraocular implant material composition each containing the benzoylformic acid amide derivative, each being high in compatibility, each being excellent in adhesion with a substrate, and each providing a cured product which less causes yellowing over time and bleed-out.

A fifth object thereof is to provide a benzoylformic acid amide derivative as a photosensitizer, which has photosensitivity to active energy rays, in particular, light beams at 360 to 420 nm radiated from an LED lamp and which provides a cured product not yellowed. A sixth object thereof is to provide a highly curable and highly compatible, active energy ray-curable composition containing the benzoylformic acid amide derivative as a photosensitizer. A seventh object thereof is to provide an ink composition, an ink-jet ink composition, a three-dimensional modeling ink composition, a pressure-sensitive adhesive composition, an adhesive composition, a sealant composition, a photosensitive composition, a nail cosmetic composition, a dental material composition, a coating agent composition, an aqueous composition, a hydrogel composition, and an intraocular implant material composition each containing the benzoylformic acid amide derivative and each being excellent in adhesion with a substrate, and each providing a cured product which less causes

yellowing over time and thus is excellent in durability.

Solution to Problem

The present inventors have made intensive studies, and as a result, have found a benzoylformic acid amide derivative having a benzoylformic acid amide group represented by general formula (1) and thus have led to the present invention.

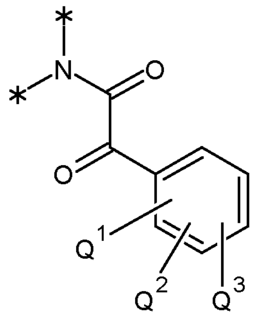

General formula (1)

$Q^1$ to $Q^3$ independently of each other represent a hydrogen atom, a substituent represented by formulas (Formula 2) to (Formula 8), a halogen group, or a nitrile group, and are each bound to any position of the 2-position to the 6-position,

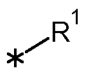

(Formula 2)

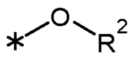

(Formula 3)

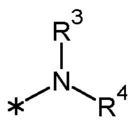

(Formula 4)

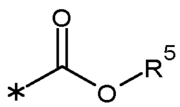

(Formula 5)

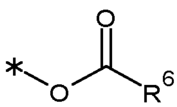

(Formula 6)

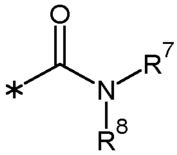

(Formula 7)

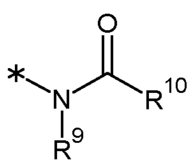

(Formula 8)

$R^1$ to $R^{10}$ each independently represent a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3

to 18 carbon atoms, a cyclic alkyl group having 3 to 18 carbon atoms, or a cyclic alkenyl group having 3 to 18 carbon atoms, and

* is a binding position.

Advantageous effects of Invention

The benzoylformic acid amide derivative of the present disclosure is high in initiation efficiency (also referred to as "polymerization initiation ability" or "photoinitiation ability".) with long-wavelength light beams at 360 to 420 nm, and light beams at wavelengths typified by, for example, 365 nm, 385 nm, 395 nm and 405 nm radiated from an LED lamp, is high in activity of a radical generated at the same time, and can be used as a photopolymerization initiator. An active energy ray-curable composition containing the benzoylformic acid amide derivative as a photopolymerization initiator, even when not used in combination with any additive such as a hydrogen donor as a co-initiator, a general-purpose photosensitizer, or a curing promoter, can easily provide a cured product completely cured at low energy (i.e. low cumulative amount of light) and a high speed (i.e. short curing time) even in an industrial production environment under an air atmosphere. The resulting cured product does not have any decomposed product of the benzoylformic acid amide derivative used as a photopolymerization initiator, and less causes odor, yellowing over time and bleed-out and is high in durability and safety. The benzoylformic acid amide derivative can be suitably used in a variety of applications such as an active energy ray-curable ink composition, an ink-jet ink composition, a three-dimensional modeling ink composition, a pressure-sensitive adhesive composition, an adhesive composition, a sealant composition, a photosensitive composition, a nail cosmetic composition, a dental material composition, a coating agent composition, an aqueous composition, a hydrogel composition, and an intraocular implant material composition.

The benzoylformic acid amide derivative of the present disclosure has a photosensitive effect to other general-purpose photoradical polymerization initiator and photoionic polymerization initiator when absorbs long-wavelength light beams at 360 to 420 nm, or light beams at 365 nm, 385 nm, 395 nm and 405 nm radiated from an LED lamp, and can be used as a photosensitizer hardly causing the occurrence of yellowing during photocuring. An active energy ray-curable composition containing the benzoylformic acid amide derivative as a photosensitizer can be used in combination with a photopolymerization initiator inferior in curability with long-wavelength light beams at 360 to 420 nm, and light beams at 365 nm, 385 nm, 395 nm and 405 nm radiated from an LED lamp, to easily provide a cured product completely cured at low energy (low cumulative amount of light) and a high speed (short curing time) even in an industrial production environment under an air atmosphere. The resulting cured product less causes odor, yellowing over time and bleed-out, and is high in both durability and safety. The benzoylformic acid amide derivative can be suitably used in a variety of applications such as an active energy ray-curable ink composition, an ink-jet ink composition, a three-dimensional modeling ink composition, a pressure-sensitive adhesive composition, an adhesive composition, a sealant composition, a photosensitive composition, a nail cosmetic composition, a dental material composition, a coating agent composition, an aqueous composition, a hydrogel composition, and an intraocular implant material composition.

Description of Embodiments

Hereinafter, embodiments of the present disclosure are described in detail, but the scope of the present invention is not limited to embodiments described here, and various modifications can be made without departing from the gist of the present invention. In a case where a plurality of upper limit values and a plurality of lower limit values are described with respect to a specified parameter, any upper limit value and lower limit value, among these upper limit values and lower limit values, can be combined to provide a suitable numerical value range.

One embodiment of the present disclosure relates to a benzoylformic acid amide derivative (D) having one or more benzoylformic acid amide groups represented by general formula (1) in a molecule.

$Q^1$ to $Q^3$ in the general formula (1) independently of each other represent a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, an alkoxy group having a cyclic alkenyl group having 3 to 18 carbon atoms, an amino group, an alkylamino group, a dialkylamino group, an alkoxycarbonyl group, an alkyl ester group, an aminocarbonyl group, an alkylaminocarbonyl group, a dialkylaminocarbonyl group, an alkylamide group and a halogen group, or a nitrile group. $Q^1$ to $Q^3$ are each bound to any position of the 2-position to the 6-position of a benzene ring. In a case where $Q^1$ to $Q^3$ are each a hydrogen atom, the benzoylformic acid amide derivative (D) exhibits favorable photopolymerization initiation ability and photosensitivity with respect to a high-pressure mercury lamp, and an UV-LED light source at 360 nm to 410 nm, and is less colored by light irradiation. In a case where $Q^1$ to $Q^3$ are each an electron-donating alkyl group, alkoxy group, amino group, alkylamino group, dialkylamino group, alkyl ester group, or alkylamide group, the benzoylformic acid amide derivative (D) is high in sensitivity also to a light source at 390 nm to 420 nm, due to shift of the absorption wavelength to a longer wavelength, and therefore is more suitably used as a photopolymerization initiator and as a photosensitizer. Such an electron-donating

substituent may cause coloration due to light irradiation, and $Q^1$ to $Q^3$ are each particularly preferably an alkoxy group or an alkyl ester group from the viewpoint of enabling coloration of D to be kept at a practical level.

The benzoylformic acid amide group of the benzoylformic acid amide derivative (D) is a monosubstituted amide group or a disubstituted amide group of benzoylformic acid. Both such benzoylformic acid monosubstituted-amide group and benzoylformic acid disubstituted-amide group have photopolymerization initiation ability and photosensitivity, and photopolymerization initiation ability is higher in the benzoylformic acid monosubstituted-amide group. The benzoylformic acid monosubstituted-amide group, in which a hydrogen atom is bound to a nitrogen atom, serves as not only a hydrogen abstraction-type photoinitiating functional group, but also a hydrogen donating group, and efficiently generates an active radical by intramolecular and/or intermolecular hydrogen abstraction. Therefore, even when not used in combination with an amine hydrogen donor or the like easily causing coloration, D having the benzoylformic acid monosubstituted-amide group is high in photopolymerization initiation ability and has polymerization initiation ability with highly safe ultraviolet light at 360 to 420 nm.

The benzoylformic acid amide derivative (D) of the present disclosure is preferably at least one compound represented by any one general formula of general formula (2) to general formula (4).

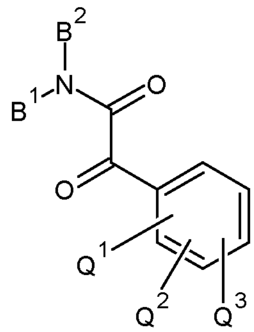

General formula (2)

wherein $Q^1$ to $Q^3$ have the same meanings as the definitions described in the general formula (1),

$B^1$ represents a hydrogen atom, or a monovalent organic group optionally having a hydroxyl group, an amino group, a thiol group, an ether group, a thioether group, an ester group, a carbonate group, a urethane group, a thiourethane group, a urea group, a siloxane group, an amide group, an imide group, an ethylenically unsaturated group or a benzoylformic acid amide group, and

$B^2$ represents a monovalent organic group optionally having a hydroxyl group, an amino group, a thiol group, an ether group, a thioether group, an ester group, a carbonate group, a urethane group, a thiourethane group, a urea group, a siloxane group, an amide group, an imide group, an ethylenically unsaturated group or a benzoylformic acid amide group.

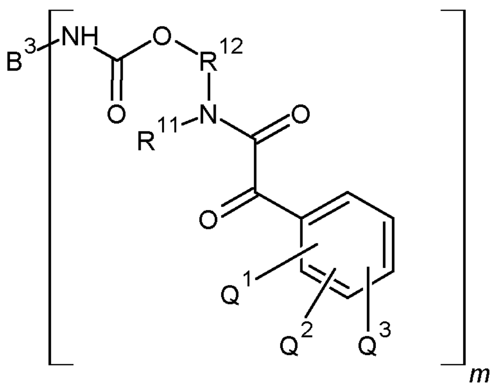

General formula (3)

wherein $Q^1$ to $Q^3$ have the same meanings as the definitions described in the general formula (1),

$B^3$ represents an m-valent organic group optionally having an ethylenically unsaturated group, an ether group, a thioether group, an ester group, a carbonate group, a urethane group, a thiourethane group, an isocyanurate group, an allophanate group, a urea group, a siloxane group, an amide group or an imide group,

$R^{11}$ represents a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon

atoms, a cyclic alkyl group having 3 to 18 carbon atoms, a cyclic alkenyl group having 3 to 18 carbon atoms, or an aryl group having 6 to 8 carbon atoms,

$R^{12}$ represents a linear saturated divalent hydrocarbon group having 1 to 18 carbon atoms, a linear unsaturated divalent hydrocarbon group having 2 to 18 carbon atoms, a branched saturated or unsaturated divalent hydrocarbon group having 3 to 18 carbon atoms, an alicyclic saturated or unsaturated divalent hydrocarbon group having 3 to 8 carbon atoms, a divalent aromatic hydrocarbon group having 6 to 8 carbon atoms, or a divalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group, and m represents an integer of 1 to 10.

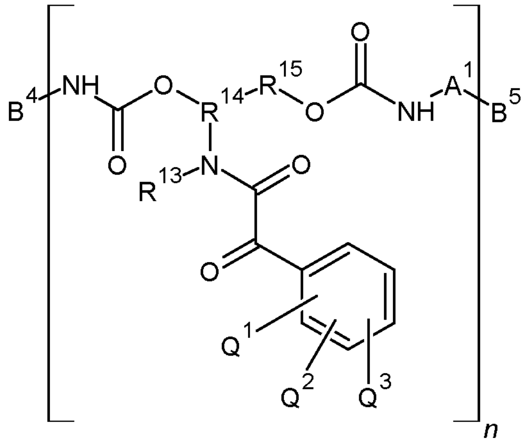

General formula (4)

wherein $Q^1$ to $Q^3$ have the same meanings as the definitions described in the general formula (1),

$A^1$ represents a divalent organic group optionally having an ether group, a thioether group, an ester group, a carbonate group, a urethane group, a thiourethane group, a urea group, a siloxane group, an amide group or an imide group,

$B^4$ and $B^5$ independently of each other optionally have an ethylenically unsaturated group, an ether group, a thioether group, an ester group, a carbonate group, a urethane group, a thiourethane group, an isocyanurate group, an allophanate group, a urea group, a siloxane group, an amide group or an imide group, and any one or both of $B^4$ and $B^5$ represent a monovalent organic group having one or more ethylenically unsaturated bonds,

$R^{13}$ represents a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, a cyclic alkenyl group having 3 to 18 carbon atoms, or an aryl group having 6 to 8 carbon atoms,

$R^{14}$ represents a linear saturated trivalent hydrocarbon group having 1 to 8 carbon atoms, a linear unsaturated trivalent hydrocarbon group having 2 to 8 carbon atoms, a branched saturated or unsaturated trivalent hydrocarbon group having 3 to 8 carbon atoms, an alicyclic saturated or unsaturated trivalent hydrocarbon group having 3 to 8 carbon atoms, a trivalent aromatic hydrocarbon group having 6 to 8 carbon atoms, or a trivalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group,

$R^{15}$ represents a linear saturated divalent hydrocarbon group having 1 to 18 carbon atoms, a linear unsaturated divalent hydrocarbon group having 2 to 18 carbon atoms, a branched saturated or unsaturated divalent hydrocarbon group having 3 to 8 carbon atoms, an alicyclic saturated or unsaturated divalent hydrocarbon group having 3 to 8 carbon atoms, a divalent aromatic hydrocarbon group having 6 to 8 carbon atoms, or a divalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group, and n represents an integer of 1 to 100.

In a case where the benzoylformic acid amide derivative (D) is represented by the general formula (2), the benzoylformic acid amide group has a hydrophobic benzene ring and a hydrophilic formic acid amide group, and is amphiphilic. The polarities of $B^1$ and $B^2$ (which are independent of each other.) are adjusted for any purpose, and thus D is high in compatibility with other component used in a curable composition and the resulting curable composition and a cured product obtained by curing this curable composition are high in transparency. $B^1$ and/or $B^2$ preferably have/has an ether group, a thioether group, an ester group, a carbonate group, a urethane group, a thiourethane group, an isocyanurate group, an allophanate group, a urea group, a siloxane group, an amide group or an imide group because

the polarity is easily adjusted. $B^1$ and $B^2$ each more preferably have an ether group, an ester group, a urethane group, or an amide group. $B^1$ is more preferably a hydrogen atom because D has the benzoylformic acid monosubstituted-amide group and photopolymerization initiation ability is higher.

$B^1$ and $B^2$ may each further have a benzoylformic acid amide group represented by general formula (1). Such a case is preferable because the benzoylformic acid amide derivative (D) has a plurality of benzoylformic acid amide groups and photopolymerization initiation ability and photosensitivity are also higher. Such benzoylformic acid amide groups contained in D may be the same as or different from each other.

The benzoylformic acid amide derivative (D) represented by the general formula (2) can be used as a photosensitizer of photoionic polymerization. Such a case is preferable because, when $B^1$ and $B^2$ each have a cyclic ether group, D is incorporated via a covalent bond into a cured product, by photoionic polymerization. One, or two or more cyclic ether groups may be contained.

$B^1$ and $B^2$ each further preferably have an ethylenically unsaturated group. Such a case is preferable because the benzoylformic acid amide derivative (D) serves as an ethylenically unsaturated group-containing photopolymerization initiator or photosensitizer and D is incorporated via a covalent bond into a cured product, by photoradical polymerization. One or more ethylenically unsaturated groups are preferably contained, and two or more ethylenically unsaturated groups are more preferably contained. One or a plurality of kinds of ethylenically unsaturated groups may be contained.

In a case where $B^1$ and $B^2$ each have a urethane group, the benzoylformic acid amide derivative (D) is favorable in compatibility with other component used in a curable composition, and the resulting curable composition and a cured product thereof are high in transparency. The ratio of the number of urethane groups (total) and the number of benzoylformic acid amide groups (total) in D is preferably 0.1 or more, more preferably 0.5 or more. An increase in number of urethane groups leads to an increase in viscosity of D, and the ratio is preferably 10.0 or less.

The benzoylformic acid amide derivative (D) more preferably has a urethane group represented by general formula (3) or general formula (4). A urethane group which has a hydrogen atom bonding to the nitrogen atom can function as a hydrogen donating group, and D is favorable in photopolymerization initiation ability with highly safe ultraviolet light at 360 to 420 nm.

In a case where benzoylformic acid amide derivative (D) has one or more urethane groups, the number of atoms directly linking a nitrogen atom of the benzoylformic acid amide group and a nitrogen atom of a proximate urethane group is preferably 3 to 20. In a case where the number of atoms directly linking is 3 or more, the benzoylformic acid amide group and a urethane group interact with each other to result in enhancements in both the hydrogen abstraction ability of the benzoylformic acid amide group and the hydrogen donating ability of a urethane group. In a case where the number of atoms directly linking is 20 or less, the benzoylformic acid amide group and a urethane group in a molecule are easily approached to easily develop a hydrogen abstraction reaction. The number of atoms directly linking is more preferably 4 to 10, further preferably 4 to 6 from these viewpoints.

The benzoylformic acid amide derivative (D) represented by the general formula (3) has one or more benzoylformic acid amide groups and one or more urethane groups in its molecule. A urethane group is favorable in compatibility with other component used in a curable composition, and the resulting curable composition and a cured product thereof are high in transparency. $B^3$ preferably further has one or more urethane groups from the viewpoint of enabling this curable composition to be more enhanced in compatibility. The ratio of the number of urethane groups (total) and the number of benzoylformic acid amide groups (total) in D is preferably 0.5 or more, more preferably 2.0 or more. An increase in number of urethane groups leads to an increase in viscosity of D, and the ratio is preferably 10.0 or less, more preferably 6.0 or less, particularly preferably 4.0 or less.

$R^{11}$ in the general formula (3) is a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, a cyclic alkenyl group having 3 to 18 carbon atoms, or an aryl group having 6 to 8 carbon atoms. A case where $R^{11}$ is a hydrogen atom is more preferable because the benzoylformic acid amide derivative (D) has the benzoylformic acid monosubstituted-amide group and is excellent in photopolymerization initiation ability.

$R^{12}$ in the general formula (3) is a linear saturated divalent hydrocarbon group having 1 to 18 carbon atoms, a linear unsaturated divalent hydrocarbon group having 2 to 18 carbon atoms, a branched saturated or unsaturated divalent hydrocarbon group having 3 to 18 carbon atoms, an alicyclic saturated or unsaturated divalent hydrocarbon group having 3 to 8 carbon atoms, a divalent aromatic hydrocarbon group having 6 to 8 carbon atoms, or a divalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group. $R^{12}$ is preferably a linear saturated divalent hydrocarbon group having 1 to 8 carbon atoms, a linear unsaturated divalent hydrocarbon group having 2 to 8 carbon atoms, or a branched saturated or unsaturated divalent hydrocarbon group having 3 to 18 carbon atoms, more preferably a linear saturated divalent hydrocarbon group having 2 to 4 carbon atoms or a branched saturated divalent hydrocarbon group having 3 to 8 carbon atoms.

The benzoylformic acid amide group is amphiphilic and the polarity of $B^3$ is adjusted for any purpose, and thus D is

high in compatibility with other component used in a curable composition and the resulting curable composition and a cured product obtained by curing this curable composition are high in transparency. $B^3$ preferably has an ethylenically unsaturated group, an ether group, a thioether group, an ester group, a carbonate group, a urethane group, a thiourethane group, an isocyanurate group, an allophanate group, a urea group, a siloxane group, an amide group or an imide group because the polarity thereof is easily adjusted.

The benzoylformic acid amide derivative (D) represented by the general formula (3) can also be used as a photosensitizer of photoionic polymerization. Such a case is preferable because, when $B^3$ has one or more cyclic ether groups, D is incorporated via a covalent bond into a cured product, by photoionic polymerization. The cyclic ether group may be one or more.

$B^3$ preferably further has one or more ethylenically unsaturated groups. Such a case is preferable because the benzoylformic acid amide derivative (D) serves as an ethylenically unsaturated group-containing photopolymerization initiator or photosensitizer and D is incorporated via a covalent bond into a cured product, by photoradical polymerization. One or more ethylenically unsaturated groups are preferably contained, and two or more ethylenically unsaturated groups are more preferably contained. One or a plurality of kinds of ethylenically unsaturated groups may be contained.

m in the general formula (3) is an integer of 1 to 10. In a case where m is 1 or more, the benzoylformic acid amide derivative (D) has one or more benzoylformic acid amide groups in its molecule and can function as a photopolymerization initiator and as a photosensitizer. A case where m is more than 10 is not preferable because the molecular weight and the viscosity of D are high and a curable composition containing D can be deteriorated in handleability. m is more preferably an integer of 2 to 4 from these viewpoints.

The benzoylformic acid amide derivative (D) represented by the general formula (3) can be produced by synthesizing benzoylformic acid amide monool by an amidation reaction of benzoylformic acid and an aminoalkyl alcohol and then furthermore subjecting the monool to a urethanization reaction with an isocyanate compound. The aminoalkyl alcohol is preferably 4-aminobenzyl alcohol, 2-(2-aminoethoxy)ethanol, 2-aminoethanol, 2-aminopropanol, 2-amino-2-methyl-1-propanol, 2-amino-2-ethyl-1-propanol, 3-aminopropanol, 2-amino-1-butanol, 3-amino-1-butanol, 4-aminobutanol, 5-aminopentanol, 2-amino-1-hexanol, 6-aminohexanol, 7-aminoheptanol, 2-amino-1-octanol, 8-aminooctanol, 2-amino-1-decanol, 10-aminodecanol, 12-aminododecanol, or 18-aminooctadecanol, more preferably 2-aminoethanol, 2-aminopropanol, 2-amino-2-methyl-1-propanol, 2-amino-2-ethyl-1-propanol, 3-aminopropanol, 2-amino-1-butanol, 3-amino-1-butanol, 4-aminobutanol, 2-amino-1-hexanol, 7-aminoheptanol, 2-amino-1-octanol, 2-amino-1-decanol, 2-amino-1-dodecanol, or 2-amino-1-octadecanol, further preferably 2-aminoethanol, 2-aminopropanol, or 2-amino-2-methyl-1-propanol.

The benzoylformic acid amide derivative (D) represented by the general formula (4) has one or more benzoylformic acid amide groups, two or more urethane groups and one or more ethylenically unsaturated groups in its molecule. A urethane group is favorable in compatibility with other components constituting a curable composition, and a curable composition containing D and a cured product obtained by curing this composition are high in transparency. Any one or more of $A^1$, $B^4$ and $B^5$ preferably further have one or more urethane groups from this viewpoint. The ratio of the number (total) of urethane groups (total) and the number of benzoylformic acid amide groups (total) in D is preferably 2.0 or more, more preferably 2.5 or more. An increase in number of urethane groups leads to an increase in viscosity of D, and therefore the ratio is preferably 15.0 or less, more preferably 8.0 or less, particularly preferably 5.0 or less.

$R^{13}$ in the general formula (4) is a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, a cyclic alkenyl group having 3 to 18 carbon atoms, or an aryl group having 6 to 8 carbon atoms. A case where $R^{13}$ is a hydrogen atom is preferable because the benzoylformic acid amide derivative (D) has the benzoylformic acid monosubstituted-amide group and is excellent in photopolymerization initiation ability.

$R^{14}$ in the general formula (4) is a linear saturated trivalent hydrocarbon group having 1 to 8 carbon atoms, a linear unsaturated trivalent hydrocarbon group having 2 to 8 carbon atoms, a branched saturated or unsaturated trivalent hydrocarbon group having 3 to 8 carbon atoms, an alicyclic saturated or unsaturated trivalent hydrocarbon group having 3 to 8 carbon atoms, a trivalent aromatic hydrocarbon group having 6 to 8 carbon atoms, or a trivalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group. $R^{14}$ is preferably a linear saturated trivalent hydrocarbon group having 1 to 8 carbon atoms, a linear unsaturated trivalent hydrocarbon group having 2 to 8 carbon atoms, a branched saturated or unsaturated trivalent hydrocarbon group having 3 to 8 carbon atoms, or an alicyclic saturated or unsaturated trivalent hydrocarbon group having 3 to 8 carbon atoms, more preferably a linear saturated trivalent hydrocarbon group having 2 to 4 carbon atoms or a branched saturated trivalent hydrocarbon group having 3 to 4 carbon atoms, from the viewpoint that the number of atoms directly linking a nitrogen atom of the benzoylformic acid amide group and a nitrogen atom of a proximate urethane group is preferably 3 to 10.

$R^{15}$ in the general formula (4) represents a linear saturated divalent hydrocarbon group having 1 to 18 carbon atoms, a linear unsaturated divalent hydrocarbon group having 2 to 18 carbon atoms, a branched saturated or unsaturated

divalent hydrocarbon group having 3 to 18 carbon atoms, an alicyclic saturated or unsaturated divalent hydrocarbon group having 3 to 8 carbon atoms, a divalent aromatic hydrocarbon group having 6 to 8 carbon atoms, or a divalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group. $R^{15}$ is preferably an alkylene group having 1 to 18 carbon atoms from the viewpoint that a urethane group to be bound to $A^1$ is easily introduced.

The benzoylformic acid amide group is amphiphilic and the polarity of $A^1$ is adjusted for any purpose, and thus D is high in compatibility with other component used in a curable composition and the resulting curable composition and a cured product obtained by curing this curable composition are high in transparency. $A^1$ preferably has an ether group, a thioether group, an ester group, a carbonate group, a urethane group, a thiourethane group, a urea group, a siloxane group, an amide group or an imide group because the polarity thereof is easily adjusted. Furthermore, $A^1$ preferably has an ether group, a thioether group, an ester group, a carbonate group, or a urethane group because the number of these groups can be easily adjusted and the polarity of $A^1$ can be more easily adjusted.

$B^4$ and $B^5$ independently of each other optionally have an ethylenically unsaturated group, an ether group, a thioether group, an ester group, a carbonate group, a urethane group, a thiourethane group, an isocyanurate group, an allophanate group, a urea group, a siloxane group, an amide group or an imide group, and any one or both of $B^4$ and $B^5$ represent a monovalent organic group having one or more ethylenically unsaturated bonds.

The benzoylformic acid amide derivative (D) represented by the general formula (4) can also be used as a photosensitizer of photoionic polymerization. Such a case is preferable because, when $B^4$ and/or $B^5$ have/has a cyclic ether group, D is incorporated via a covalent bond into a cured product, by photoionic polymerization. The cyclic ether group may be one or more.

Any one or both of $B^4$ and $B^5$ have one or more ethylenically unsaturated bonds, and thus the benzoylformic acid amide derivative (D) is incorporated via a covalent bond into a cured product, by photoradical polymerization. Both $B^4$ and $B^5$ each preferably have an ethylenically unsaturated group from the viewpoint of easy incorporation into a cured product. One or a plurality of kinds of ethylenically unsaturated groups may be contained.

The polarities of $B^4$ and $B^5$ can be adjusted for any purpose. $B^4$ and $B^5$ each preferably have an ether group, a thioether group, an ester group, a carbonate group, a urethane group, a thiourethane group, a urea group, a siloxane group, an amide group or an imide group because the polarities thereof are easily adjusted. Furthermore, $B^4$ and $B^5$ are each preferably an ether group, a thioether group, an ester group, a carbonate group, or a urethane group because the number of these groups can be easily adjusted and the polarities of $B^4$ and $B^5$ can be more easily adjusted.

n in the general formula (4) is an integer of 1 to 100. In a case where n is 1 or more, the benzoylformic acid amide derivative (D) has one or more benzoylformic acid amide groups and can function as a photopolymerization initiator and as a photosensitizer. A case where n is 2 or more is preferable because both photopolymerization initiation ability and photosensitivity are high. A case where n is more than 100 is not preferable because the molecular weight and the viscosity of D are high and a curable composition containing D can be deteriorated in handleability. n is more preferably an integer of 2 to 50, particularly preferably an integer of 2 to 20 from these viewpoints.

The general formula (4) can be produced by synthesizing benzoylformic acid amide diol by an amidation reaction of benzoylformic acid and an aminoalkyl alcohol and then furthermore subjecting the diol to a urethanization reaction with an isocyanate compound. The aminoalkyl diol is preferably 2-aminoethylene glycol, 2-amino-1,3-propanediol, 3-amino-1,2-propanediol, 2-amino-2-methyl-1,3-propanediol, 2-amino-2-ethyl-1,3-propanediol, 2-amino-2-butyl-1,3-propanediol, 2-amino-2-hexyl-1,3-propanediol, 2-amino-2-octyl-1,3-propanediol, 2-amino-2-dodecyl-1,3-propanediol, 2-amino-2-octadecyl-1,3-propanediol, 2-amino-1,4-butanediol, or 2-amino-1,6-hexanediol, more preferably 2-amino-1,3-propanediol, 3-amino-1,2-propanediol, 2-amino-2-methyl-1,3-propanediol, or 2-amino-2-ethyl-1,3-propanediol.

The benzoylformic acid amide derivative (D) is a hydrogen abstraction-type photopolymerization initiator, and a decomposed product is not generated by photopolymerization. Also in a case where the benzoylformic acid amide derivative (D) is used as a photosensitizer, a decomposed product is not generated by photopolymerization. The molecular weight of D is preferably 300 or more, more preferably 500 or more, particularly preferably 1,000 or more. A molecular weight of D of 300 or more is preferable because the volatility of D is low, odor of the resulting cured product is low and bleed-out of D from the cured product is hardly caused. The molecular weight of D is more preferably higher because safety is high, but a molecular weight of more than 200,000 can cause extreme increases in viscosity of D and viscosity of a curable composition containing D, leading to deterioration in handleability. The molecular weight of D is preferably 200,000 or less, more preferably 150,000 or less, particularly preferably 100,000 or less from these viewpoints. In the present invention, a compound having a molecular weight of less than 300 is referred to as "low-molecular-weight component". Since most low-molecular-weight components are high in volatility and low in safety, such a low-molecular-weight component, if present in a cured product, causes the problems of bleed-out from such a cured product over time and thus deterioration in appearance of such a cured product, the occurrence of odor, and the like.

The benzoylformic acid amide derivative (D) can be synthesized by the following method. An amidation reaction of benzoylformic acid or benzoylformic acid ester (hereinafter, collectively defined as "raw material (a1)".) and an amine compound (hereinafter, also referred to as "amino group-containing compound", and defined as "raw material (a2)".) is

allowed to occur, to obtain the benzoylformic acid amide derivative (D) represented by the general formula (2). The raw material (a2) can have a plurality of amino groups and, can further have hydroxyl groups, carboxyl groups, urethane groups, urea groups, or amide groups. a2 preferably has a reactive group such as a hydroxyl group, an amino group, or a carboxyl group. After the amidation reaction of a1 and a2, other reactive groups can be utilized to provide a further reaction with various compounds. The reactive group in a2 is more preferably a hydroxyl group. In a case where a hydroxyl group is contained, a1 and a2 are reacted to produce benzoylformic acid amide and thereafter an etherification reaction, an esterification reaction, and a urethanization reaction can be easily performed with a hydroxyl group. The benzoylformic acid amide derivative (D) represented by the general formula (3) or the general formula (4) can be synthesized by the urethanization reaction using a compound having an ethylenically unsaturated group, a hydroxyl group, an amino group, a carboxyl group, an isocyanate group, or the like as a raw material.

Examples of the benzoylformic acid or benzoylformic acid ester (a1) include benzoylformic acid, benzoylformic acid alkyl (a linear alkyl group having 1 to 18 carbon atoms, a branched alkyl group having 3 to 18 carbon atoms, or a cyclic alkyl group having 3 to 18 carbon atoms) ester, and benzoylformic acid alkenyl (a linear alkenyl group having 2 to 18 carbon atoms, a branched alkenyl group having 3 to 18 carbon atoms, or a cyclic alkenyl group having 3 to 18 carbon atoms) ester. A substituent represented by (Formula 2) to (Formula 8) is bound at any position of the 2-position to the 6-position of a benzene ring of benzoylformic acid, benzoylformic acid alkyl ester, or benzoylformic acid alkenyl ester as a1. Specific examples include methyl benzoylformate, ethyl benzoylformate, methyl 2-methylbenzoylformate, methyl 3-methylbenzoylformate, methyl 4-methylbenzoylformate, ethyl 4-methylbenzoylformate, methyl 4-ethylbenzoylformate, methyl 4-butylbenzoylformate, methyl 4-octylbenzoylformate, methyl 4-dodecylbenzoylformate, methyl 4-octadecylbenzoylformate, methyl 4-ethynylbenzoylformate, methyl 4-ethylbenzoylformate, methyl 2-methoxycarbonylbenzoylformate, methyl 3-methoxycarbonylbenzoylformate, methyl 4-methoxycarbonylbenzoylformate, methyl 3-ethoxycarbonylbenzoylformate, methyl 4-ethoxycarbonylbenzoylformate, methyl 4-butoxycarbonylbenzoylformate, methyl 3,5-dimethoxybenzoylformate, methyl 2,4-dimethoxybenzoylformate, methyl 2,4-diethoxybenzoylformate, methyl 2,4-dibutoxybenzoylformate, methyl 3,4,5-trimethoxybenzoylformate, methyl 4-methoxycarbonylbenzoylformate, methyl 4-acetoxybenzoylformate, methyl 4-dimethylaminobenzoylformate, methyl 2-acetoamidebenzoylformate, methyl 3-chlorobenzoylformate, methyl 4-chlorobenzoylformate, methyl 3-bromobenzoylformate, methyl 4-bromobenzoylformate, ethyl 3-bromobenzoylformate, ethyl 4-bromobenzoylformate, methyl 4-nitrilebenzoylformate, and ethyl 4-nitrilebenzoylformate. Such a1 may be used singly or in combinations of a plurality thereof.

Examples of the amino group-containing compound (a2) include amine compounds such as alkylamine, alkenylamine, dialkylamine, dialkenylamine, alkylalkenylamine, and arylamine, amine compounds each having a hydroxyl group, such as aminoalkyl monool, aminoalkyl diol, aminoalkyl triol, aminoalkyl tetraol, aminoalkyl pentanol, N-alkyl-aminoalkyl monool, N-alkyl-aminoalkyl diol, N-alkyl-aminoalkyl triol, N-alkyl-aminoalkyl tetraol, N-alkyl-aminoalkyl pentanol, N,N-bis(hydroxyalkyl)amine, N,N-bis(dihydroxyalkyl)amine, and hydroxyalkylarylamine, amine compounds each having a thiol group, such as aminoalkylthiol and aminoalkenylthiol, amine compounds each having an ether group, such as (aminoalkoxy)alkanol, dialkylene glycol monoamine, trialkylene glycol monoamine, and polyalkylene glycol monoamine, amine compounds each having a plurality of amino groups, such as alkylenediamine, polyalkyleneimine, dialkylene glycol diamine, trialkylene glycol diamine, polyalkylene glycol diamine, and diaminoalkanol, amino acid compounds, and amine compounds each having a carboxyl group, such as aminobenzoic acid. The alkyl is a linear alkyl group having 1 to 18 carbon atoms, a branched alkyl group having 3 to 18 carbon atoms, or a cyclic alkyl group having 3 to 18 carbon atoms, and the alkenyl is a linear alkylene group having 2 to 18 carbon atoms, a branched alkylene group having 3 to 18 carbon atoms, or a cyclic alkylene group having 3 to 18 carbon atoms. Such an amino group-containing compound may be used singly or in combinations of a plurality thereof.

In a case where the amino group-containing compound (a2) has a hydroxyl group, a carboxyl group, a thiol group, or a plurality of amino groups, the benzoylformic acid amide derivative (D), which has a hydroxyl group, a carboxyl group, a thiol group, or an amino group, can be obtained. D can be further subjected to urethanization, thiourethanization, etherification, esterification, ureation, amidation, or imidization, by use of such a reaction group. Urethanization is a reaction of a hydroxyl group and an isocyanate group, thiourethanization is a reaction of a thiol group and an isocyanate group, etherification is a reaction of a hydroxyl group and organic halogen, esterification is a reaction of a hydroxyl group and a carboxyl group or a reaction of a carboxyl group and an epoxy group, ureation is a reaction of an amino group and an isocyanate group, amidation is a reaction of an amino group and a carboxyl group or a reaction of a carboxyl group and an isocyanate group, and imidization is a reaction of an amino group and a carboxylic anhydride group. These raw materials having such various functional groups can be appropriately selected and combined to synthesize D represented by each of the general formula (3) and the general formula (4).

The amidation reaction of the benzoylformic acid or benzoylformic acid ester (a1) and the amino group-containing compound (a2) is preferably performed under a condition where light is shielded. Specifically, the reaction is performed, for example, under light shielding, under an environment where ultraviolet light is cut, for example, in a yellow room, under a fluorescent lamp where no ultraviolet light is radiated, or under a red safelight for a darkroom. The reaction can progress under mild conditions of ordinary pressure and 100°C or less. A solvent (c) may be used in the reaction.

Examples of the solvent (c) include general-purpose solvents such as toluene, xylene, acetone, methyl ethyl ketone, methyl isobutyl ketone, cyclohexanone, tetrahydrofuran, 1,4-dioxane, chloroform, 1,2-dichloroethane, ethyl acetate, butyl acetate, N,N-dimethylformamide, 3-methoxy-N,N-dimethylpropanamide, 3-butoxy-N,N-dimethylpropanamide, N,N-dimethylpropanamide, dimethylacetamide, dimethylsulfoxide, 2-pyrrolidone, N-methyl-2-pyrrolidone, and 1,3-dimethyl-2-imidazolidinone. A polymerizable (radical-type by light or heat, or cationic or anionic polymerization) compound which is a liquid at a reaction temperature, and which does not react with any raw materials and product can also be used as the solvent (c). Examples of the polymerizable compound solvent include N-(meth)acryloylmorpholine, a (meth)acrylic acid ester having a linear alkyl group or alkoxy group having 1 to 18 carbon atoms, or a branched or cyclic alkyl group or alkoxy group having 3 to 18 carbon atoms, N-substituted (meth)acrylamide, and N,N-disubstituted (meth)acrylamide.

In a case where the benzoylformic acid amide derivative (D) is synthesized by the amidation reaction of the benzoylformic acid ester (a1) and the amino group-containing compound (a2), alcohol is produced as a by-product and D including alcohol is obtained as a crude product. In a case where the solvent (c) is used, D including alcohol and c is obtained as a crude product. Such a crude product can be directly used in a curable composition, or can be used in a curable composition after removal of alcohol and c. In a case where the resulting D has a hydroxyl group, a carboxyl group, a thiol group, or an amino group, such a group can be utilized to synthesize new D. In such a case, a reaction can be made while alcohol and c are contained, or a reaction can be made after removal of alcohol and c. Examples of the method for removal of alcohol and c include a method involving distillation under ordinary pressure or reduced pressure, a method involving bubbling with an inert gas such as dry air or nitrogen, and a freeze-drying method.

The benzoylformic acid amide derivative (D) having a hydroxyl group can be reacted with an isocyanate compound, thereby allowing a urethane group to be introduced into a molecule of D. D can be reacted with an isocyanate compound having an ethylenically unsaturated group, thereby allowing for introduction of an ethylenically unsaturated group. D can be reacted with a polyisocyanate compound and a compound having an ethylenically unsaturated group and a hydroxyl group, thereby allowing for introduction of a urethane group and an ethylenically unsaturated group. D can be reacted with a polyol via a polyisocyanate. A polyol having an ether group, a thioether group, an ester group, a carbonate group, a siloxane group, an amide group, or an imide group can be used to easily introduce such a functional group into D. Furthermore, D can be reacted with a polyisocyanate and a compound having a cyclic ether group and a hydroxyl group, thereby allowing for introduction of a cyclic ether group into D. In a case where D having an ethylenically unsaturated group and/or a cyclic ether group is used as a photopolymerization initiator and a photosensitizer, for a curable composition, D is incorporated into a cured product of photoradical polymerization and/or photoionic polymerization, via a chemical bond. In this case, even a molecular weight of D of less than 300 does not cause the occurrence of bleed-out from such a cured product and D is suitably used as a photopolymerization initiator and as a photosensitizer, in each application.

The ethylenically unsaturated group of the benzoylformic acid amide derivative (D) is one or more groups selected from a (meth)acrylate group, a (meth)acrylamide group, a vinyl group, a vinyl ether group, an alkyl vinyl ether group, an allyl group, a (meth)allyl ether group, a styryl group and a maleimide group. A (meth)acrylate group and a (meth) acrylamide group are preferable from the viewpoint of high polymerizability, an acrylate group and an acrylamide group are more preferable from the viewpoint of high active energy ray-curability, and an acrylamide group is particularly preferable from the viewpoint that a hydrogen bond, in addition to a covalent bond, can be formed in a molecule and between molecules. Furthermore, in a case where D is used as a photopolymerization initiator, an N-monosubstituted acrylamide group is most preferable from the viewpoint of a hydrogen donor. In a case where D is used as a photosensitizer, an acrylate group and an N,N-disubstituted acrylamide group are most preferable from the viewpoint that D and a curable composition containing D are low in viscosity.

Examples of the compound used in the reaction with the benzoylformamide derivative (D) having a hydroxyl group include a compound having two or more isocyanate groups (b1), a compound having one or more hydroxyl groups (b2), a compound having one or more ethylenically unsaturated groups and one or more reactive groups (b3), and a compound having one or more cyclic ether groups and one or more reactive groups (b4). Examples of the reactive groups of b3 and b4 include a hydroxyl group, acid halide, halogen, an isocyanate group, an acid anhydride group, and an epoxy group. b1 encompasses a general-purpose polyisocyanate, a polyisocyanate having a polyol backbone, and a polyisocyanate having an isocyanurate ring.

The isocyanate compound (b1) is a compound having two or more isocyanate groups in its molecule. Specific examples include aliphatic polyisocyanates such as trimethylene diisocyanate, tetramethylene diisocyanate, pentamethylene diisocyanate, hexamethylene diisocyanate, 1,2-propylene diisocyanate, 1,2-butylene diisocyanate, 2,3-butylene diisocyanate, 1,3-butylene diisocyanate, 2,4,4-trimethylhexamethylene diisocyanate, and 2,2,4-trimethylhexamethylene diisocyanate, aromatic polyisocyanates such as 1,3-phenylene diisocyanate, 1,4-phenylene diisocyanate, 2,4-tolylene diisocyanate, 2,6-tolylene diisocyanate, 4,4'-diphenylmethane diisocyanate, 2,4-diphenylmethane diisocyanate, 1,3-xylylene diisocyanate, and 1,4-xylylene diisocyanate, alicyclic polyisocyanates such as cyclopentylene diisocyanate, cyclohexylene diisocyanate, isophorone diisocyanate, 4,4'-dicyclohexylmethane diisocyanate, 1,3-hydrogenated xylylene diisocyanate, 1,4-hydrogenated xylylene diisocyanate, 2,5-norbornane diisocyanate, and 2,6-norbomane diisocyanate, or multimers such as adduct forms, isocyanurate forms or buret forms of such polyisocyanates. Such b1 may

be used singly or in combinations of a plurality thereof.

The compound (b2) having a hydroxyl group is an alcohol compound or a polyol compound. Examples of the alcohol compound include monoalcohols with a linear alkyl group having 1 to 18 carbon atoms, or a branched or cyclic alkyl group having 3 to 18 carbon atoms, such as methanol, ethanol, isopropanol, octanol, and isostearyl alcohol, alkylene glycol compounds with a linear chain having 2 to 18 carbon atoms, a branched chain having 3 to 18 carbon atoms, or a cyclic ring having 3 to 18 carbon atoms, such as ethylene glycol and 1,2-propylene glycol, and polyhydric alcohol compounds such as glycerin, trimethylolpropane, pentaerythritol, and dipentaerythritol.

Examples of the polyol compound of b2 include a polyether polyol, a polyester polyol, a polycarbonate polyol, carbinol-modified silicone, and a polyolefin polyol. Examples of the polyether polyol include a linear polyalkylene glycol having 2 to 18 carbon atoms, a branched polyalkylene glycol having 3 to 18 carbon atoms, and a cyclic polyalkylene glycol having 3 to 18 carbon atoms, and examples of the polyolefin polyol include a hydrogenated polyalkadiene polyol and a polyalkadiene polyol. Such b2 may be used singly or in combinations of a plurality thereof.

**In** the compound (b3) having an ethylenically unsaturated group and a reactive group, in a case where the reactive group is an acid anhydride group or an acid chloride group, examples of b3 include (meth)acrylic acid chloride, (meth)acrylic anhydride, maleic anhydride, and itaconic anhydride. **In** a case where the reactive group is an epoxy group, examples of b3 include (meth)acrylic acid glycidyl ether and 4-hydroxybutyl (meth)acrylate glycidyl ether. **In** a case where the reactive group is an isocyanate group, examples of b3 include 2-(meth)acryloyloxyethyl isocyanate. In a case where the reactive group is a hydroxyl group, examples of b3 include hydroxyalkyl (meth)acrylate, N-hydroxyalkyl(meth) acrylamide, N-alkyl-N-hydroxyalkyl(meth)acrylamide, hydroxyalkyl (meth)vinyl ether, hydroxyalkyl (meth)allyl ether, hydroxyalkylmaleimide, hydroxyalkylstyrene, polyalkylene glycol mono(meth)acrylate, N-polyalkylene glycol mono(meth)acrylamide, N-alkyl-N-polyalkylene glycol mono(meth)acrylamide, N,N-bis(polyalkylene glycol) (meth)acrylamide, polyalkylene glycol mono(meth)vinyl ether, polyalkylene glycol mono(meth)allyl ether, polyalkylene glycol monomaleimide, hydroxyphenyl (meth)acrylate, hydroxyphenyl(meth)acrylamide, alkenyl alcohol, glycerin mono(meth)acrylate, glycerin mono(meth)acrylamide, glycerin di(meth)acrylate, trimethylolpropane di(meth)acrylate, pentaerythritol tri(meth)acrylate, dipentaerythritol penta(meth)acrylate, glycerin mono(meth)acrylamide, trimethylolpropane di(meth)acrylamide, pentaerythritol tri(meth)acrylamide, and dipentaerythritol penta(meth)acrylamide. The alkyl is a linear alkyl group having 1 to 18 carbon atoms, a branched alkyl group having 3 to 18 carbon atoms, or a cyclic alkyl group having 3 to 18 carbon atoms, the alkylene is an alkylene group having 1 to 9 carbon atoms, and the alkenyl is a linear alkenyl group having 2 to 18 carbon atoms, a branched alkenyl group having 3 to 18 carbon atoms, or a cyclic alkenyl group having 3 to 18 carbon atoms. Such b3 may be used singly or in combinations of a plurality thereof.

In the compound (b4) having a cyclic ether group and a reactive group, in a case where the reactive group is halogen, examples of b4 include epichlorohydrin. In a case where the reactive group is a hydroxyl group, b4 is not particularly limited as long as it is a compound having one or more cyclic ether groups and one or more hydroxyl groups. Examples include hydroxyalkyl glycidyl ether and hydroxyalkyl epoxide each having a linear alkyl group having 1 to 18 carbon atoms, a branched alkyl group having 3 to 18 carbon atoms, or a cyclic alkyl group having 3 to 18 carbon atoms, and 7-oxabicyclo[4.1.0]heptane-3-methanol as a compound containing an alicyclic epoxy group and a hydroxyl group. Such b4 may be used singly or in combinations of a plurality thereof.

The method for introducing a urethane group (urethanization) is not particularly limited as long as it is a known method. The reaction temperature is preferably in the range from room temperature to 90°C. If necessary, the solvent (c), a urethanization catalyst, and/or any other additive may be used. A polymerizable compound can also be used as a solvent instead of c. Examples of such a polymerizable compound solvent include N-(meth)acryloylmorpholine, alkyl (meth) acrylic acid ester, alkenyl(meth)acrylic acid ester, aryl(meth)acrylic acid ester, alkylene di(meth)acrylic acid ester, dialkylene glycol di(meth)acrylic acid ester, trialkylene glycol di(meth)acrylic acid ester, polyalkylene glycol di(meth) acrylic acid ester, N-substituted (meth)acrylamide, and N,N-disubstituted (meth)acrylamide. The alkyl is a linear alkyl group having 1 to 18 carbon atoms, a branched alkyl group having 3 to 18 carbon atoms, or a cyclic alkyl group having 3 to 18 carbon atoms, the alkenyl is a linear alkenyl group having 2 to 18 carbon atoms or a cyclic alkenyl group having 3 to 18 carbon atoms, and the aryl is an aryl group having 6 to 8 carbon atoms. The urethanization reaction is preferably performed under an environment where light is shielded. Specifically, the reaction is performed, for example, under light shielding, under an environment where ultraviolet light is cut, for example, in a yellow room, under a fluorescent lamp where no ultraviolet light is radiated, or under a red safelight for a darkroom.

After the urethanization reaction, a crude product D containing the solvent (c) or the polymerizable compound instead of c is obtained, the crude product can be directly used in a curable composition, or can be used in a curable composition after removal of c or the polymerizable compound. Examples of the method for removal of c include a method involving distillation under ordinary pressure or reduced pressure, a method involving bubbling with an inert gas such as dry air or nitrogen, and a freeze-drying method.

Examples of the reaction catalyst used in the urethanization reaction include a quaternary ammonium salt, a tertiary phosphine derivative, a tertiary amine derivative, and an organometallic compound. Examples of the quaternary ammonium salt include tetrabutylammonium bromide, triethylbenzylammonium chloride, tetrabutylphosphonium bromide, and tetraphenylphosphonium bromide. Examples of the tertiary phosphine include triarylphosphines such as triphenylphosphine, tribenzylphosphine, and tritolylphosphine, tricycloalkylphosphines such as tricyclohexylphosphine, and trialkylphosphines such as triethylphosphine, tripropylphosphine, tributylphosphine, and trioctylphosphine. Examples of the tertiary amine include trialkylamines such as triethylamine and tributylamine, dialkylarylamines such as dimethylbenzylamine and diethylbenzylamine, and triethanolamines. Examples of the organometallic compound include a metal salt of metal such as zinc, tin, lead, zirconium, bismuth, cobalt, manganese, or iron and an organic acid such as octenoic acid or naphthenic acid, a metal chelate compound of dibutyltin dilaurate, dioctyltin dilaurate, tin 2-ethylhexanoate, dibutyltin diacetylacetonate, zirconium tetraacetylacetonate, titanium acetylacetonate, aluminum acetylacetone, cobalt acetylacetone, iron acetylacetone, copper acetylacetone, or zinc acetylacetone, a potassium or sodium salt of alkylphosphonic acid, and a sodium or potassium salt of a fatty acid having 8 to 20 carbon atoms. These may be used singly or in combinations of a plurality thereof. Among them, a quaternary ammonium salt, a tertiary phosphine derivative, and tin-based, bismuth-based, zirconium-based, and iron-based organometallic compounds which are high in catalyst effect are more preferable.

The amount of the urethanization reaction catalyst used is preferably 0.001 to 10% by mass based on the total mass of raw materials. An amount of 0.001% by mass or more can allow for rapid progression of the reaction. A case of 10% by mass or less allows for low coloration by the catalyst. The amount is still more preferably 0.01 to 1.00% by mass.

The benzoylformic acid amide derivative (D) of the present disclosure generates a radical as growth active species, by active energy ray irradiation. Examples of the active energy ray include photoenergy rays such as visible light, electron beam, ultraviolet light, infrared light, X-ray, $\alpha$-ray, $\beta$-ray, and $\gamma$-ray. Among them, ultraviolet light is preferably used in terms of the balance among an active energy ray generator, the rate of photopolymerization initiation, and safety. Examples of an ultraviolet light source include a xenon lamp, a low-pressure mercury lamp, a high-pressure mercury lamp, a metal halide lamp, an UV-LED lamp, and a microwave excimer lamp. An UV-LED lamp is preferable which can radiate highly safe ultraviolet light at 360 to 420 nm at a high output. An LED lamp is suitably used which can radiate light beams at 365 nm, 385 nm, 395 nm, and 405 nm.

The irradiation energy necessary for radical generation of the benzoylformic acid amide derivative (D) of the present disclosure can be expressed by the cumulative amount of light. The cumulative amount of light is preferably in the range of 5 to 50,000 mJ/cm$^2$, more preferably in the range of 10 to 20,000 mJ/cm$^2$. When the irradiation energy is in this range, a sufficient number of growth active species can be generated from the photopolymerization initiator.

The benzoylformic acid amide derivative (D) of the present disclosure can be contained as a photopolymerization initiator in an active energy ray-curable composition and thus used in various applications. The content of D in the curable composition differs depending on the structure of D and the compositional ratio of the curable composition, and is preferably 0.1% by mass or more. When 0.1% by mass or more of D is contained, photopolymerization can be immediately initiated by active energy ray irradiation and the curable composition can be sufficiently cured. In a case where D does not contain any ethylenically unsaturated group, the content of D in the curable composition, while differs depending on the structure and the molecular weight of D, is preferably 50% by mass or less. The content of D is more preferably 0.5 to 20% by mass, particularly preferably 1 to 10% by mass relative to the entire curable composition from the viewpoint that the balance between the curability (curing rate) of the curable composition and physical properties of the resulting cured product is easily adjusted. In a case where D contains an ethylenically unsaturated group, the cured product can be formed singly from D and thus 100% by mass of D can be contained. D can be used in combination with, as any other polymerizable compound (h), a compound having one ethylenically unsaturated group in its molecule (hereinafter, referred to as "monofunctionally unsaturated compound (h1)".) and/or a compound having two or more ethylenically unsaturated groups in its molecule (hereinafter, referred to as "polyfunctionally unsaturated compound (h2)".), from the viewpoint that a curable composition can be sufficiently cured and physical properties of the resulting cured product are favorable. **In** such a case, the content of D is more preferably 0.5 to 90% by mass, particularly preferably 1 to 70% by mass relative to the entire curable composition.

The benzoylformic acid amide derivative (D) of the present disclosure can be contained as a photosensitizer in an active energy ray-curable composition. The content of D in the curable composition differs depending on the structure of D and the compositional ratio of the curable composition, and is preferably 0.1% by mass or more. **In** a case where 0.1% by mass or more of D is contained, D is excited by active energy ray irradiation, to activate the photopolymerization initiator in the curable composition, and enable photopolymerization to be immediately initiated or enable the curable composition to be sufficiently cured. D exhibits photosensitivity to not only a photoradical polymerization initiator, but also a photoionic polymerization initiator (photocationic polymerization or photoanionic polymerization), and thus can be used in combination with such a photopolymerization initiator. **In** a case where D does not contain any ethylenically unsaturated group or cyclic ether group, the content of D in the curable composition, while differs depending on the structure and the molecular weight of D, is preferably 30% by mass or less. The content of D is more preferably 0.5 to 20% by mass, particularly preferably 1 to 10% by mass relative to the entire curable composition from the viewpoint that the balance between the curability of the curable composition and physical properties of the resulting cured product is easily adjusted. **In** a case where D contains an ethylenically unsaturated group and/or a cyclic ether group, the cured product can be formed singly

from D and thus 100% by mass of D can be contained. D can be used in combination with, as any other polymerizable compound (h), a compound having a cyclic ether group in its molecule (hereinafter, referred to as "cyclic ether-containing compound (h3)".), from the viewpoint that the curable composition can be sufficiently cured and physical properties of the resulting cured product are favorable. In such a case, the content of D is more preferably 0.5 to 90% by mass, particularly preferably 1 to 70% by mass relative to the entire curable composition.

The benzoylformic acid amide derivative (D) of the present disclosure can be used singly as each of a photopolymerization initiator and a photosensitizer, or can be appropriately combined with D different in structure therefrom as a photopolymerization initiator or as a photosensitizer. In a case where D is used as a photopolymerization initiator and also as a photosensitizer, the content of D in the curable composition, in total, is 0.5 to 80% by mass, preferably 1 to 75% by mass, more preferably 2 to 50% by mass, particularly preferably 3 to 30% by mass.

The polymerizable compound (h) includes a monofunctionally unsaturated compound (h1), a polyfunctionally unsaturated compound (h2) and a cyclic ether-containing compound (h3) other than D. The content of h is 0 to 99.9% by mass relative to the entire curable composition. The content of h is preferably 10 to 99.5% by mass, more preferably 30 to 99% by mass from the viewpoint that physical properties of a cured product can be suitably adjusted.

Examples of the monofunctionally unsaturated compound (h1) include each compound containing a (meth) acrylate group, a (meth)acrylamide group, a vinyl group, an allyl group, a styryl group and an acetylene group. Such a group may be used singly or in combinations of two or more kinds thereof. The content of h1 is preferably 0 to 90% by mass, more preferably 5 to 70% by mass, particularly preferably 10 to 50% by mass relative to the entire curable composition. h1 is usually low in viscosity, and can be contained and thus expected to provide the effects of a reduction in viscosity and an improvement in handleability of the curable composition.

Examples of the monofunctionally unsaturated compound (h1) containing a (meth)acrylate group include an alkyl (meth)acrylate compound, a hydroxyalkyl (meth)acrylate compound, (meth)acrylic acid/alkyl carboxylic acid compounds, (meth)acrylic acid/alkylsulfonic acid compounds, (meth)acrylic acid/alkylphosphoric acid compounds, an alkyloxy (hereinafter, also referred to as "alkoxy".) alkylene glycol (meth)acrylate compound, an alkoxydialkylene glycol (meth)acrylate compound, an alkoxytrialkylene glycol (meth)acrylate compound, an alkoxypolyalkylene glycol (meth) acrylate compound, a phenoxyalkylene glycol (meth)acrylate compound, a phenoxydialkylene glycol (meth)acrylate compound, a phenoxytrialkylene glycol (meth)acrylate compound, phenoxypolyalkylene glycol (meth)acrylate, an N-alkylamino(meth)acrylate compound, an N-alkylaminoalkyl (meth)acrylate compound, an N,N-dialkylaminoalkyl (meth) acrylate compound, and a (meth)acrylate compound with a cyclic structure introduced, such as benzyl (meth)acrylate, phenoxyethyl (meth)acrylate, dicyclopentanyl (meth)acrylate, dicyclopentanyloxyethyl (meth)acrylate, dicyclopentenyl (meth)acrylate, dicyclopentenyloxyethyl (meth)acrylate, bornyl (meth)acrylate, isobornyl (meth)acrylate, tetrahydrofurfuryl (meth)acrylate, 2-methyl-2-adamantyl (meth)acrylate, and N-(meth)acryloyloxyethyl norbornenecarboxamide. The alkyl is a linear alkyl group having 1 to 18 carbon atoms, a branched alkyl group having 3 to 18 carbon atoms, or a cyclic alkyl group having 3 to 18 carbon atoms, and the alkylene is an alkylene group having 1 to 4 carbon atoms.

Examples of the monofunctionally unsaturated compound (h1) containing a (meth)acrylamide group include (meth)acrylamide, mono- or disubstituted (meth)acrylamide, N-(meth)acryloylmorpholine, and diacetone (meth)acrylamide. Examples of the mono- or disubstituted (meth)acrylamide include N-alkyl(meth)acrylamide, N,N-dialkyl(meth) acrylamide, N-hydroxyalkyl(meth)acrylamide, N,N-di(hydroxyalkyl)(meth)acrylamide, N-hydroxyalkyl-N-(4-hydroxyphenyl)(meth)acrylamide, N-alkyl-N-hydroxyalkyl(meth)acrylamide, N-alkyl-N-(4-hydroxyphenyl)(meth)acrylamide, 4-hydroxyphenyl(meth)acrylamide, N,N-di(4-hydroxyphenyl)(meth)acrylamide, N-alkoxyalkyl(meth)acrylamide, N,N-di(alkoxyalkyl)(meth)acrylamide, N-alkyl-N-alkoxyalkyl(meth)acrylamide, N-sulfoalkylacrylamide, N-alkylamino(meth)acrylamide, N-alkylaminoalkyl(meth)acrylamide, and N,N-dialkylaminoalkyl(meth)acrylamide. The alkyl is a linear alkyl group having 1 to 18 carbon atoms, a branched alkyl group having 3 to 18 carbon atoms, or a cyclic alkyl group having 3 to 18 carbon atoms.

Examples of the monofunctionally unsaturated compound (h1) containing a vinyl group include carboxylic acid vinyl ester having a carboxyl group having 1 to 18 carbon atoms, alkyl vinyl ether, vinyl chloride, N-vinylpyrrolidone, N-vinylcaprolactam, N-vinyloxazoline, maleic acid, maleic anhydride, fumaric acid, itaconic acid, itaconic anhydride, maleic acid monoalkyl ester, maleic acid dialkyl ester, maleic acid monoalkylamide, maleic acid dialkylamide, maleic acid alkylimide, fumaric acid monoalkyl ester, fumaric acid dialkyl ester, fumaric acid monoalkylamide, fumaric acid dialkylamide, itaconic acid monoalkyl ester, itaconic acid dialkyl ester, itaconic acid monoalkylamide, itaconic acid dialkylamide, itaconic acid alkylimide, vinyl carboxylic acid, vinylsulfonic acid, and vinylphosphoric acid. The alkyl is a linear alkyl group having 1 to 18 carbon atoms, a branched alkyl group having 3 to 18 carbon atoms, or a cyclic alkyl group having 3 to 18 carbon atoms.

Examples of the monofunctionally unsaturated compound (h1) containing an allyl group include carboxylic acid allyl ester having a carboxyl group having 1 to 18 carbon atoms, an alkyl allyl ether compound, phenyl allyl ether, alkyl phenyl allyl ether, allylamine, and mono- or dialkylallylamine. The alkyl is a linear alkyl group having 1 to 18 carbon atoms, a branched alkyl group having 3 to 18 carbon atoms, or a cyclic alkyl group having 3 to 18 carbon atoms.

Examples of the monofunctionally unsaturated compound (h1) containing a styryl group include styrene, α-alkylstyrene, an α-methylstyrene dimer, o-alkylstyrene, m-alkylstyrene, p-alkylstyrene, and p-styrenesulfonic acid. The alkyl is a linear alkyl group having 1 to 18 carbon atoms, a branched alkyl group having 3 to 18 carbon atoms, or a cyclic alkyl group having 3 to 18 carbon atoms.

Examples of the polyfunctionally unsaturated compound (h2) include each compound containing two or more of unsaturated groups such as a (meth)acrylate group, a (meth)acrylamide group, a vinyl group, an allyl group, a styrene group and an acetylene group. A compound containing one kind of such unsaturated groups may be adopted, or a compound containing two or more kinds of such unsaturated groups may be adopted. It is more preferable for obtaining favorable curability to contain a (meth)acrylate group or (meth)acrylamide group having one or more unsaturated groups. The content of h2 is preferably 0 to 99% by mass, more preferably 1 to 70% by mass, particularly preferably 5 to 50% by mass relative to the entire curable composition. h2 can be contained and thus expected to allow the resulting cured product to be high in strength and hardness and excellent in durability.

Examples of the polyfunctionally unsaturated compound (h2) include allyl (meth)acrylate, allyloxyalkoxy (meth) acrylate, allyl(meth)acrylamide, allyloxyalkoxy(meth)acrylamide, vinyloxyalkoxy (meth)acrylate, diallylamine, alkyldiallylamine, a dialkyldiallylammonium quaternary salt, an alkylene glycol di(meth)acrylate compound, a polyalkylene glycol di(meth)acrylate compound, a bisphenol A diglycidyl ether (meth)acrylic acid adduct compound, an alkoxylated bisphenol A di(meth)acrylate compound, a polyester di(meth)acrylate compound, a polycarbonate di(meth)acrylate compound, a polyurethane di(meth)acrylate compound, a polyurethane di(meth)acrylamide compound, pentaerythritol tri(meth)acrylate, pentaerythritol tetra(meth)acrylate, trimethylolpropane tri(meth)acrylate, dipentaerythritol tri(meth)acrylate, ditrimethylolpropane tetra(meth)acrylate, dipentaerythritol tetra(meth)acrylate, dipentaerythritol penta(meth)acrylate, dipentaerythritol hexa(meth)acrylate, tri(meth)acryloyloxyethoxytrimethylolpropane, glycerin polyglycidyl ether poly(meth) acrylate, isocyanuric acid ethylene oxide-modified tri(meth)acrylate, ethylene oxide-modified dipentaerythritol penta(meth)acrylate, ethylene oxide-modified dipentaerythritol hexa(meth)acrylate, ethylene oxide-modified pentaerythritol tri(meth)acrylate, ethylene oxide-modified pentaerythritol tetra(meth)acrylate, and succinic acid -modified pentaerythritol tri(meth)acrylate. The alkyl is a linear alkyl group having 1 to 18 carbon atoms, a branched alkyl group having 3 to 18 carbon atoms, or a cyclic alkyl group having 3 to 18 carbon atoms, and the alkylene is an alkylene group having 1 to 4 carbon atoms.

The number average molecular weight of the polyfunctionally unsaturated compound (h2) is preferably 100 to 50,000. A case of a molecular weight of 100 or more is preferable because the resulting cured product is low in curing shrinkage. A case of a molecular weight of 50,000 or less is preferable because the curable composition is low in viscosity and excellent in handleability. The molecular weight of h2 is more preferably 200 to 20,000, particularly preferably 300 to 15,000 from these viewpoints.

The cyclic ether-containing compound (h3) is a compound having one or more cyclic ether groups in its molecule, and such a cyclic ether group in h3 encompasses an epoxy group, a glycidyl group and an oxetane group. **In** a case where a plurality of such cyclic ether groups is contained, only one kind of such cyclic ether groups may be contained or two or more kinds thereof may be contained in combination. Examples of h3, which is a compound having one cyclic ether group, include alkyl glycidyl ether, alkyl epoxide, aryl glycidyl ether, epoxycycloalkane, alkyl oxetane, glycidyl (meth)acrylate, 4-hydroxybutyl (meth)acrylate glycidyl ether, and vinyl glycidyl ether. Examples of h3, which is a compound having a plurality of cyclic ether groups, include alkylene glycol diglycidyl ether, aryl diglycidyl ether, trimethylolpropane triglycidyl ether, (3,4-epoxycyclohexylmethyl)3,4-epoxycyclohexanecarboxylate, and alkylenebisoxetane. The alkyl is a linear alkyl group having 1 to 18 carbon atoms, a branched alkyl group having 3 to 18 carbon atoms, or a cyclic alkyl group having 3 to 18 carbon atoms, the alkylene is a linear alkylene group having 1 to 18 carbon atoms, a branched alkylene group having 3 to 18 carbon atoms, or a cyclic alkylene group having 3 to 18 carbon atoms, and the aryl is an aryl group having 6 to 18 carbon atoms. Such h3 may be used singly or in combinations of a plurality thereof.

The content of the cyclic ether-containing compound (h3) is preferably 0 to 99% by mass, more preferably 5 to 90% by mass, particularly preferably 10 to 50% by mass relative to the entire curable composition. h3 is usually low in viscosity, and can be contained and thus expected to provide the effects of a reduction in viscosity and an improvement in handleability of the curable composition.

The benzoylformic acid amide derivative (D) of the present disclosure has high photopolymerization initiation ability in photoradical polymerization, and can be suitably used as a photopolymerization initiator in various applications. **In** a case where higher photopolymerization initiation ability is demanded, D and any other photopolymerization initiator can be used in combination. Such a photopolymerization initiator usable in combination is not particularly limited, and examples thereof include benzoin compounds such as benzoin and benzoin alkyl ether compounds, acetophenone compounds such as 2-hydroxy-2-methyl-1-phenylpropan-1-one, acylphosphine oxide compounds such as 2,4,6-trimethylbenzoyl diphenylphosphine oxide, benzoylformic acid ester compounds such as methyl benzoylformate, aminoacetophenone compounds such as 2-methyl-2-morpholino(4-thiomethylphenyl)propan-1-one, and oxime ester compounds such as 1-(9,9-dimethyl-9H-fluoren-2-yl)-1,2-propanedione 2-(O-acetoxime). Such a photopolymerization initiator used in combination can be, if necessary, used in combination with D at any ratio, and may be used singly or in combinations of a plurality thereof.

The benzoylformic acid amide derivative (D) can be used in a hybrid polymerization system of photoradical polymerization and thermal radical polymerization. A thermal polymerization initiator usable in combination with D is not particularly limited, and examples thereof include ketone peroxides such as methyl ethyl ketone peroxide, peroxyketals such as 1,1-di(t-hexylperoxy)-3,3,5-trimethylcyclohexane, 1,1-di(t-hexylperoxy)cyclohexane, and 1.1-di(t-butylperoxy) cyclohexane, hydroperoxides such as 1,1,3,3-tetramethylbutyl hydroperoxide, cumene hydroperoxide, and p-menthane hydroperoxide, dialkyl peroxides such as dicumyl peroxide and di-t-butyl peroxide, diacyl peroxides such as dilauroyl peroxide and dibenzoyl peroxide, peroxydicarbonates such as di(4-t-butylcyclohexyl)peroxydicarbonate and di(2-ethylhexyl)peroxydicarbonate, peroxyesters such as t-butylperoxy-2-ethylhexanoate, t-hexylperoxyisopropyl monocarbonate, t-butylperoxybenzoate, and 1,1,3,3-tetramethylbutylperoxy-2-ethylhexanoate, bis(1-phenyl-1-methylethyl)peroxide, azo initiators such as 2,2'-azobis(isobutyronitrile), 2,2'-azobis(2-methylbutyronitrile), 2,2'-azobis(isobutyric acid) dimethyl, and 1,1'-azobis(cyclohexane-1-carbonitrile), and polymeric azo polymerization initiators such as a polydimethylsiloxane unit-containing polymeric azo polymerization initiator (VPS-1001N manufactured by FUJIFILM Wako Pure Chemical Corporation) and a polyethylene glycol unit-containing polymeric azo polymerization initiator (VPE-0201 manufactured by FUJIFILM Wako Pure Chemical Corporation). Such a thermal polymerization initiator can be, if necessary, used in combination with D at any ratio, and may be used singly or in combinations of a plurality thereof.

The benzoylformic acid amide derivative (D) has a sufficient photosensitive effect on photoradical polymerization, and can be suitably used as a photosensitizer of photoradical polymerization, in various applications. In a case where a further photosensitive effect is demanded, use in combination with any other photosensitizer can be made. A photosensitizer usable in combination with D is not particularly limited, and examples thereof include a benzophenone compound, an unsaturated ketone compound typified by an anthracene derivative, benzil, a 1,2-diketone derivative typified by camphorquinone, a benzoin derivative, an anthraquinone derivative, a thioxanthone derivative, a coumarin derivative, a thiol compound, and a disulfide compound. Such a photosensitizer can be, if necessary, used in combination with D at any ratio, and may be used singly or in combinations of a plurality thereof.

The benzoylformic acid amide derivative (D) can be suitably used as a photosensitizer of photoionic polymerization, in various applications. In a case where D is used in photoionic polymerization, a photoionic polymerization initiator is not particularly limited, and examples thereof include photoanionic polymerization initiators such as 2-(9-oxoxanthen-2-yl)propionic acid 1,5,7-triazabicyclo[4.4.0]deca-5-ene and 1,2-dicyclohexyl-4,4,5,5-tetramethylbiguanidium N-butyltriphenylborate, and antimony-based and triarylsulfonium salt-based photocationic polymerization initiators. In a photoanionic polymerization system or a photocationic polymerization system, a polymerization initiator for such each polymerization system may be used singly or in combinations of a plurality thereof.

The benzoylformic acid amide derivative (D) has a sufficient photosensitive effect also on photoionic polymerization, and can be used singly as a photosensitizer of photoionic polymerization. In a case where a further photosensitive effect is demanded, use in combination with any other photosensitizer of photoionic polymerization can be made. Such a photoionic polymerization initiator usable in combination with D is not particularly limited, and is suitably used similarly as a photosensitizer of photoionic polymerization as long as it is a photosensitizer usable in photoradical polymerization. Such any other photosensitizer can be, if necessary, used in combination with D at any ratio, and may be used singly or in combinations of a plurality thereof.

The curable composition may further contain an organic solvent and water depending on the usage methods and the objects of the curable composition and the resulting cured product. **In** such a case, the polymerization reaction (curing) may be performed after removal of an organic solvent and water in advance, or the polymerization reaction may be performed with an organic solvent and water being contained and such organic solvent and water may be removed after curing. The content of such organic solvent and water is not particularly limited, and is preferably 80% by mass or less, more preferably 50% by mass or less relative to the entire curable composition from the viewpoints of energy saving and high efficiency.

Examples of the organic solvent used in the curable composition include alcohol compounds such as methanol, ethanol, and isopropanol, ketone compounds such as acetone, methyl ethyl ketone, methyl isobutyl ketone, and cyclohexanone, ester compounds such as ethyl acetate, propyl acetate, butyl acetate, methyl lactate, and ethyl lactate, alkylene glycol compounds such as ethylene glycol and propylene glycol, polyalkylene glycol compounds such as polyethylene glycol and polypropylene glycol, glycol ether compounds such as ethoxy diethylene glycol and methoxy propylene glycol, glycol ester compounds such as propylene glycol acetate, ether compounds such as tetrahydrofuran, methyl tetrahydrofuran, cyclopentyl methyl ether, methyl tetrahydropyran, and methyl-tert-butyl ether toluene, aromatic hydrocarbon compounds such as xylene, aliphatic hydrocarbon compounds such as hexane and cyclohexane, amide compounds such as N,N-dimethylformamide, dimethylacetamide, and N,N-dimethylpropionamide, amide ether compounds such as β-methoxy-N,N-dimethylpropionamide and 3-butoxy-N,N-dimethylpropionamide, pyrrolidone compounds such as 2-pyrrolidone and N-methylpyrrolidone, piperidine compounds such as N-methylpiperidine, halogenated hydrocarbon compounds such as methylene chloride, chloroform, and dichloroethane, sulfoxide compounds such as dimethylsulfoxide, and imidazolidinone compounds such as 1,3-dimethyl-2-imidazolidinone. Such an organic solvent may be used singly or in combinations of a plurality thereof.

The benzoylformic acid amide derivative (D) of the present disclosure can be suitably used in, for example, an UV flexographic ink, an UV offset ink, an UV screen ink, an UV ink-jet ink, an active energy ray-curable nail cosmetic composition (gel nail), an UV curing pressure-sensitive adhesive, an UV curing adhesive, a material for sealing or an active energy ray-curable sealant composition used for materials for sealing or sealants, an active energy ray-curable coat agent used for paint or coating agents for automobiles, electrical appliances, furniture, or the like, an active energy ray-curable decorative sheet resin composition used for decorative sheets used in surface coat or the like of automobiles or electrical appliances, a functional member having self-repairing properties, such as a coat agent, a solid-modelled product, a nail decoration material, an automobile exterior protective or a decorative film, an active energy ray-curable self-repairing-material resin composition used for devices or the like, an active energy ray-curable elastomer composition for elastomers used for transparent pressure-sensitive adhesive sheets, cushioning materials, packings, vibration isolating materials, acoustic absorption materials, printing plates, sealing materials, polishing agents, or the like, an active energy ray-curable three-dimensional modeling ink composition used for model materials or support materials for 3D printers, an active energy ray-curable vehicular coating agent composition such as paint for automobiles, an active energy ray-curable composition used in fields of various coatings such as ship bottom paint, an anti-fogging material, and antifouling paint, an active energy ray-curable composition used in the medical device surface coating field, an active energy ray-curable dental material composition, an active energy ray-curing photosensitive composition, an active energy ray-curable hydrogel composition, and an active energy ray-curable intraocular implant material composition. The resulting hydrogel composition can also be suitably used as a material for a wide variety of fields, for example, the health field of high water-absorption resins, paper diapers, soft contact lenses, and the like, the medical field of artificial organs and the like, the civil engineering and construction field of soil amendments and the like, the agricultural field of water retention agents and the like, and the energy-absorbing material field.

Examples

Hereinafter, the present invention is described in more detail with reference to Examples, but these Examples are merely illustrative for suitably describing the present invention, and are not intended to limit the present invention at all. Hereinafter, all of "part(s)" and "%" are on mass basis, unless particularly noted.

Hereinafter, there are described apparatuses and analysis conditions used with respect to analysis methods used in Examples and Comparative Examples of the present disclosure.

(1) Fourier transform infrared spectroscopy (FT-IR analysis)

FT-IR analysis was performed with the following apparatus.
Nicolet iS50 (manufactured by Thermo Fisher Scientific)

(2) Liquid chromatography mass spectrometric method (LC-MS analysis)

Conditions of LC-MS analysis are as described below.
Column: XBride C18, 4.6 mm-150 mm, 3.5 μm (manufactured by Waters Corporation)
Eluent conditions: water/methanol/aqueous 1% formic acid solution = 60/30/10
Measurement wavelength: 258 nm
Column oven: 40°C

(3) Nuclear magnetic resonance spectroscopy ($^1$H-NMR analysis)

$^1$H-NMR analysis was performed with a 400-MHz apparatus manufactured by JEOL Ltd., and the resonant frequency of the methyl group of tetramethylsilane was defined as 0.0 ppm.

(4) Gel permeation chromatography analysis (GPC analysis)

Conditions of GPC analysis are as described below.
Apparatus: Prominence-I LC-2030C (manufactured by Shimadzu Corporation)
Guard column: Shodex KF-G one column (manufactured by Showa Denko K.K.)
Column: Shodex KF-803 one column (manufactured by Showa Denko K.K.)
Column temperature: 40°C
Mobile phase: tetrahydrofuran (THF)
Rate of liquid sent: 0.5 mL/min
Reference sample: polystyrene

(5) High-performance liquid chromatography analysis (HPLC analysis)

Conditions of HPLC analysis are as described below.
Apparatus: Prominence-I LC-2030C (manufactured by Shimadzu Corporation)
Column: Mightysil RP-18GP, 4.6 mm-250 mm, 5 μm (manufactured by Kanto Kagaku)
Eluent conditions: methanol/aqueous 10 mM phosphoric acid solution = 50/50
Measurement wavelength: 258 nm
Column oven: 40°C

Various raw materials and solvents used in Examples and Comparative Examples are shown below.

(1) Benzoylformic acid compound (a1)

a1-1: methyl benzoylformate
a1-2: methyl 3,4,5-trimethoxybenzoylformate
a1-3: methyl 4-methoxycarbonylbenzoylformate
a1-4: methyl 4-acetoxybenzoylformate
a1-5: methyl 4-dimethylaminobenzoylformate
a1-6: ethyl 4-methylbenzoylformate
a1-7: methyl 2-acetoaminobenzoylformate
a1-8: methyl 4-methoxybenzoylformate
a1-9: methyl 3,5-dimethoxybenzoylformate
a1-10: methyl 3-methoxybenzoylformate
a1-11: ethyl 3-butoxybenzoylformate
a1-12: methyl 4-bromobenzoylformate

(2) Amino group-containing compound (a2)

a2-1: (2S,3S,4R)-2-amino-1,3,4-octadecanetriol
a2-2: diethanolamine
a2-3: 3-piperidinemethanol
a2-4: dimethylamine (11% methanol solution, 2.0 mol/L)
a2-5: 8-amino-1-octanol
a2-6: aminoethanol
a2-7: 2-amino-2-methyl-1-propanol
a2-8: 1,3-diamino-2-propanol
a2-9: DL-2-amino-1-butanol
a2-10: 4-aminobenzyl alcohol
a2-11: 2-(2-aminoethoxy)ethanol
a2-12: trimethylolaminomethane
a2-13: D-glucamine
a2-14: 3-amino-1,2-propanediol
a2-15: 2-amino-1,3-propanediol
a2-16: 2-amino-2-ethyl-1,3-propanediol

(3) Isocyanate compound (b1)

b1-1: isophorone diisocyanate
b1-2: trimethylhexamethylene diisocyanate
b1-3: 1,3,5-tris[(5-isocyanate-1,3,3-trimethylcyclohexyl)methyl]-1,3,5-triazin-2,4,6-(1H,3H,5H)-trione (isocyanurate form of isophorone diisocyanate)
b1-4: 1,3,5-tris(6-isocyanate hex-1-yl)-1,3,5-triazin-2,4,6 (1H,3H,5H)-trione (isocyanurate form of hexamethylene diisocyanate)
b1-5: hexamethylene diisocyanate
b1-6: methylenebisphenyl isocyanate
b1-7: pentamethylene diisocyanate (Stabio PDI, manufactured by Mitsui Chemicals, Inc.)
b1-8: dicyclohexylmethane-4,4-diisocyanate

(4) Compound (b2) having hydroxyl group

b2-1: polyethylene glycol (number average molecular weight 300)
b2-2: hydrogenated poly1,2-butadiene with hydroxy group on both terminals (number average molecular weight 1,000) (GI-1000, manufactured by Nippon Soda Co., Ltd.)
b2-3: ETERNACOLL UH-50 (number average molecular weight 500) (manufactured by UBE Corporation)
b2-4: silicone with both terminals modified by hydroxyl groups (number average molecular weight 1,700) (Shin-Etsu Silicone KF-6001, manufactured by Shin-Etsu Chemical Co., Ltd.)
b2-5: 1,4-butanediol
b2-6: isopropanol
b2-7: isostearyl alcohol
b2-8: ADEKA POLYETHER BPX-2000 (manufactured by ADEKA Corporation)
b2-9: Kuraray Polyol P-1010 (number average molecular weight 1,000) (manufactured by Kuraray Co., Ltd.)
b2-10: Uniol TG330 (polyoxypropylene glyceryl ether, molecular weight 330, manufactured by NOF Corporation)
b2-11: polypropylene glycol (number average molecular weight 1,000)
b2-12: Kuraray Polyol P-6010 (number average molecular weight 6,000) (manufactured by Kuraray Co., Ltd.)
b2-13: polytetramethylene glycol (number average molecular weight 650) (BioPTMG650, manufactured by Mitsubishi Chemical Corporation)

(5) Compound (b3) having ethylenically unsaturated group and reactive group

b3-1: 2-acryloyloxyethyl isocyanate
b3-2: N-(2-hydroxyethyl)acrylamide (registered trademark "Kohshylmer", "HEAA" manufactured by KJ Chemicals Corporation)
b3-3: acrylic acid chloride
b3-4: allyl chloride
b3-5: acrylonitrile
b3-6: unsaturated polyester diol (polyester of 1,5-methylpentanediol/maleic acid/adipic acid = 4/1/2 (molar ratio))
b3-7: 2-hydroxyethylmaleimide
b3-8: hydroxyethyl acrylate
b3-9: N-(hydroxymethyl)acrylamide
b3-10: 4-hydroxybutyl acrylate
b3-11: pentaerythritol triacrylate
b3-12: hydroxyethyl methacrylate
b3-13: N-(2-hydroxyethyl)methacrylamide
b3-14: 4-hydroxybutyl vinyl ether
b3-15: oleyl alcohol
b3-16: polypropylene glycol (6) monoacrylate
b3-17: dipentaerythritolpentaacrylate

(6) Compound (b4) having cyclic ether group and reactive group

b4-1: epichlorohydrin
b4-2: 7-oxabicyclo[4.1.0]heptane-3-methanol
b4-3: 2-hydroxyethyl glycidyl ester

(7) Solvent (c)

c-1: toluene
c-2: 1,2-dichloroethane
c-3: ethyl acetate
c-4: 4-methyltetrahydrofuran
c-5: 3-methoxy-N,N-dimethylpropionamide (registered trademark "Kohshylvent", "KJCMPA" Manufactured by KJ Chemicals Corporation)

Monofunctionally unsaturated compound (h1), polyfunctionally unsaturated compound (h2), cyclic ether-containing compound (h3), photopolymerization initiator (E), photosensitizer (I), thermal polymerization initiator (J), and other additive (k) used in active energy ray-curable compositions of Examples and Comparative Examples are shown below.

(8) Polymerizable compound (h)

(8-1) Monofunctionally unsaturated compound (h1)

h1-1: N-acryloylmorpholine (registered trademark "Kohshylmer", "ACMO", manufactured by KJ Chemicals Corporation)
h1-2: isobornyl acrylate
h1-3: N,N-diethylacrylamide (registered trademark "Kohshylmer", "DEAA", manufactured by KJ Chemicals Corporation)
h1-4: 4-hydroxybutyl acrylate
h1-5: methyl 3-acryloylaminopropionate
h1-6: N-acryloyloxyethyl norbornenecarboxamide (registered trademark "Kohshylmer", manufactured by KJ Chemicals Corporation)
h1-7: N-octylacrylamide (registered trademark "Kohshylmer", manufactured by KJ Chemicals Corporation)
h1-8: N-(2-hydroxyethyl)acrylamide (registered trademark "Kohshylmer", "HEAA" Manufactured by KJ Chemicals Corporation)
h1-9: t-butylcyclohexyl acrylate (registered trademark "Kohshylmer", manufactured by KJ Chemicals Corporation)
h1-10: tetrahydrofurfuryl acrylate
h1-11: N-vinylpyrrolidone
h1-12: N-oleylacrylamide (registered trademark "Kohshylmer", manufactured by KJ Chemicals Corporation)
h1-13: diacetone acrylamide (registered trademark "Kohshylmer", manufactured by KJ Chemicals Corporation)
h1-14: N,N-dimethylacrylamide (registered trademark "Kohshylmer", "DMAA", manufactured by KJ Chemicals Corporation)
h1-15: hydroxyethyl methacrylate
h1-16: phenoxyethyl acrylate
h1-17: lauryl acrylate
h1-18: isobornyl methacrylate
h1-19: 2-methacryloyloxyethyl acid phosphate

(8-2) Polyfunctionally unsaturated compound (h2)

h2-1: hexanediol diacrylate
h2-2: dipentaerythritol hexaacrylate
h2-3: Quick Cure 8100 (registered trademark "Quick Cure", manufactured by KJ Chemicals Corporation)
h2-4: Quick Cure 7100 (registered trademark "Quick Cure", manufactured by KJ Chemicals Corporation)
h2-5: polyethylene glycol (14) diacrylate
h2-6: urethane diacrylate (Shikoh UV3000, manufactured by Mitsubishi Chemical Corporation)
h2-7: trimethylolpropane triacrylate
h2-8: pentaerythritol triacrylate
h2-9: Quick Cure 7300 (registered trademark "Quick Cure", manufactured by KJ Chemicals Corporation)
h2-10: dimethylol-tricyclodecane diacrylate
h2-11: urethane diacrylate (Shikoh UV6630, manufactured by Mitsubishi Chemical Corporation)
h2-12: polyethylene glycol (20)-introduced bisphenol A diacrylate h2-13: 2-(2-vinyloxyethoxy)ethyl acrylate
h2-14: diethylene glycol divinyl ether
h2-15: bisphenol A epoxy acrylate oligomer (Miramer PE-210, manufactured by Miwon Specialty Chemical Co., Ltd.)
h2-16: polyethylene glycol (10)-introduced bisphenol A diacrylate
h2-17: 2,4-dimethyl-1,6-hexanediylbis[carbamic acid 2-(methacryloyloxy)ethyl]
h2-18: ethylenebisacrylamide
h2-19: triethylene glycol dimethacrylate

(9) Cyclic ether-containing compound (h3)

h3-1: 2-ethylhexylglycidyl ether
h3-2: bisphenol A diglycidyl ether
h3-3: 1,6-hexanediol diglycidyl ether

h3-4: trimethylolpropane triglycidyl ether
h3-5: butyl glycidyl ether
h3-6: 4-hydroxybutyl acrylate glycidyl ether

(10) Photopolymerization initiator (E)

E-1: oligomer of 2-hydroxy-1-(4-isopropenylphenyl)-2-methylpropan-1-one (ESACURE KIP 150, manufactured by IGM Resin B.V.)
E-2: methyl benzoylformate (Omnirad MBF, manufactured by IGM Resin B.V.)
E-3: 2,4,6-trimethylbenzoyl diphenylphosphine oxide (Omnirad TPO, manufactured by IGM Resin B.V.)
E-4: Kohshylex-I 3003 (registered trademark "Kohshylex", manufactured by KJ Chemicals Corporation)
E-5: 1-hydroxycyclohexyl phenyl ketone (Omnirad 184, manufactured by IGM Resin B.V.)
E-6: 2-hydroxy-2-methylpropiophenone (Omnirad 1173, manufactured by IGM Resin B.V.)
E-7: 1-[4-(2-hydroxyethyl)-phenyl]-2-hydroxy-methylpropanone (Omnirad 2959, manufactured by IGM Resin B.V.)
E-8: benzophenone
E-9: α-[(4-benzoylphenoxy)acetyl]-ω-{[(4-benzoylphenoxy)acetyl]oxy}poly(oxybutan-1,4-diyl) (Omnipol BP, manufactured by IGM Resin B.V.)
E-10: 2-(9-oxoxanthen-2-yl)propionic acid 1,5,7-triazabicyclo[4.4.0]deca-5-ene E-11: 1,2-dicyclohexyl-4,4,5,5-tetramethylbiguanidium N-butyltriphenylborate

(11) Photosensitizer (I)

I-1: poly(ethylene glycol) bis(p-dimethylaminobenzoate) (Omnipol ASA, manufactured by IGM Resin B.V.)
I-2: bis-N,N-[2-(4-dimethylaminobenzoyl)oxyethylen-1-yl]methylamine (Esacure A 198, manufactured by IGM Resin B.V.)
I-3: isopropylthioxanthone
I-4: 2-ethylanthraquinone
I-5: polytetramethylene glycol (3) carboxymethoxythioxanthone diester (Omnipol TX, manufactured by IGM Resin B.V.)

(12) Thermal polymerization initiator (J)
J-1: azobisisobutyronitrile
(13) Other additive (k)

k-1: pentaerythritol tetrakis(3-mercaptobutyrate)
k-2: methyl-5-norbornene-2,3-dicarboxylic anhydride
k-3: BYK JET9151 (pigment dispersant, maleimide-styrene copolymer having ammonium salt structure, manufactured by BYK Chemie)
k-4: carbon black dispersion liquid (manufactured by Mitsubishi Chemical Corporation) k-5: Pigment Yellow 155
k-6: VALIFAST BLUE1613 (manufactured by ORIENT CHEMICAL INDUSTRIES Co., LTD.)
k-7: BYK-331 (leveling agent, polyether-modified polydimethylsiloxane, manufactured by BYK Chemie)
k-8: Petrotack 100V (manufactured by Tosoh Corporation)
k-9: silica with surface modified by trimethylsilyl group (AEROSIL RX200, manufactured by Nippon Aerosil Co., Ltd.)
k-10: hydrogenated rosin (non-polymerizable polymer, Tackifire KE-359, manufactured by Arakawa Chemical Industries, Ltd.)
k-11: Reolosil QS-30 (manufactured by Tokuyama Corporation)
k-12: inorganic filler (titanium oxide)
k-13: methacrylic acid/methyl methacrylate/styrene copolymerized binder
k-14: polyvinyl alcohol JC-25 (manufactured by Japan Vam & Poval Co., Ltd.)
k-15: Eleminol JS-20 (manufactured by Sanyo Chemical Industries, Ltd.)

Example 1 Synthesis of benzoylformic acid amide derivative (D-1)

To a 1,000 mL flask provided with a reflux condenser, a stirrer, a thermometer and a dropping funnel were added 197.0 g (1.20 mol) of methyl benzoylformate (a1-1), 317.5 g (1.00 mol) of (2S,3 S,4R)-2-amino-1,3,4-octadecanetriol (a2-1), 500 g of toluene (c-1) as a solvent, and 1.0 g (0.01 mol) of triethylamine (TEA) as a catalyst, and the temperature

was raised to 70°C with stirring. After a reaction was performed at 70°C for 8 hours, the unreacted raw materials, the solvent, methanol as a by-product, and the catalyst were distilled off under reduced pressure, to obtain benzoylformic acid amide derivative (D-1) as a light yellow solid (yield 85%). Identification of D-1 was performed by $^1$H-NMR analysis. Chemical shift values of representative protons are shown in Table 1-1. The molecular weight of the product was 450 (a molecular ion peak in a mass spectrum was located at 451) according to a liquid chromatography mass spectrometric method (LC-MS analysis). It was confirmed from the results of $^1$H-NMR and LC-MS analysis that the product was benzoylformic acid amide derivative (D-1) shown in Table 1-1.

Examples 2, 3, and 5 to 20 Synthesis of benzoylformic acid amide derivatives (D-2), (D-3), and (D-5) to (D-20)

A reaction was performed in the same conditions as in Example 1 according to raw materials and each loading ratio shown in Table 1-1 and Table 1-2, to obtain each of benzoylformic acid amide derivatives (D-2), (D-3), and (D-5) to (D-20). Identification of the resulting benzoylformic acid amide (D) was performed in the same manner by $^1$H-NMR analysis and LC-MS analysis. Chemical shift values of representative protons, and the molecular weights and the yields of the products are shown in Table 1-1 and Table 1-2. It was confirmed from the results of $^1$H-NMR and LC-MS analysis that the products were benzoylformic acid amide derivatives (D-2), (D-3), and (D-5) to (D-20) shown in Table 1-1 and Table 1-2.

Example 4 Synthesis of benzoylformic acid amide derivative (D-4)

The catalyst in Example 1, TEA, was changed to sodium methoxide, and methyl 4-acetoxybenzoylformate (a1-4) and dimethylamine were reacted and the resultant was purified in the same conditions as in Example 1, to obtain benzoylformic acid amide derivative (D-4) as a light yellow solid (yield 75%). Identification of D-4 was performed by $^1$H-NMR and LC-MS analysis. Each analysis data and the chemical formula of D-4 are shown in Table 1-1.

Example 21 Synthesis of benzoylformic acid amide derivative (D-21)

Added were 243.1 g (1.00 mol) of methyl 4-bromobenzoylformate (a1-12), 143.0 g (1.20 mol) of 2-amino-2-ethyl-1,3-propanediol (a2-16), and 362 g of ADEKA POLYETHER BPX-2000 (b2-8) instead of the solvent (c), and a reaction was performed in the same conditions as in Example 1. Thereafter, a nitrogen gas was allowed to pass at 25°C at a flow rate of 100 $cm^3$/min for 10 minutes (bubbling), to remove unreacted raw materials, and methanol as a by-product, thereby obtaining a b2-8 solution of benzoylformic acid amide derivative (D-21) (yield 84%). Identification of D-21 was performed in the same manner by $^1$H-NMR analysis. Chemical shift values of representative protons are shown in Table 1-2. According to LC-MS analysis, the molecular weight of D-21 was confirmed and also the resulting solution was confirmed to be a mixture of D-21 and b2-8 at a mass ratio of 1/1.

Example 22 Synthesis of benzoylformic acid amide derivative (D-22)

To a 300 mL flask provided with a reflux condenser, a stirrer, a thermometer and a dropping funnel were added 45.7 g of benzoylformic acid amide derivative (D-2) synthesized in Example 2, 54.3 g of 2-acryloyloxyethyl isocyanate (b3-1), and 50 g of ethyl acetate (c-3), and these were mixed. To such a mixed liquid was added 0.02 g of bismuth tris(2-ethylhexanoate) as a catalyst, and a reaction was performed under stirring at 70°C for 4 hours. The disappearance of an isocyanate group was confirmed by FT-IR analysis, and the solvent was removed under reduced pressure, to obtain a light yellow viscous solid product (yield 98%). The presence of a urethane group and a D-2-derived benzoylformic acid amide group (disubstituted) was confirmed by FT-IR analysis of the product. The presence of an acrylate group (5.85 ppm, 6.20 ppm, 6.45 ppm) was confirmed by $^1$H-NMR analysis. Furthermore, it was confirmed by LC-MS analysis that the molecular weight of the product was 520 and the product was benzoylformic acid amide derivative (D-22) shown in Table 2-1. Table 2-1 shows the chemical formula, the molecular weight and the number of benzoylformic acid amide groups per molecule, the number of atoms directly linking a nitrogen atom of the benzoylformic acid amide group and a nitrogen atom of a proximate urethane group, and the ratio between the number of urethane groups and the number of benzoylformic acid amide groups per molecule of D-22.

Example 23 Synthesis of benzoylformic acid amide derivative (D-23)

A reaction of 50.0 g of benzoylformic acid amide derivative (D-3), 32.9 g of isophorone diisocyanate (b1-1) and 17.1 g of N-(2-hydroxyethyl)acrylamide (b3-2) was performed in the presence of 0.01 g of dibutyltin dilaurate as a catalyst in the same manner as in Example 22 except that no reaction solvent was used, and thus a light yellow viscous solid product was obtained (yield 96%). The presence of a urethane group and a D-3-derived benzoylformic acid amide group (disubstituted) was confirmed by FT-IR analysis of the product. The presence of an acrylamide group (5.60 ppm, 6.10 ppm,

6.50 ppm) was confirmed by $^1$H-NMR analysis. Furthermore, it was confirmed by LC-MS analysis that the molecular weight of the product was 675 and the product was benzoylformic acid amide derivative (D-23) shown in Table 2-1. The chemical formula of D-23 and other data are collectively shown in Table 2-1.

Example 24 Synthesis of benzoylformic acid amide derivative (D-24)

To a 300 mL flask provided with a reflux condenser, a stirrer, a thermometer and a dropping funnel were added 74.7 g of benzoylformic acid amide derivative (D-5) synthesized in Example 5 and 100 g of 1,2-dichloroethane (c-2), and these were mixed. Such a mixed liquid was cooled to -10°C, and 25.3 g of acrylic acid chloride (b3-3) was dropped at -10 to 0°C kept, to perform a reaction. Thereafter, extraction was performed with an aqueous alkaline solution and the solvent in an organic layer was removed under reduced pressure, to obtain a light yellow viscous liquid product (yield 84%). The presence of a D-5-derived benzoylformic acid amide group (monosubstituted) was confirmed by FT-IR analysis of the product and the presence of an acrylate group was confirmed by $^1$H-NMR analysis of the product. Furthermore, it was confirmed by LC-MS analysis that the molecular weight of the product was 331. The chemical formula of product D-24 and other data are collectively shown in Table 2-1.

Example 25 Synthesis of benzoylformic acid amide derivative (D-25)

A 500 mL flask provided with a reflux condenser, a stirrer, a thermometer and a dropping funnel was charged with 6.0 g of sodium hydride and 50 g of 4-methyltetrahydrofuran (c-4). In 50 g of c-4 was dissolved 63.6 g of benzoylformic acid amide derivative (D-18), and the solution was placed in a dropping funnel, and dropped over 30 minutes with the amount of hydrogen generated being confirmed. After completion of dropping, 36.4 g of allyl chloride (b3-4) was added, and a reaction was performed at 25°C for 24 hours. Thereafter, 100 mL of ion-exchange water was added to deactivate the remaining sodium hydride, and c-4 was separated, and furthermore extracted with saturated saline three times. The extracted liquid was concentrated by an evaporator and purified by silica gel column chromatography, and furthermore the solvent was removed under reduced pressure, to obtain a light yellow viscous liquid product (yield 43%). The presence of a D-18-derived benzoylformic acid amide group (monosubstituted) was confirmed by FT-IR analysis of the product. The presence of an allyl ether group (5.00 ppm, 5.05 ppm, 5.85 ppm, 3.85 ppm) was confirmed by $^1$H-NMR analysis. It was confirmed by LC-MS analysis that the molecular weight of the product was 347. The chemical formula of D-25 and other data are collectively shown in Table 2-1.

Example 26 Synthesis of benzoylformic acid amide derivative (D-26)

A 500 mL flask provided with a reflux condenser, a stirrer, a thermometer and a dropping funnel was charged with 40.4 g of trifluoromethanesulfonic acid, and 4.8 g of ion-exchange water was added thereto and mixed under cooling, to obtain trifluoromethanesulfonic acid hydrate. A solution of 86.6 g of benzoylformic acid amide derivative (D-9), 13.4 g of acrylonitrile (b3-5) and 100 g of 4-methyltetrahydrofuran (c-4) was dropped at 40°C into the flask over 2 hours. After completion of the reaction, extraction was performed with ion-exchange water twice, the organic layer was concentrated, and purification by silica gel column chromatography was performed. Furthermore, the solvent was removed under reduced pressure, to obtain a light yellow viscous liquid product (yield 38%). The presence of a D-9-derived benzoylformic acid amide group (monosubstituted) was confirmed by FT-IR analysis of the product, and the presence of an acrylamide group and the presence of two benzoylformic acid amide groups were confirmed by $^1$H-NMR analysis of the product. Furthermore, it was confirmed by LC-MS analysis that the molecular weight of the product was 407. The chemical formula of D-26 and other data are collectively shown in Table 2-1.

Examples 27, 28, 30, 32, and 36 to 39 Synthesis of benzoylformic acid amide derivatives (D-27), (D-28), (D-30), (D-32), and (D-36) to (D-39)

Synthesis of benzoylformic acid amide derivatives (D) was performed with raw materials shown in Table 2-1 to Table 2-3 in the same manner as in Example 22. Identification of each product was performed by FT-IR analysis, $^1$H-NMR analysis and LC-MS analysis. The chemical formula of each product and other data are collectively shown in Table 2-1 to Table 2-3. Specifically, the presence of a maleimide group (7.05 ppm), an acrylate group (5.85 ppm, 6.20 ppm, 6.45 ppm), and an acrylamide group (5.60 ppm, 6.10 ppm, 6.50 ppm) was confirmed by $^1$H-NMR analysis.

Example 29 Synthesis of benzoylformic acid amide derivative (D-29)

The same reaction apparatus as in Example 23 was used, 18.8 g of benzoylformic acid amide derivative (D-7), 23.5 g of trimethylhexamethylene diisocyanate (b1-2), 57.7 g of unsaturated polyester diol (b3-6), and 0.05 g of zirconium

tetrakisacetylacetonate as a catalyst were mixed, and a reaction was performed under stirring at 60°C for 5 hours. The disappearance of an isocyanate group was confirmed by FT-IR analysis of a reaction liquid, and thus a light yellow viscous liquid product was obtained (yield 96%). The presence of a urethane group and a benzoylformic acid amide group was confirmed by FT-IR analysis of the product, and the presence of a maleic acid ester group (6.30 ppm) was confirmed by $^1$H-NMR analysis of the product. The number average molecular weight (Mn) was calculated by GPC analysis and thus was 2,700. The chemical formula of benzoylformic acid amide derivative (D-29) and other data, as confirmed from these results, are shown in Table 2-1.

Examples 31, 34, 35, 40 to 46, and 48 to 54 Synthesis of benzoylformic acid amide derivatives (D-31), (D-34), (D-35), (D-40) to (D-46), and (D-48) to (D-54)

Synthesis of benzoylformic acid amide derivatives (D) was performed with raw materials shown in Table 2-2 to Table 2-6 in the same manner as in Example 23. The presence of a urethane group and a benzoylformic acid amide group was confirmed by FT-IR analysis, and the presence of various unsaturated groups, an acrylate group (5.85 ppm, 6.20 ppm, 6.45 ppm), a methacrylate group (5.65 ppm, 6.20 ppm), an acrylamide group (5.60 ppm, 6.10 ppm, 6.50 ppm), a methacrylamide group (5.60 ppm, 6.20 ppm), and a vinyl ether group (4.75 ppm, 4.80 ppm, 6.75 ppm), was confirmed by $^1$H-NMR analysis. Furthermore, the number average molecular weight (Mn) of the product was calculated by GPC analysis. The chemical formulas of benzoylformic acid amide derivatives (D-31), (D-34), (D-35), (D-40) to (D-46), and (D-48) to (D-54) and other data are collectively shown in Table 2-2 to Table 2-6. D-41 was synthesized from biomass diisocyanate (b1-7), and thus the biobased content was 27.0%. D-53 was synthesized from biomass polyol (b2-13), and thus the biobased content was 24.9%. These biobased contents were calculated by a method described in ISO 16620-1.

Example 33 Synthesis of benzoylformic acid amide derivative (D-33)

Raw materials shown in Table 2-2 were used, 39.6 g of benzoylformic acid amide derivative (D-10), 39.8 g of isophorone diisocyanate (b1-1), 20.6 g of N-(hydroxymethyl)acrylamide (b3-9), 100.0 g of a polymerizable compound, N-acryloylmorpholine (h1-1), instead of solvent (c), and 0.02 g of bismuth tris(2-ethylhexanoate) as a catalyst were mixed in the same manner as in Example 22, and a reaction was performed under stirring at 60°C for 6 hours. The disappearance of an isocyanate group was confirmed by FT-IR analysis of a reaction liquid, and thus a light yellow liquid product was obtained (yield 99%). The presence of a urethane group and a benzoylformic acid amide group was confirmed by FT-IR analysis of the product, and the presence of a b3-9-derived acrylamide group (5.60 ppm, 6.10 ppm, 6.50 ppm) was confirmed by $^1$H-NMR analysis of the product. It was also confirmed by LC-MS analysis that the molecular weight of D-33 was 588 and an h1-1 solution of D-33 (50% by mass) was obtained. These analysis results are shown in Table 2-2.

Example 47 Synthesis of benzoylformic acid amide derivative (D-47)

The same reaction apparatus as in Example 23 was used, 19.8 g of a b2-8 solution of benzoylformic acid amide derivative (D-21) (D-21/b2-8 = 1/1, mass ratio), 15.7 g of trimethylhexamethylene diisocyanate (b1-2), 58.8 g of b2-8, 1.7 g of N-(hydroxyethyl)acrylamide (b3-2), 4.0 g of oleyl alcohol (b3-15), and 0.01 g of dibutyltin dilaurate as a catalyst were mixed, and a reaction was performed under stirring at 60°C for 6 hours. The disappearance of an isocyanate group was confirmed by FT-IR analysis of a reaction liquid, and thus a light yellow liquid product was obtained (yield 95%). The presence of a urethane group and a benzoylformic acid amide group was confirmed by FT-IR analysis of the product, and the presence of a b3-2-derived acrylamide group (5.60 ppm, 6.10 ppm, 6.50 ppm), and an unsaturated group (5.35 ppm) of a b3-15-derived oleyl group was confirmed by $^1$H-NMR analysis of the product. Furthermore, the number average molecular weight (Mn) of D-47 was calculated by GPC analysis and thus was 7,200. The chemical formula of D-47 and other data, confirmed from these results, are collectively shown in Table 2-4.

Example 55 Synthesis of benzoylformic acid amide derivative (D-55)

A 300 mL flask provided with a reflux condenser, a stirrer, a thermometer and a dropping funnel was charged with 67.6 g of benzoylformic acid amide derivative (D-6) and 50.0 g of 4-methyltetrahydrofuran (c-4), and mixed, and thereafter 21.6 g of epichlorohydrin (b4-1) was added. After 0.5 g of boron trifluoride diethyl ether was added at 20°C kept, 10.8 g of b4-1 was further added over 1 hour, and a reaction was additionally performed for 2 hours after completion of dropping. The reaction liquid was filtrated, the filtrate was washed with ion-exchange water, and the solvent was removed from the organic layer under reduced pressure, to obtain a light yellow liquid product (yield 65%). The presence of a benzoylformic acid amide group was confirmed by FT-IR analysis of the product, and the presence of a glycidyl group (3.00 ppm, 3.85 ppm) was confirmed by $^1$H-NMR analysis of the product. Furthermore, it was confirmed by LC-MS analysis that the molecular weight of the product was 249. It was confirmed from these results that the product was benzoylformic acid

amide derivative (D-55) shown in Table 2-6.

Examples 56 to 58 Synthesis of benzoylformic acid amide derivatives (D-56) to (D-58)

Synthesis of benzoylformic acid amide derivatives (D) was performed with raw materials shown in Table 2-6 in the same manner as in Example 23. The presence of a urethane group and a benzoylformic acid amide group was confirmed by FT-IR analysis, and the presence of a cyclic ether and an unsaturated group was confirmed by $^{1}$H-NMR analysis. Specifically, the presence of an alicyclic epoxy group (2.95 ppm, 3.05 ppm), a glycidyl group (3.00 ppm, 3.85 ppm), and an acrylate group (5.85 ppm, 6.20 ppm, 6.45 ppm) was confirmed by $^{1}$H-NMR analysis. Furthermore, the molecular weight of the product was measured by LC-MS analysis in Example 56, and the number average molecular weight (Mn) of the product was calculated by GPC analysis in each of Examples 57 and 58. These are shown in Table 2-6. The chemical formulas of benzoylformic acid amide derivatives (D-56) to (D-58) and other data are collectively shown in Table 2-6.

[Table 1-1]

| Example | (D) | Material (a1) | Material (a2) | Molar ratio a1/a2 | Photopolymerization initiator (D), Chemical formula | Yield (%) | Molecular weight | Chemical shift | | |
|---|---|---|---|---|---|---|---|---|---|---|
| | | | | | | | | α-position | β-position | γ-position |
| Example 1 | D-1 | a1-1 | a2-1 | 1.1/1 | 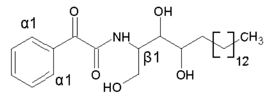 | 85 | 450 | 2H, d, 8.10 | 1H, dt, 4.00 | |
| Example 2 | D-2 | a1-1 | a2-2 | 1.3/1 | 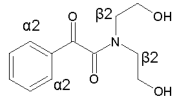 | 92 | 237 | 2H, d, 8.10 | 4H, t, 3.80 | |
| Example 3 | D-3 | a1-2 | a2-3 | 1.3/1 | 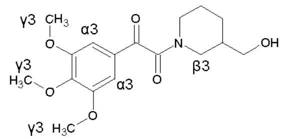 | 91 | 337 | 2H, s, 7.60 | 2H, dd, 3.35 | 6H,s, 3.80<br>3H, s, 3.85 |
| Example 4 | D-4 | a1-3 | a2-4 | 0.8/2 | 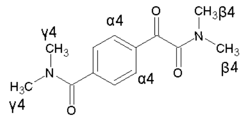 | 75 | 222 | 2H, s, 8.00 | 6H, s, 2.75 | 6H, s, 2.65 |
| Example 5 | D-5 | a1-1 | a2-5 | 1.1/1 | 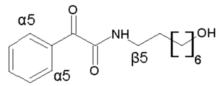 | 81 | 277 | 2H, d, 8.10 | 2H, t, 3.35 | |
| Example 6 | D-6 | a1-1 | a2-6 | 1.1/1 | 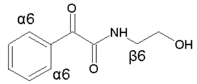 | 94 | 193 | 2H, d, 8.10 | 2H, t, 3.60 | |
| Example 7 | D-7 | a1-4 | a2-6 | 1.1/1 | 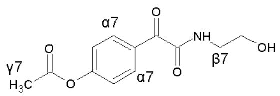 | 65 | 251 | 2H, d, 7.80 | 2H, t, 3.60 | 3H, s, 2.10 |
| Example 8 | D-8 | a1-1 | a2-7 | 1.1/1 | 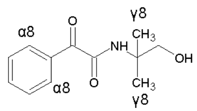 | 91 | 221 | 2H, d, 8.10 | | 2H, s, 1.20 |

(continued)

| Example | (D) | Material (a1) | Material (a2) | Molar ratio a1/a2 | Photopolymerization initiator (D), Chemical formula | Yield (%) | Molecular weight | Chemical shift | | |
|---|---|---|---|---|---|---|---|---|---|---|
| | | | | | | | | α-position | β-position | γ-position |
| Example 9 | D-9 | a1-1 | a2-8 | 2.5/1 | 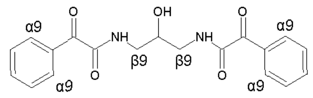 | 77 | 354 | 4H, d, 8.10 | 4H, d, 3.60 | |
| Example 10 | D-10 | a1-5 | a2-9 | 1.1/1 | 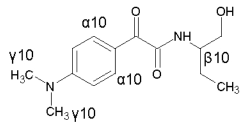 | 96 | 264 | 2H, d, 7.60 | 1H, tt, 4.10 | 6H, s, 2.85 |
| Example 11 | D-11 | a1-6 | a2-6 | 1.1/1 | 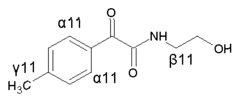 | 96 | 207 | 2H, d, 7.90 | 2H, t, 3.60 | 3H, s, 2.40 |
| Example 12 | D-12 | a1-7 | a2-10 | 1.1/1 | 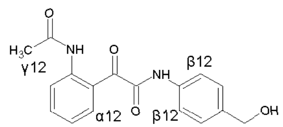 | 95 | 312 | 1H, d, 7.80 | 2H, d, 8.90 | 3H, s, 2.20 |

[Table 1-2]

| Example | (D) | Material (a1) | Material (a2) | Molar ratio a1/a2 | Photopolymerization initiator (D), Chemical formula | Yield (%) | Molecular weight | Chemical shift | | |
|---|---|---|---|---|---|---|---|---|---|---|
| | | | | | | | | α-position | β-position | γ-position |
| Example 13 | D-13 | a1-8 | a2-11 | 1.2/1 | 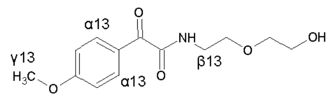 | 85 | 237 | 2H, d, 7.90 | 2H, t, 3.60 | 3H, s, 3.80 |
| Example 14 | D-14 | a1-1 | a2-12 | 1.2/1 | 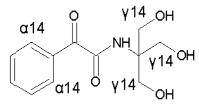 | 92 | 253 | 2H, d, 8.10 | | 6H, s, 3.45 |
| Example 15 | D-15 | a1-1 | a2-13 | 1.2/1 | 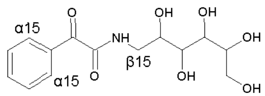 | 91 | 313 | 2H, d, 8.10 | 2H, d, 3.65 | |
| Example 16 | D-16 | a1-1 | a2-14 | 1.1/1 | 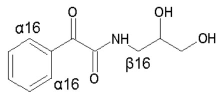 | 75 | 223 | 2H, d, 8.10 | 2H, d, 3.55 | |
| Example 17 | D-17 | a1-9 | a2-14 | 1.1/1 | 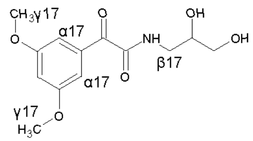 | 81 | 283 | 2H, s, 7.70 | 2H, d, 3.55 | 6H, s, 3.75 |
| Example 18 | D-18 | a1-10 | a2-15 | 1.3/1 | 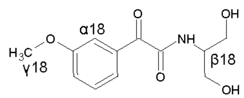 | 94 | 267 | 1H, s, 7.65 | 1H, q, 3.85 | 3H, s, 3.80 |
| Example 19 | D-19 | a1-1 | a2-15 | 1.3/1 | 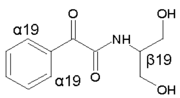 | 65 | 223 | 2H, d, 8.10 | 1H, q, 3.85 | |
| Example 20 | D-20 | a1-11 | a2-16 | 1.5/1 | 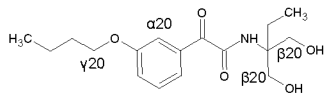 | 91 | 323 | 2H, s, 7.95 | 4H, s, 3.45 | 2H, t, 4.05 |

(continued)

| Example | (D) | Material (a1) | Material (a2) | Molar ratio a1/a2 | Photopolymerization initiator (D), Chemical formula | Yield (%) | Molecular weight | Chemical shift | | |
|---|---|---|---|---|---|---|---|---|---|---|
| | | | | | | | | α-position | β-position | γ-position |
| Example 21 | D-21 | a1-12 | a2-16 | 1.5/1 | 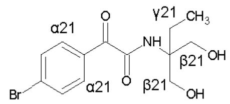 | 84 | 330 | 2H, d, 7.95 | 4H, s, 3.45 | 3H, q, 1.50 |

[Table 2-1]

| Example (D) | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups /number of benzoylformic acid amide groups |
|---|---|---|---|---|---|---|---|---|---|
| Example 22 | D-2 | 45.7 | b3-1 | 54.3 | 98 | 520 | 1 | 4 | 2 |
| D-22 Chemical formula | 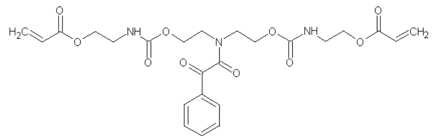 | | | | | | | | |
| Example 23 | D-3 | 50.0 | b1-1<br>b3-2 | 32.9<br>17.1 | 96 | 675 | 1 | 5 | 2 |
| D-23 Chemical formula | 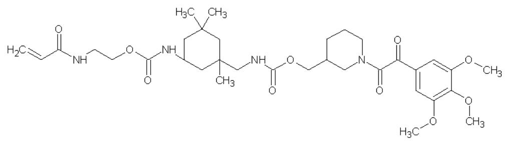 | | | | | | | | |
| Example 24 | D-5 | 74.7 | b3-3 | 25.3 | 84 | 331 | 1 | - | - |
| D-24 Chemical formula | 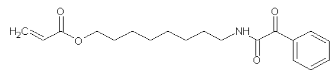 | | | | | | | | |
| Example 25 | D-18 | 63.6 | b3-4 | 36.4 | 43 | 347 | 1 | - | - |
| D-25 Chemical formula | 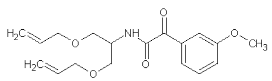 | | | | | | | | |
| Example 26 | D-9 | 86.6 | b3-5 | 13.4 | 38 | 407 | 2 | - | - |
| D-26 Chemical formula | 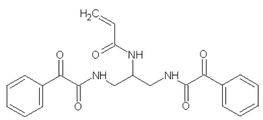 | | | | | | | | |

(continued)

| Example (D) | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups /number of benzoylformic acid amide groups |
|---|---|---|---|---|---|---|---|---|---|
| Example 27 | D-9 | 71.5 | b3-1 | 28.5 | 38 | 407 | 2 | 4 | 0.5 |
| D-27 Chemical formula | 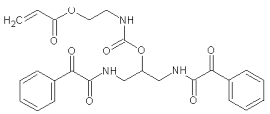 | | | | | | | | |
| Example 28 | D-6 | 63.5 | b1-1 | 36.5 | 98 | 609 | 2 | 4 | 1 |
| D-28 Chemical formula | 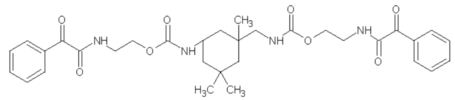 | | | | | | | | |
| Example 29 | D-7 | 18.8 | bl-2<br>b3-6 | 23.5<br>57.7 | 96 | 2700 | 2 | 4 | 3 |
| D-29 Chemical formula | 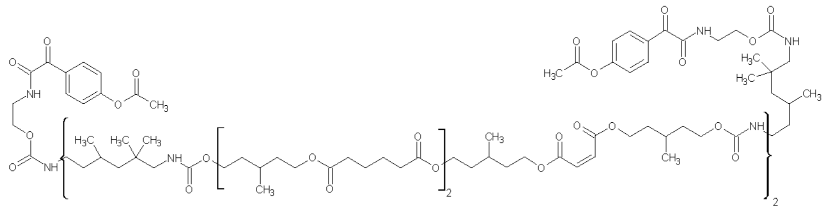 | | | | | | | | |
| Example 30 | D-5 | 22.6 | b1-3<br>b3-7 | 54.4<br>23.0 | 95 | 1300 | 1 | 10 | 3 |

(continued)

| Example (D) | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups /number of benzoylformic acid amide groups |
|---|---|---|---|---|---|---|---|---|---|
| D-30 Chemical formula | 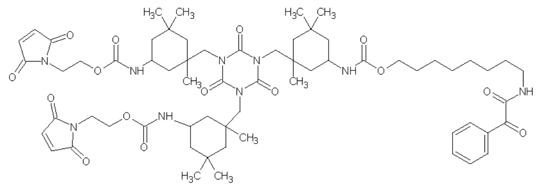 | | | | | | | | |
| (*1) Number of benzoylformic acid amide groups introduced per molecule<br>(*2) Number of atoms directly bound between nitrogen atom of benzoylformic acid amide group and nitrogen atom of proximate urethane group | | | | | | | | | |

[Table 2-2]

| Photopolymerization initiator | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups/number of benzoylformic acid amide groups |
|---|---|---|---|---|---|---|---|---|---|
| Example 31 | D-8 | 41.6 | bl-4<br>b3-2 | 47.5<br>10.9 | 96 | 1100 | 2 | 4 | 1.5 |
| D-31 Chemical formula | 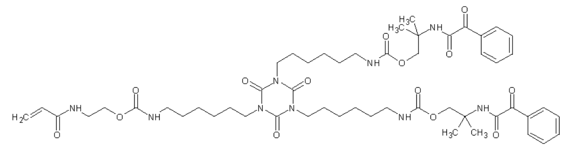 | | | | | | | | |
| Example 32 | D-9 | 20.4 | b1-1<br>b2-1<br>b3-8 | 38.4<br>34.5<br>6.7 | 97 | 1800 | 2 | 4 | 3 |
| D-32 Chemical formula | 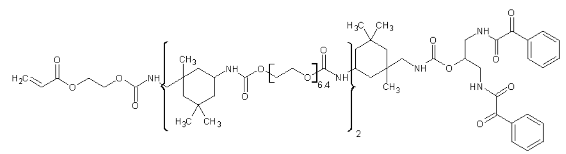 | | | | | | | | |
| Example 33 | D-10 | 39.6 | b1-1<br>b3-9 | 39.8<br>20.6 | 99 | 588 | 1 | 4 | 2 |
| D-33 Chemical formula | 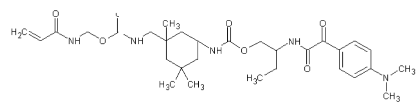 | | | | | | | | |
| Example 34 | D-11 | 9.3 | b1-1<br>b1-5<br>b2-2<br>b3-10 | 9.9<br>7.5<br>66.9<br>6.4 | 91 | 2300 | 1 | 4 | 4 |
| D-34 Chemical formula | 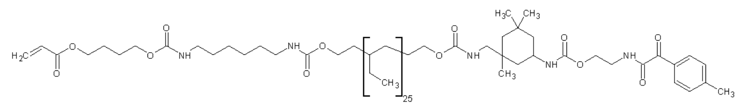 | | | | | | | | |

(continued)

| Photopolymerization initiator | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups/number of benzoylformic acid amide groups |
|---|---|---|---|---|---|---|---|---|---|
| Example 31 | D-8 | 41.6 | bl-4<br>b3-2 | 47.5<br>10.9 | 96 | 1100 | 2 | 4 | 1.5 |
| Example 35 | D-12 | 13.2 | bl-6<br>b2-3<br>b3-11 | 31.8<br>42.4<br>12.6 | 96 | 2400 | 1 | 7 | 6 |
| D-35 Chemical formula | 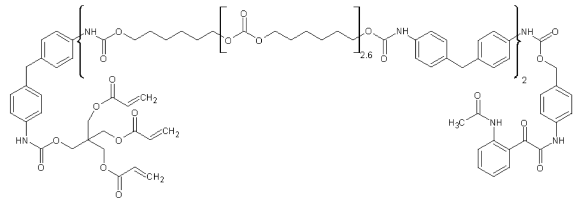 | | | | | | | | |
| Example 36 | D-13 | 11.0 | b1-1<br>b2-4<br>b3-12 | 17.4<br>66.5<br>5.1 | 97 | 2600 | 1 | 7 | 4 |
| D-36 Chemical formula | 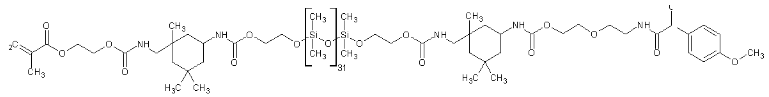 | | | | | | | | |
| Example 37 | D-14 | 20.0 | b1-1<br>b3-2 | 52.7<br>27.3 | 99 | 1300 | 1 | 4 | 6 |
| D-37 Chemical formula | 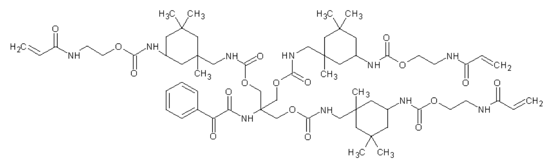 | | | | | | | | |

(*1) Number of benzoylformic acid amide groups introduced per molecule
(*2) Number of atoms directly bound between nitrogen atom of benzoylformic acid amide group and nitrogen atom of proximate urethane group

[Table 2-3]

| Photopolymerization initiator | Benzoylformic acid amide intermediate | Mass (g) | Raw material (b) | Mass (g) | Yield (%) | Number average molecular weight | Number of benzoylformic acid amide groups (*1) | Number of atoms (*2) | Ratio of number of urethane groups/number of benzoylformic acid amide groups |
|---|---|---|---|---|---|---|---|---|---|
| Example 38 | D-1 | 15.3 | b1-1<br>b2-5<br>b3-11 | 45.2<br>9.2<br>30.3 | 92 | 3000 | 1 | 4 | 12 |
| D-38 Chemical formula | 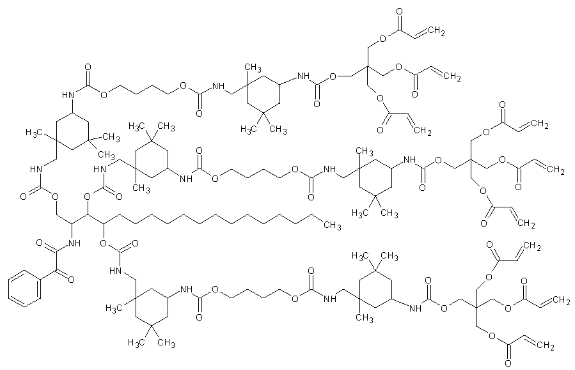 | | | | | | | | |
| Example 39 | D-15 | 30.7 | b3-1 | 69.3 | 97 | 1100 | 1 | 4 | 5 |
| D-39 Chemical formula | 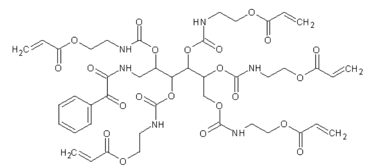 | | | | | | | | |
| Example 40 | D-16 | 44.2 | b3-1 | 55.8 | 99 | 505 | 1 | 4 | 2 |
| D-40 Chemical formula | 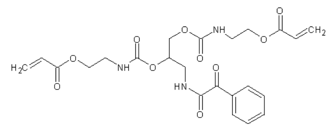 | | | | | | | | |

(continued)

| Photopolymerization initiator | Benzoylformic acid amide intermediate | Mass (g) | Raw material (b) | Mass (g) | Yield (%) | Number average molecular weight | Number of benzoylformic acid amide groups (*1) | Number of atoms (*2) | Ratio of number of urethane groups/number of benzoylformic acid amide groups |
|---|---|---|---|---|---|---|---|---|---|
| Example 41 | D-17 | 16.2 | b1-7<br>b2-1<br>b2-6<br>b3-13 | 38.6<br>34.4<br>3.4<br>7.4 | 92 | 1800 | 1 | 4 | 8 |
| D-41 Chemical formula | 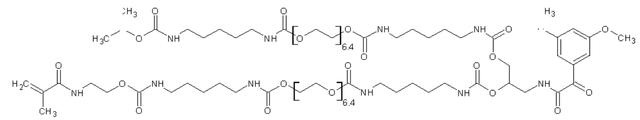 | | | | | | | | |
| Example 42 | D-18 | 41.7 | b1-1<br>b3-8 | 46.2<br>12.1 | 93 | 2000 | 3 | 4 | 2.7 |
| D-42 Chemical formula | 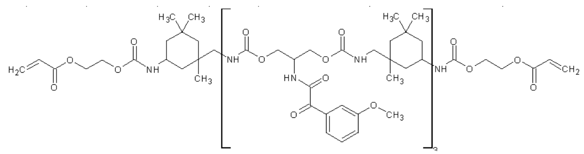 | | | | | | | | |
| Example 43 | D-18 | 41.7 | b1-1<br>b3-2 | 46.3<br>12.0 | 96 | 2000 | 3 | 4 | 2.7 |
| D-43 Chemical formula | 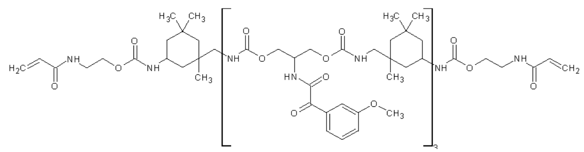 | | | | | | | | |

(*1) Number of benzoylformic acid amide groups introduced per molecule
(*2) Number of atoms directly bound between nitrogen atom of benzoylformic acid amide group and nitrogen atom of proximate urethane group

[Table 2-4]

| Photo-polymerization initiator | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups /number of benzoylformic acid amide groups |
|---|---|---|---|---|---|---|---|---|---|
| Example 44 | D-19 | 36.3 | b1-1<br>b3-10 | 48.1<br>15.6 | 96 | 1900 | 3 | 4 | 2.7 |
| D-44 Chemical formula | 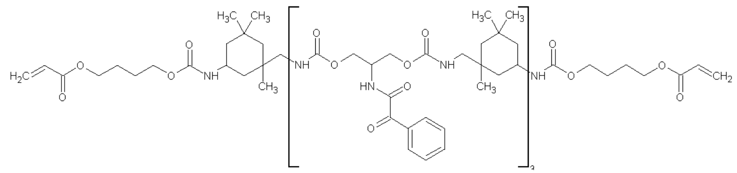 | | | | | | | | |
| Example 45 | D-19 | 27.0 | b1-1<br>b2-5<br>b3-14 | 53.6<br>5.4<br>14.0 | 96 | 1700 | 2 | 4 | 4 |
| D-45 Chemical formula | 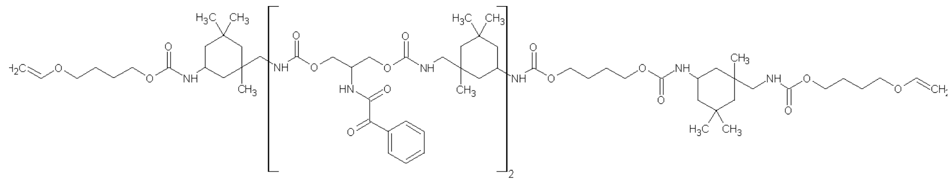 | | | | | | | | |
| Example 46 | D-20 | 47.2 | b1-8<br>b2-7<br>b3-10 | 46.2<br>4.3<br>2.3 | 92 | 6300 | 10 | 4 | 2.2 |
| D-46 Chemical formula | 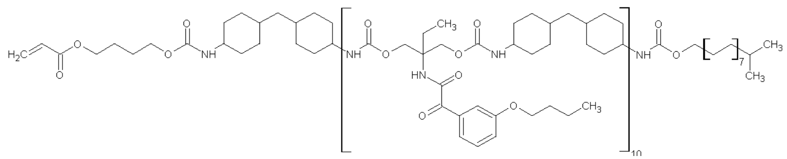 | | | | | | | | |
| Example 47 | D-21 | 9.9 | b1-2<br>b2-8<br>b3-2<br>b3-15 | 15.7<br>68.7<br>1.7<br>4.0 | 95 | 7200 | 2 | 4 | 5 |

(continued)

| Photo-polymerization initiator | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups /number of benzoylformic acid amide groups |
|---|---|---|---|---|---|---|---|---|---|
| D-47 Chemical formula | 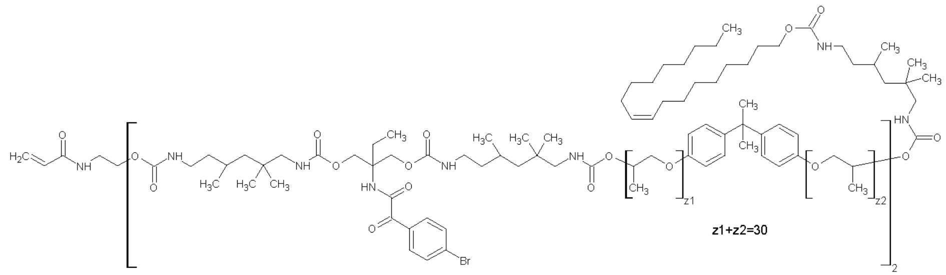 z1+z2=30 | | | | | | | | |
| Example 48 | D-18 | 52.3 | b1-1<br>b3-16 | 44.4<br>3.3 | 86 | 26000 | 50 | 4 | 2.04 |
| D-48 Chemical formula | 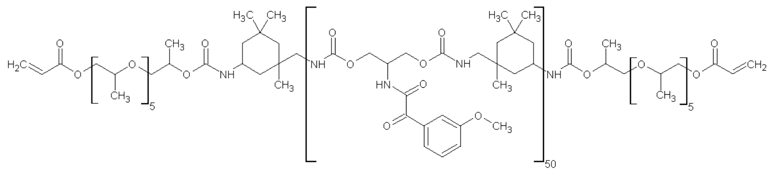 | | | | | | | | |
| (*1) Number of benzoylformic acid amide groups introduced per molecule<br>(*2) Number of atoms directly bound between nitrogen atom of benzoylformic acid amide group and nitrogen atom of proximate urethane group | | | | | | | | | |

[Table 2-5]

| Photopolymerization initiator | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups /number of benzoylformic acid amide groups |
|---|---|---|---|---|---|---|---|---|---|
| Example 49 | D-16 | 15.8 | b1-1<br>b2-9<br>b3-8 | 31.5<br>47.2<br>5.5 | 92 | 4300 | 3 | 4 | 4 |
| D-49 Chemical formula | 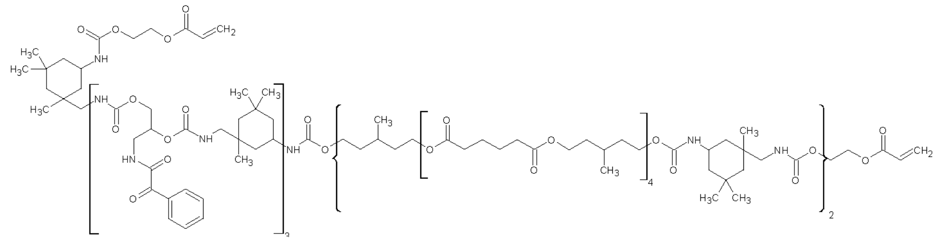 | | | | | | | | |
| Example 50 | D-19 | 25.1 | b1-2<br>b2-10<br>b3-10 | 47.3<br>14.6<br>13.0 | 95 | 4500 | 5 | 4 | 4 |
| D-50 Chemical formula | 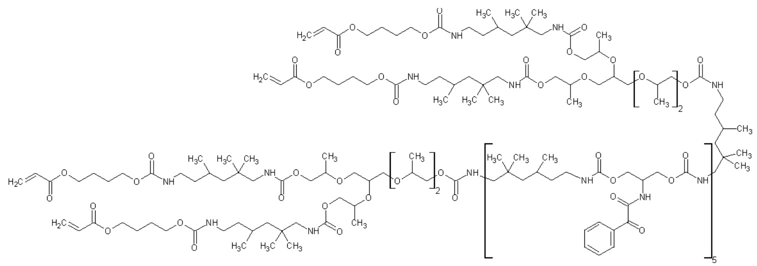 | | | | | | | | |
| Example 51 | D-16 | 16.1 | b1-1<br>b1-4<br>b2-11<br>b3-2<br>b3-10 | 20.1<br>18.2<br>36.2<br>4.2<br>5.2 | 96 | 5600 | 4 | 4 | 4 |

(continued)

| Photopolymerization initiator | Benzoylformic acid amide intermediate | Mass (g) | Material (b) | Mass (g) | Yield (%) | Number average molecular weight | Number of benzoylformic acid amide groups (*1) | Number of atoms (*2) | Ratio of number of urethane groups /number of benzoylformic acid amide groups |
|---|---|---|---|---|---|---|---|---|---|
| D-51 Chemical formula | 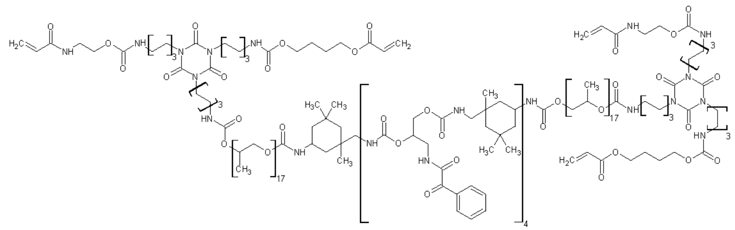 | | | | | | | | |
| Example 52 | D-18 | 2.5 | b1-1<br>b2-12<br>b3-17 | 6.4<br>86.1<br>5.0 | 91 | 22000 | 2 | 4 | 6 |
| D-52 Chemical formula | 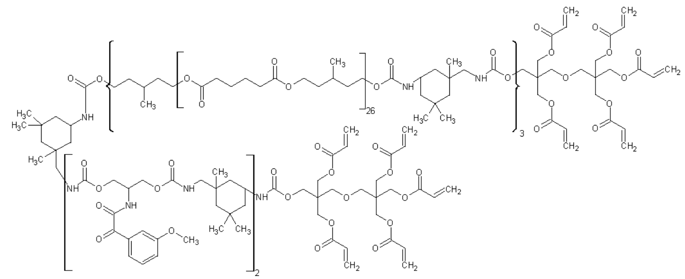 | | | | | | | | |
| (*1) Number of benzoylformic acid amide groups introduced per molecule<br>(*2) Number of atoms directly bound between nitrogen atom of benzoylformic acid amide group and nitrogen atom of proximate urethane group | | | | | | | | | |

[Table 2-6]

| Photopolymerization initiator | Benzoylformic acid amide intermediate | Mass (g) | Material (b) | Mass (g) | Yield (%) | Number average molecular weight | Number of benzoylformic acid amide groups (*1) | Number of atoms(*2) | Ratio of number of urethane groups/number of benzoylformic acid amide groups |
|---|---|---|---|---|---|---|---|---|---|
| Example 53 | D-16 | 32.5 | b1-1<br>b2-13<br>b3-8 | 42.1<br>23.7<br>1.7 | 92 | 14000 | 20 | 4 | 2.6 |
| D-53 Chemical formula | 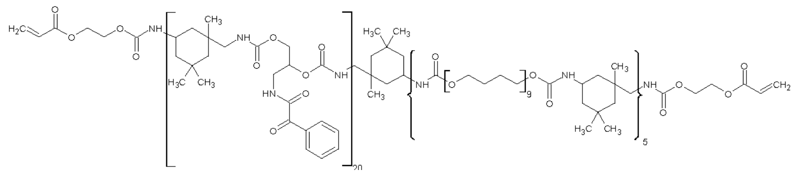 | | | | | | | | |
| Example 54 | D-2<br>D-17 | 9.9<br>11.8 | b1-1<br>b1-5<br>b2-3<br>b3-10 | 20.3<br>14.0<br>41.6<br>2.4 | 93 | 13000 | 10 | 4 | 4.2 |
| D-54 Chemical formula | 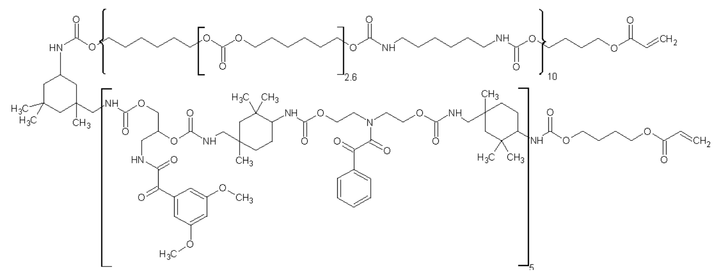 | | | | | | | | |
| Example 55 | D-6 | 67.6 | b4-1 | 32.4 | 65 | 249 | 1 | - | - |
| D-55 Chemical formula | 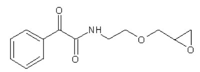 | | | | | | | | |
| Example 56 | D-3 | 46.4 | b1-8<br>b4-2 | 36.0<br>17.6 | 96 | 688 | 1 | 5 | 2 |
| D-56 Chemical formula | 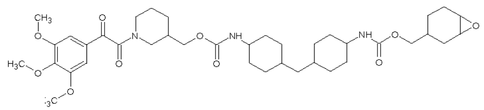 | | | | | | | | |

(continued)

| Photopolymerization initiator | Benzoylformic acid amide intermediate | Mass (g) | Material (b) | Mass (g) | Yield (%) | Number average molecular weight | Number of benzoylformic acid amide groups (*1) | Number of atoms(*2) | Ratio of number of urethane groups/number of benzoylformic acid amide groups |
|---|---|---|---|---|---|---|---|---|---|
| Example 57 | D-2 | 9.4 | b1-1<br>b2-11<br>b3-8<br>b4-3 | 26.4<br>59.5<br>2.3<br>2.4 | 95 | 5100 | 2 | 4 | 6 |
| D-57 Chemical formula | 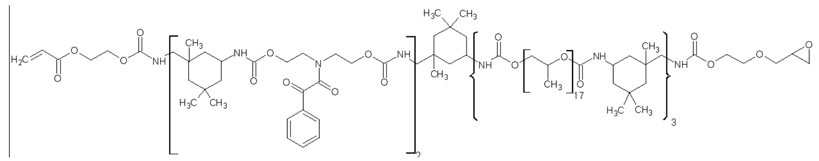 | | | | | | | | |
| Example 58 | D-16 | 22.6 | b1-2<br>b2-9<br>b4-3 | 35.6<br>33.8<br>8.0 | 96 | 3100 | 3 | 4 | 3.3 |
| D-58 Chemical formula | 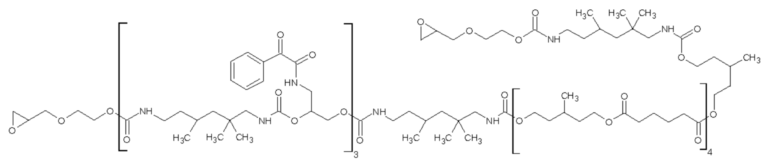 | | | | | | | | |

(*1) Number of benzoylformic acid amide groups introduced per molecule
(*2) Number of atoms directly bound between nitrogen atom of benzoylformic acid amide group and nitrogen atom of proximate urethane group

Examples 59 to 100 and Comparative Examples 1 to 3 (preparation and evaluation of active energy ray-curable compositions)

Benzoylformic acid amide derivatives (D-1) to (D-54) synthesized in Examples and commercially available photopolymerization initiators (E-1) to (E-3) in Comparative Examples were used, monofunctionally unsaturated compound (h1), polyfunctionally unsaturated compound (h2) and photosensitizer (I), and other component (k) were weighed at each proportion shown in Table 3-1 and Table 3-2, and mixed at 25°C for 30 minutes, to obtain each active energy ray-curable composition (hereinafter, abbreviated as "curable composition".). The compatibility and the curability with light beams of different wavelengths, of the curable composition obtained, were evaluated. A photocured product of the curable composition was produced, and the content rate of the low-molecular-weight component derived from the photopolymerization initiator in the cured product, and the light yellowing resistance and the durability of the cured product were evaluated by the following methods. The results are shown in Table 3-1 and Table 3-2.

<Compatibility>

The state of the curable composition was visually observed, and the compatibility was evaluated on a 4-point scale.

++: high transparency, and neither clouding, nor phase separation observed at all.
+: high transparency, but clouding slightly observed.
±: no phase separation observed, but clouding observed.
-: not only clouding, but also phase separation observed.

<Curability>

The curable composition was applied to a polyethylene terephthalate film (Cosmoshine A-4100, corona-treated surface, thickness 100 μm, manufactured by Toyobo Co., Ltd.) (hereinafter, referred to as "PET film".) by a bar coater so that the thickness was 20 μm. A coating film was cured by irradiation with a light beam of a different wavelength, the cumulative amount of light until disappearance of tack during contact with a cured product was determined, and the curability was evaluated on a 4-point scale. The following three kinds 1) to 3) of lamps for irradiation with ultraviolet light were used. The cumulative amount of light necessary until disappearance of tack is smaller, the curability is higher.

1) High-pressure mercury lamp: wavelength 200 to 450 nm, illuminance 100 $mW/cm^2$
2) UV-LED lamp: wavelength 385 nm, illuminance 100 $mW/cm^2$
3) UV-LED lamp: wavelength 405 nm, illuminance 100 $mW/cm^2$

++: tack disappeared in a cumulative amount of light of less than 500 $mJ/cm^2$.
+: tack disappeared in a cumulative amount of light of 500 $mJ/cm^2$ or more and less than 1,000 $mJ/cm^2$.
±: tack disappeared in a cumulative amount of light of 1,000 $mJ/cm^2$ or more and less than 5,000 $mJ/cm^2$.
-: tack remained even in a cumulative amount of light of 5,000 $mJ/cm^2$.

<Content rate of low-molecular-weight component>

The curable composition was applied to a polyester-based heavy release film (E7001, thickness 75 μm, manufactured by Toyobo Co., Ltd.) (hereinafter, referred to as "heavy release film".), and the resultant was bonded with a polyester-based light release film (E7002, thickness 50 μm, manufactured by Toyobo Co., Ltd.) (hereinafter, referred to as "light release film".) so that the thickness was 20 μm, by use of a desktop type roll laminator machine (RSL-382S manufactured by Royal Sovereign) so that air bubbles were not entrained, and irradiated with ultraviolet light (high-pressure mercury lamp, illuminance 100 $mW/cm^2$, cumulative amount of light 5,000 $mJ/cm^2$). Thereafter, the light release film was released, three 5-$cm^2$-sized test pieces were cut out, dried at 90°C for 2 minutes, and weighed, and the mass of a cured film before extraction was defined. Into an ultraviolet-impermeable brown glass bottle were placed 25 g of acetone and the cured film weighed, the glass bottle was sealed, and the glass bottle was rotated at 30°C for 48 hours, to extract a soluble component in the cured film. The solution after extraction was filtered with a 0.45-μm filter, HPLC analysis was performed to quantitatively determine the amount of the low-molecular-weight component based on a calibration curve, and the content rate of the low-molecular-weight component was calculated by the following expression and evaluated as described below.

Content rate (%) of low-molecular-weight component = (Mass of low-molecular-weight component extracted/Mass of cured film before extraction) × 100%

++: a content rate of the low-molecular-weight component of 1.0% or less.
+: a content rate of the low-molecular-weight component of more than 1.0% and 2.0% or less.
±: a content rate of the low-molecular-weight component of more than 2.0% and 4.0% or less.
-: a content rate of the low-molecular-weight component of more than 4.0%.

<Light yellowing resistance>

A heavy release film was closely contacted with a glass plate horizontally disposed, a spacer made of silicone having an inner volume of 10 mm × 10 mm × 0.5 mm (hereinafter, one made of silicone being used in the case of no designation of any material) was disposed thereon, and the curable composition was packed in the spacer. A light release film was covered on a liquid surface of the spacer so that air bubbles were not entrained, and irradiated with ultraviolet light by an UV-LED lamp (wavelength 405 nm, illuminance 100 mW/cm$^2$, cumulative amount of light 20,000 mJ/cm$^2$). Thereafter, the light release film was released, a cured product was taken out from the spacer, and visually observed, and light yellowing resistance was evaluated according to the following criteria.

++: no yellowing observed at all.
+: yellowing extremely slightly observed.
±: yellowing observed.
-: clear yellowing observed.

<Durability>

A cured product of the curable composition was produced as in light yellowing resistance evaluation except that the cumulative amount of light was changed to 5,000 mJ/cm$^2$. Thereafter, the cured product was left to stand still in a constant temperature and humidity chamber at a temperature of 40°C and a relative humidity of 50% for 168 hours, the presence of bleed-out on a surface of the cured product was visually observed, and durability was evaluated according to the following criteria.

++: no bleed-out observed at all.
+: bleed-out extremely slightly observed.
±: bleed-out slightly observed.
-: bleed-out remarkably observed.

[Table 3-1]

| Active energy ray-curable composition | | Photo-polymerization initiator(D), (E) | Mass(%) | Mono-functionally unsaturated compound (h1) | Mass(%) | Poly-functionally unsaturated compound (h2) | Mass(%) | Photosensitizer(l) or Other(k) | Mass(%) | Compatibility | Curability (light source) | | | Content rate of low-molecular-weight component | Light yellowing resistance | Durability |
|---|---|---|---|---|---|---|---|---|---|---|---|---|---|---|---|---|
| | | | | | | | | | | | High-pressure mercury | LED 385 | LED 405 | | | |
| Example 59 | F-1 | D-1 | 5 | h1-1<br>h1-2 | 50<br>20 | h2-1 | 25 | | | + | ++ | + | + | + | + | + |
| Example 60 | F-2 | D-3 | 10 | h1-1 | 50 | h2-2 | 40 | | | + | + | ± | ± | + | + | + |
| Example 61 | F-3 | D-6 | 1 | | | h2-1<br>h2-3 | 49<br>50 | | | + | + | + | + | + | + | + |
| Example 62 | F-4 | D-7 | 0.5 | h1-1<br>h1-3 | 50<br>15 | h2-2 | 30 | k-1 | 4.5 | + | ++ | + | + | + | + | + |
| Example 63 | F-5 | D-9 | 5 | h1-1<br>h1-4 | 20<br>25 | h2-4 | 50 | | | + | ++ | ++ | + | ++ | + | ++ |
| Example 64 | F-6 | D-12 | 1 | h1-1<br>h1-2 | 35<br>40 | h2-2 | 20 | I-1 | 4 | + | ++ | + | + | + | ± | + |
| Example 65 | F-7 | D-15 | 5 | h1-1<br>h1-2 | 35<br>40 | h2-2 | 20 | | | ± | + | + | + | + | + | + |
| Example 66 | F-8 | D-20 | 10 | h1-1 | 50 | h2-2 | 40 | | | + | ++ | + | + | + | + | + |
| Example 67 | F-9 | D-21 | 5 | h1-1<br>h1-2 | 35<br>40 | h2-2 | 20 | | | + | + | + | + | + | + | + |
| Example 68 | F-10 | D-8<br>D-22 | 0.5<br>99 | h1-1 | 0.5 | | | | | ++ | + | + | + | ++ | + | ++ |
| Example 69 | F-11 | D-23 | 10 | h1-1 | 50 | h2-2 | 40 | | | ++ | + | + | ± | + | + | + |
| Example 70 | F-12 | D-24 | 10 | h1-1 | 50 | h2-2 | 40 | | | + | + | + | + | + | + | + |
| Example 71 | F-13 | D-25 | 10 | h1-1<br>h1-2 | 40<br>20 | h2-6 | 30 | | | + | ++ | ++ | + | ++ | + | ++ |
| Example 72 | F-14 | D-26 | 5 | h1-5 | 50 | h2-3 | 45 | | | + | ++ | ++ | + | ++ | + | ++ |
| Example 73 | F-15 | D-27 | 5 | h1-5 | 50 | h2-3 | 45 | | | ++ | ++ | ++ | ++ | ++ | + | ++ |
| Example 74 | F-16 | D-28 | 20 | h1-1 | 40 | h2-6 | 40 | | | ++ | ++ | ++ | + | ++ | + | ++ |
| Example 75 | F-17 | D-29 | 25 | h1-1 | 50 | h2-7 | 25 | | | ++ | ++ | ++ | ++ | ++ | + | ++ |
| Example 76 | F-18 | D-30 | 10 | h1-5 | 45 | h2-7 | 45 | | | + | + | + | + | ++ | + | ++ |
| Example 77 | F-19 | D-31 | 25 | h1-2 | 25 | h2-1 | 50 | | | + | ++ | ++ | ++ | ++ | + | ++ |
| Example 78 | F-20 | D-32 | 30 | h1-6 | 30 | h2-3<br>h2-8 | 30<br>10 | | | ++ | ++ | ++ | ++ | ++ | + | ++ |
| Example 79 | F-21 | D-33 | 0.5 | h1-1<br>h1-7 | 50<br>28 | h2-9 | 20 | I-2 | 1.5 | ++ | ++ | + | ± | ++ | ± | ++ |
| Example 80 | F-22 | D-34 | 50 | | | h2-1 | 50 | | | ++ | ++ | ++ | ++ | ++ | + | ++ |
| Example 81 | F-23 | D-35 | 15 | h1-8 | 35 | h2-5 | 50 | | | ++ | ++ | ++ | ++ | ++ | ± | ++ |

[Table 3-2]

| Active energy ray-curable composition | | Photopolymerization initiator(D), (E) | Mass(%) | Mono-functionally unsaturated compound(h1) | Mass(%) | Poly-functionally unsaturated compound(h2) | Mass(%) | Photosensitizer(I) or Other(k) | Mass(%) | Compatibility | Curability (light source) | | | Content rate of low-molecular-weight component | Light yellowing resistance | Durability |
|---|---|---|---|---|---|---|---|---|---|---|---|---|---|---|---|---|
| | | | | | | | | | | | High-pressure mercury | LED 385 | LED 405 | | | |
| Example 82 | F-24 | D-36 | 10 | h1-5 | 45 | h2-7 | 45 | | | + | ++ | + | + | ++ | + | ++ |
| Example 83 | F-25 | D-37 | 90 | | | h2-10 | 10 | | | + | ++ | ++ | ++ | ++ | + | ++ |
| Example 84 | F-26 | D-38 | 50 | h1-10 | 50 | | | | | + | ++ | ++ | + | ++ | + | ++ |
| Example 85 | F-27 | D-39 | 25 | h1-1 | 50 | h2-7 | 25 | | | + | ++ | ++ | + | ++ | + | ++ |
| Example 86 | F-28 | D-40 | 10 | h1-1 | 50 | h2-2 | 40 | | | ++ | ++ | ++ | + | ++ | + | ++ |
| Example 87 | F-29 | D-9<br>D-41 | 10<br>20 | h1-1<br>h1-4 | 10<br>20 | h2-5 | 10 | | | + | ++ | ++ | ++ | ++ | + | ++ |
| Example 88 | F-30 | D-42 | 2 | h1-1<br>h1-2 | 38<br>40 | h2-2 | 20 | | | + | ++ | ++ | + | ++ | + | ++ |
| Example 89 | F-31 | D-43 | 2 | h1-1<br>h1-2 | 38<br>40 | h2-2 | 20 | | | + | ++ | ++ | + | ++ | + | ++ |
| Example 90 | F-32 | D-44 | 2 | h1-1<br>h1-2 | 38<br>40 | h2-2 | 20 | | | + | ++ | + | + | ++ | + | ++ |
| Example 91 | F-33 | D-45 | 30 | h1-10 | 50 | h2-3 | 20 | | | ++ | ++ | ++ | + | ++ | + | ++ |
| Example 92 | F-34 | D-46 | 1 | h1-1<br>h1-13 | 50<br>19 | h2-4 | 30 | | | ++ | ++ | + | + | ++ | + | ++ |
| Example 93 | F-35 | D-47 | 30 | h1-14 | 20 | h2-9 | 50 | | | ++ | ++ | + | + | ++ | + | ++ |
| Example 94 | F-36 | D-48 | 0.1 | h1-1 | 9 | h2-11 | 90 | k-1 | 0.9 | ++ | + | ± | ± | + | + | + |
| Example 95 | F-37 | D-49 | 5 | h1-2<br>h1-8 | 20<br>15 | h2-6 | 60 | | | ++ | ++ | + | + | ++ | + | ++ |
| Example 96 | F-38 | D-50 | 3 | h1-1<br>h1-15 | 70<br>20 | h2-2 | 7 | | | + | ++ | + | + | ++ | + | ++ |
| Example 97 | F-39 | D-51 | 10 | h1-1<br>h1-16 | 20<br>20 | h2-12 | 50 | | | + | ++ | + | + | ++ | + | ++ |
| Example 98 | F-40 | D-48<br>D-52 | 10<br>20 | h1-4<br>h1-9 | 20<br>50 | | | | | ++ | ++ | ++ | ++ | ++ | + | ++ |
| Example 99 | F-41 | D-53 | 5 | h1-1<br>h1-17 | 35<br>40 | h2-6 | 20 | | | ++ | ++ | + | + | ++ | + | ++ |
| Example 100 | F-42 | D-54 | 10 | h1-10<br>h1-16 | 30<br>10 | h2-1 | 50 | | | + | ++ | ++ | ++ | ++ | + | ++ |
| Comparative Example 1 | G-1 | E-1 | 5 | h1-1<br>h1-17 | 35<br>40 | h2-6 | 20 | | | ++ | ++ | ± | - | - | + | - |
| Comparative Example 2 | G-2 | E-2 | 5 | h1-1<br>h1-2 | 35<br>40 | h2-2 | 20 | | | + | ++ | + | + | - | + | - |
| Comparative Example 3 | G-3 | E-3 | 2 | h1-1<br>h1-2 | 38<br>40 | h2-2 | 20 | | | ++ | ++ | ++ | + | - | + | - |

As clear from the evaluation results in Table 3-1 and Table 3-2, the curable composition of each Example, with the benzoylformic acid amide derivative (D) of the present disclosure, was favorable in compatibility, and also high in curability with not only a high-pressure mercury lamp, but also light beams at 385 nm and 405 nm of an UV-LED lamp. The cured products obtained in Examples were low in content rate of the low-molecular-weight component, high in safety, and excellent in light yellowing resistance and durability. D-20 (Example 66) having a benzoylformic acid monosubstituted-amide group exhibited higher curability than D-3 (Example 60) having a benzoylformic acid disubstituted-amide group. D-40 (Example 86) containing a urethane group exhibited higher compatibility and curability than D-24 (Example 70) containing no urethane group. With respect to each D in which the respective numbers of atoms directly linking a nitrogen atom of the benzoylformic acid amide group and a nitrogen atom of a proximate urethane group were 10 (D-30 of Example

76), 7 (D-36 of Example 82), and 4 (D-40 of Example 86), those in which the respective numbers of atoms were 7 and 4 exhibited high curability than one in which the number of atoms was 10, and one in which the number of atoms was 4 exhibited the highest curability. D-42 (Example 88) and D-43 (Example 89) each having a methoxy group on a benzene ring of a benzoylformic acid amide group exhibited higher curability than D-44 (Example 90) having no methoxy group. D-22 (Example 68), D-23 (Example 69), D-27 (Example 73), D-28 (Example 74), D-33 (Example 79) and D-40 (Example 86) each having a molecular weight of less than 1,000 and having a urethane group exhibited favorable compatibility. Furthermore, D-29 (Example 75), D-32 (Example 78), D-34 (Example 80), D-35 (Example 81), D-45 to D-49 (Examples 91 to 95), D-48 and D-52 (Example 98), and D-53 (Example 99) each having a molecular weight of 1,000 or more and having a urethane group and a polyol-derived alkylene structure unit, a polyether structure unit, a polyester structure unit, a polycarbonate structure unit, a polyolefin structure unit or a polysiloxane structure unit not only were high in molecular weight, but also exhibited favorable compatibility. These cured products obtained in Examples were low in content rate of the low-molecular-weight component, and the cured products were high in both light yellowing resistance and durability. On the other hand, in Comparative Example 1 in which Esacure KIP 150 (E-1) was used as a photopolymerization initiator, the curability with a light beam of a wavelength of 405 nm was low, both the remaining content (unpolymerized) of the polymerizable compound (h) and a decomposed product generated due to intramolecular cleavage of E-1 remained in the cured product, and the content rate of the low-molecular-weight component in the cured product was high. Although curing with a light beam of 405 nm was possible in Comparative Example 2 in which methyl benzoylformate (E-2) was used and Comparative Example 3 in which 2,4,6-trimethylbenzoyl diphenylphosphine oxide (E-3) was used, E-2 itself having a molecular weight of 164 was a low-molecular-weight component and E-3 was an intramolecular cleavage-type photopolymerization initiator, and therefore both the cured products of Comparative Examples 2 and 3 were high in content rate of the low-molecular-weight component. The cured products of all of Comparative Examples 1 to 3 were low in light yellowing resistance and durability.

Examples 101 to 137 and Comparative Examples 4 to 10 (preparation and evaluation of photoradical polymerization-type active energy ray-curable compositions)

Benzoylformic acid amide derivatives (D-2) to (D-57) obtained in respective Examples and commercially available photosensitizers (I-3) and (I-4) in Comparative Examples were used, monofunctionally unsaturated compound (h1), polyfunctionally unsaturated compound (h2) and the photoradical polymerization initiator (E) were weighed at the composition shown in Table 4-1 and Table 4-2, and mixed at 25°C for 30 minutes, to prepare each photoradical polymerization-type active energy ray-curable composition (hereinafter, also referred to as "radical-type curable composition".). The compatibility and the curability with light beams of different wavelengths, of the radical-type curable composition obtained, and the light yellowing resistance and the durability of the resulting cured product were evaluated by the same methods as in evaluation of the curable composition. The results are shown in Table 4-1 and Table 4-2. As the curability of the curable composition is higher, the photosensitivity of D is higher.

[Table 4-1]

| Photoradical polymerization sensitization-type active energy ray-curable composition | | Photosensitizer (D), (I) | Mass(%) | Photopolymerization initiator(D), (E) | Mass(%) | Monofunctionally unsaturated compound(h1) | Mass(%) | Poly-functionally unsaturated compound(h2) | Mass(%) | Compatibility | Curability (light source) | | | Light yellowing resistance | Durability |
|---|---|---|---|---|---|---|---|---|---|---|---|---|---|---|---|
| | | | | | | | | | | | High-pressure mercury | LED 385 | LED 405 | | |
| Example 101 | F-43 | D-2 | 1 | E-1 | 5 | h1-1 | 40 | h2-1 | 54 | + | ++ | + | + | + | ± |
| Example 102 | F-44 | D-3 | 1 | E-4 | 5 | h1-1 | 40 | h2-1 | 54 | + | ++ | ++ | ++ | + | ++ |
| Example 103 | F-45 | D-3 | 0.1 | E-5 | 4.9 | h1-1 | 50 | h2-2 | 45 | + | ++ | + | ± | + | ± |
| Example 104 | F-46 | D-4 | 0.5 | E-6 | 2.5 | h1-6<br>h1-9 | 40<br>25 | h2-7 | 32 | + | ++ | + | + | + | + |
| Example 105 | F-47 | D-5 | 1 | E-1 | 5 | h1-1 | 40 | h2-1 | 54 | + | ++ | + | + | + | ± |
| Example 106 | F-48 | D-7 | 0.1 | E-5 | 0.9 | h1-1<br>h1-3 | 50<br>19 | h2-2 | 30 | + | + | + | ± | + | ± |
| Example 107 | F-49 | D-10 | 0.3 | E-7 | 1.7 | h1-12<br>h1-16 | 50<br>8 | h2-5 | 40 | + | ++ | + | + | + | + |
| Example 108 | F-50 | D-12 | 0.1 | E-5 | 0.9 | h1-1<br>h1-2 | 35<br>40 | h2-2 | 24 | + | + | + | ± | + | ± |
| Example 109 | F-51 | D-16 | 0.5 | E-1 | 1.5 | h1-3<br>h1-11 | 38<br>30 | h2-9 | 30 | + | ++ | ++ | + | + | + |
| Example 110 | F-52 | D-17 | 0.5 | E-5 | 1.5 | h1-1<br>h1-2 | 38<br>40 | h2-2 | 20 | + | ++ | ++ | ++ | + | + |
| Example 111 | F-53 | D-18 | 0.5 | E-5 | 1.5 | h1-1<br>h1-2 | 38<br>40 | h2-2 | 20 | + | ++ | ++ | + | + | + |
| Example 112 | F-54 | D-21 | 0.5 | E-5 | 1.5 | h1-1<br>h1-17 | 38<br>40 | h2-6 | 20 | + | ++ | + | + | + | + |
| Example 113 | F-55 | D-22 | 1 | E-1 | 5 | h1-2<br>h1-8 | 20<br>14 | h2-6 | 60 | ++ | ++ | + | + | + | ± |
| Example 114 | F-56 | D-22 | 1 | D-49 | 5 | h1-2<br>h1-8 | 20<br>14 | h2-6 | 60 | ++ | ++ | ++ | + | + | ++ |
| Example 115 | F-57 | D-23 | 1 | D-49 | 5 | h1-2<br>h1-8 | 20<br>14 | h2-6 | 60 | ++ | ++ | ++ | ++ | + | ++ |
| Example 116 | F-58 | D-8<br>D-24 | 0.5<br>1.5 | E-4 | 8 | h1-2 | 10 | h2-5 | 80 | + | ++ | ++ | ++ | + | ++ |
| Example 117 | F-59 | D-25 | 1 | E-4 | 5 | h1-12 | 44 | h2-4 | 50 | + | ++ | ++ | ++ | + | ++ |
| Example 118 | F-60 | D-26 | 1 | E-5 | 1 | h1-5 | 50 | h2-3 | 48 | + | ++ | ++ | + | + | + |
| Example 119 | F-61 | D-27 | 0.5 | E-5 | 1.5 | h1-5 | 50 | h2-3 | 48 | ++ | ++ | ++ | + | + | + |
| Example 120 | F-62 | D-28 | 0.5 | E-5 | 1.5 | h1-1 | 48 | h2-6 | 50 | ++ | ++ | + | + | + | + |
| Example 121 | F-63 | D-29 | 0.5 | E-5 | 2.5 | h1-1 | 50 | h2-7 | 47 | ++ | ++ | ++ | + | + | + |
| Example 122 | F-64 | D-32 | 3 | E-8 | 1 | h1-6 | 36 | h2-8<br>h2-9 | 30<br>30 | ++ | ++ | ++ | + | + | ++ |

[Table 4-2]

| Photoradical polymerization sensitization-type active energy ray-curable composition | | Photosensitizer(D), (I) | Mass(%) | Photopolymerization initiator(D), (E) | Mass(%) | Mono-functionally unsaturated compound(h1) | Mass(%) | Poly-functionally unsaturated compound(h2) | Mass(%) | Compatibility | Curability (light source) | | | Light yellowing resistance | Durability |
|---|---|---|---|---|---|---|---|---|---|---|---|---|---|---|---|
| | | | | | | | | | | | High-pressure mercury | LED 385 | LED 405 | | |
| Example 123 | F-65 | D-33 | 0.1 | E-5 | 0.9 | h1-1<br>h1-7 | 50<br>29 | h2-3 | 20 | ++ | ++ | + | + | + | + |
| Example 124 | F-66 | D-34 | 0.5 | E-5 | 1.5 | | | h2-10 | 98 | ++ | ++ | + | + | + | + |
| Example 125 | F-67 | D-35 | 0.3 | E-6 | 1.7 | h1-8 | 48 | h2-5 | 50 | + | ++ | + | + | + | + |
| Example 126 | F-68 | D-40 | 1 | E-1 | 5 | h1-2<br>h1-8 | 20<br>14 | h2-6 | 60 | ++ | ++ | + | + | + | ± |
| Example 127 | F-69 | D-41 | 10 | E-4 | 20 | h1-14 | 20 | h2-9 | 50 | ++ | ++ | ++ | ++ | + | ++ |
| Example 128 | F-70 | D-42 | 0.5 | E-5 | 1.5 | h1-1<br>h1-2 | 38<br>40 | h2-2 | 20 | + | ++ | ++ | + | + | + |
| Example 129 | F-71 | D-43 | 0.5 | E-5 | 1.5 | h1-1<br>h1-2 | 38<br>40 | h2-2 | 20 | + | ++ | ++ | + | + | + |
| Example 130 | F-72 | D-44 | 0.5 | E-5 | 1.5 | h1-1<br>h1-2 | 38<br>40 | h2-2 | 20 | + | ++ | + | + | + | + |
| Example 131 | F-73 | D-46 | 0.1 | E-5 | 0.9 | h1-1<br>h1-13 | 50<br>19 | h2-4 | 30 | ++ | + | + | ± | + | ± |
| Example 132 | F-74 | D-48 | 1 | E-7 | 2 | h1-3<br>h1-11 | 40<br>27 | h2-3 | 30 | + | ++ | ++ | + | + | ++ |
| Example 133 | F-75 | D-48 | 0.1 | E-5 | 1 | h1-1 | 9 | h2-10 | 90 | ++ | + | ± | ± | + | + |
| Example 134 | F-76 | D-50 | 2.5 | E-5 | 2.5 | h1-1<br>h1-15 | 70<br>20 | h2-2 | 5 | + | + | + | + | + | + |
| Example 135 | F-77 | D-52 | 1 | E-4 | 9 | h1-12 | 40 | h2-4 | 50 | + | ++ | ++ | + | + | ++ |
| Example 136 | F-78 | D-54 | 5 | E-4 | 5 | h1-10<br>h1-16 | 30<br>10 | h2-1 | 50 | ++ | ++ | ++ | ++ | + | ++ |
| Example 137 | F-79 | D-57 | 0.5 | E-5 | 2.5 | h1-1 | 50 | h2-7 | 47 | ++ | ++ | + | + | + | + |
| Comparative Example 4 | G-4 | | | E-5 | 5 | h1-1<br>h1-17 | 35<br>40 | h2-6 | 20 | ++ | + | - | - | *3 | *3 |
| Comparative Example 5 | G-5 | | | E-6 | 5 | h1-12 | 45 | h2-4 | 50 | ++ | + | - | - | *3 | *3 |
| Comparative Example 6 | G-6 | | | E-7 | 3 | h1-6<br>h1-9 | 40<br>25 | h2-7 | 32 | ++ | + | - | - | *3 | *3 |
| Comparative Example 7 | G-7 | | | E-8 | 5 | h1-6 | 35 | h2-8<br>h2-9 | 30<br>30 | ++ | + | - | - | *3 | *3 |
| Comparative Example 8 | G-8 | | | E-9 | 30 | h1-14 | 20 | h2-9 | 50 | ± | ++ | ± | - | *3 | *3 |
| Comparative Example 9 | G-9 | I-3 | 1 | E-1 | 5 | h1-1 | 40 | h2-1 | 54 | + | ++ | + | + | - | - |
| Comparative Example 10 | G-10 | I-4 | 5 | E-4 | 5 | h1-10<br>h1-16 | 30<br>10 | h2-1 | 50 | ± | ++ | ++ | + | - | ± |

(*3) No cured product obtained and no evaluation carried out.

Examples 138 to 143 and Comparative Examples 11 to 15 (preparation and evaluation of photoionic polymerization-type active energy ray-curable compositions)

The benzoylformic acid amide derivative (D) obtained in each Example and commercially available photosensitizers (I-3) to (I-5) were used, and cyclic ether compound (h3) and photoionic polymerization initiator (E-10) or (E-11),

and other additive (k) were weighed at the composition shown in Table 5, and mixed at 25°C for 30 minutes, to prepare each photoionic polymerization-type active energy ray-curable composition (hereinafter, also referred to as "ionic curable composition".). The compatibility and the curability with light beams of different wavelengths, of the ionic curable composition obtained, and the light yellowing resistance and the durability of the resulting cured product were evaluated by the following methods. The results are shown in Table 5. As the curability of the curable composition is higher, the photosensitivity of D is higher.

<Compatibility>

The compatibility of the photoionic polymerization-type curable composition was evaluated by the same method and evaluation criteria as in compatibility evaluation of the photoradical polymerization-type curable composition.

<Curability>

A coating film having a thickness of 20 μm was produced on a PET film in the same manner as in curability evaluation of the photoradical polymerization-type curable composition, and irradiated with an active energy ray by use of each light source of 4) to 6) described below, in conditions described below. Thereafter, the resultant was left to stand still in a constant temperature machine at 70°C for 1 hour, to obtain a film-shaped cured product. The cumulative amount of light, in which tack during touch with a surface of the cured product obtained disappeared, was determined, and the curability was evaluated according to the following criteria. As the cumulative amount of light necessary until disappearance of tack is smaller, the curability is higher.

4) High-pressure mercury lamp: wavelength 200 to 450 nm, illuminance 500 $mW/cm^2$
5) UV-LED lamp: wavelength 385 nm, illuminance 500 $mW/cm^2$
6) UV-LED lamp: wavelength 405 nm, illuminance 500 $mW/cm^2$

++: tack disappeared in a cumulative amount of light of less than 5,000 $mJ/cm^2$.
+: tack disappeared by irradiation in a cumulative amount of light of 10,000 $mJ/cm^2$.
±: tack disappeared by irradiation in a cumulative amount of light of 50,000 $mJ/cm^2$.
-: tack remained by irradiation even in a cumulative amount of light of 50,000 $mJ/cm^2$.

<Light yellowing resistance>

A cured product of photoion-type was produced in the same manner as in curability evaluation of the photoionic polymerization-type curable composition except that curing conditions were changed as described below. The light yellowing resistance of the cured product obtained was evaluated in the same manner as in light yellowing resistance evaluation of the photoradical polymerization-type cured product. Curing conditions: UV-LED lamp: wavelength 405 nm, illuminance 1,000 $mW/cm^2$, irradiated in a cumulative amount of light of 100,000 $mJ/cm^2$, and thereafter left to stand still at 70°C for 1 hour.

<Durability>

A cured product was produced as in the photoionic polymerization-type curable composition except that the curing condition was changed to irradiation in a cumulative amount of light of 50,000 mJ/cm. The durability of the cured product obtained was evaluated in the same manner as in light yellowing resistance evaluation of the photoionic polymerization-type curable composition.

[Table 5]

| Photoionic polymerization sensitization-type active energy ray-curable composition | | Photosensitizer(D), (I) | Mass(%) | Photopolymerization initiator(E) | Mass(%) | Cyclic ether compound(h3) | Mass(%) | Other(k) | Mass(%) | Compatibility | Curability (light source) | | | Light yellowing resistance | Durability |
|---|---|---|---|---|---|---|---|---|---|---|---|---|---|---|---|
| | | | | | | | | | | | High-pressure mercury | LED 385 | LED 405 | | |
| Example 138 | F-80 | D-3 | 1 | E-10 | 5 | h3-1<br>h3-2 | 21<br>65 | k-1 | 8 | + | ++ | ++ | + | + | + |
| Example 139-1 | F-81-1 | D-6 | 2 | E-11 | 3 | h3-3<br>h3-4 | 40<br>55 | | | + | + | + | + | + | ± |
| Example 139-2 | F-81-2 | D-20 | 1 | E-10 | 5 | h3-3<br>h3-5 | 55<br>14 | k-2 | 25 | + | ++ | ++ | + | + | + |
| Example 140 | F-82 | D-55 | 2 | E-11 | 3 | h3-3<br>h3-4 | 40<br>55 | | | ++ | + | + | + | + | ++ |
| Example 141 | F-83 | D-56 | 10 | E-11 | 5 | h3-3<br>h3-5 | 50<br>10 | k-2 | 25 | ++ | ++ | ++ | ++ | ++ | ++ |
| Example 142 | F-84 | D-57 | 20 | E-10 | 5 | h3-3<br>h3-5 | 35<br>15 | k-2 | 25 | ++ | ++ | ++ | + | ++ | ++ |
| Example 143 | F-85 | D-58 | 74 | E-10 | 1 | | | k-2 | 25 | ++ | ++ | ++ | ++ | ++ | ++ |
| Comparative Example 11 | G-11 | | | E-10 | 5 | h3-1<br>h3-2 | 22<br>65 | k-1 | 8 | + | + | - | - | *3 | *3 |
| Comparative Example 12 | G-12 | I-3 | 1 | E-10 | 5 | h3-3<br>h3-5 | 55<br>14 | k-2 | 25 | + | + | + | ± | - | - |
| Comparative Example 13 | G-13 | I-3 | 10 | E-11 | 5 | h3-3<br>h3-5 | 50<br>10 | k-2 | 25 | + | ++ | ++ | + | - | - |
| Comparative Example 14 | G-14 | I-4 | 2 | E-11 | 3 | h3-3<br>h3-4 | 40<br>55 | | | ± | + | - | - | *3 | *3 |
| Comparative Example 15 | G-15 | I-5 | 20 | E-10 | 5 | h3-3<br>h3-5 | 35<br>15 | k-2 | 25 | ± | + | ± | ± | - | - |

(*3) No cured product obtained and no evaluation carried out.

Examples 144 to 151 and Comparative Examples 16 to 18 (preparation of photohybrid polymerization-type active energy ray-curable composition and evaluation of photosensitizer)

The benzoylformic acid amide derivative (D) obtained in each Example, and commercially available photosensitizers (I-3), (I-5) and commercially available photopolymerization initiator (E-5) for Comparative Examples were used, and unsaturated compounds (h1) and (h2), cyclic ether compound (h3) and photoionic polymerization initiator (E-10) or (E-11), and other additive (k) were weighed at each proportion shown in Table 6, to obtain photohybrid polymerization-type active energy ray-curable compositions and cured products by the same method as in the above evaluation of the photoionic polymerization-type photosensitizer. The compatibility and the curability of each of the curable compositions, and the light yellowing resistance and the durability of each of the cured products were evaluated by the same method as in the above evaluation of the photoionic polymerization-type photosensitizer. The results are shown in Table 6. As the curability of the curable composition is higher, the photosensitivity of D is higher.

[Table 6]

| Photohybrid polymerization-type active energy ray-curable composition | | Photopolymerization initiator-Photosensitizer (D), Photopolymerization initiator (E) or Photosensitizer (I) | Mass(%) | Unsaturated compound(h1), (h2) | Mass(%) | Alicyclic ether compound(h3) | Mass(%) | Other(k) | Mass(%) | Compatibility | Curability (light source) | | | Light yellowing resistance | Durability |
|---|---|---|---|---|---|---|---|---|---|---|---|---|---|---|---|
| | | | | | | | | | | | High-pressure mercury | LED 385 | LED 405 | | |
| Example 144 | F-86 | D-1<br>E-10 | 3<br>2 | h1-1<br>h2-7 | 30<br>25 | h3-2 | 30 | k-1 | 10 | + | ++ | + | + | + | + |
| Example 145 | F-87 | D-3<br>D-25<br>E-10 | 1<br>2<br>2 | h1-8<br>h2-6 | 15<br>50 | h3-3 | 25 | k-1 | 5 | + | ++ | ++ | + | + | + |
| Example 146 | F-88 | D-17<br>E-5<br>E-11 | 0.5<br>1<br>2 | h1-1<br>h2-13 | 40<br>20 | h3-3<br>h3-6 | 21.5<br>15 | | | + | ++ | + | + | ++ | + |
| Example 147 | F-89 | D-23<br>E-11 | 7<br>3 | h1-10<br>h2-2 | 20<br>30 | h3-4 | 30 | k-1 | 10 | ++ | + | + | + | + | + |
| Example 148 | F-90 | D-25<br>E-10 | 5<br>5 | h2-1<br>h2-13<br>h2-14 | 30<br>30<br>30 | | | | | + | ++ | ++ | + | + | + |
| Example 149 | F-91 | D-45<br>E-10 | 10<br>2 | h2-1<br>h2-13 | 30<br>30 | h3-6 | 25 | k-1 | 3 | ++ | ++ | ++ | + | + | + |
| Example 150 | F-92 | D-57<br>E-10 | 79<br>1 | h2-10 | 10 | h3-1<br>h3-6 | 5<br>5 | | | ++ | ++ | ++ | ++ | ++ | ++ |
| Example 151 | F-93 | D-58<br>E-11 | 20<br>1 | h1-2<br>h1-11 | 34<br>10 | h3-2<br>h3-6 | 25<br>10 | | | ++ | ++ | ++ | ++ | ++ | + |
| Comparative Example 16 | G-16 | E-1<br>E-11 | 7<br>3 | h1-10<br>h2-2 | 20<br>30 | h3-4 | 30 | k-1 | 10 | + | + | - | - | *3 | *3 |
| Comparative Example 17 | G-17 | I-3<br>E-5<br>E-10 | 1<br>2<br>2 | h1-10<br>h2-1 | 25<br>30 | h3-2 | 30 | k-1 | 10 | ++ | + | ± | ± | - | - |
| Comparative Example 18 | G-18 | I-5<br>E-10 | 10<br>2 | h2-1<br>h2-13 | 30<br>30 | h3-6 | 25 | k-1 | 3 | ± | + | ± | ± | - | ± |

(*3) No cured product obtained and no evaluation carried out.

As clear from the results in Table 4-1, Table 4-2, Table 5, and Table 6, the curable composition of each Example, with the benzoylformic acid amide derivative (D) as a photosensitizer, exhibited favorable compatibility. D exhibited photosensitivity to not only a continuous light beam in a wide range of wavelength from a high-pressure mercury lamp, but also light beams at wavelengths of 385 nm and 405 nm from an UV-LED lamp, and a curable composition containing D exhibited high curability. It was confirmed that D had high photosensitivity even if used in combination with any one or more photopolymerization initiators of a general-purpose photoradical-type polymerization initiator, a general-purpose photoionic photopolymerization initiator or a photoradical-type polymerization initiator. All of the photoradical-type, photoion-type and photohybrid-type curable compositions had high curability, and could each provide a cured product favorable in both light yellowing resistance and durability. It was clear that D-3 (Example 102), D-17 (Example 110), D-18 (Example 111), D-41 to D-43 (Example 127 to Example 129) each having a methoxy group were very high in photosensitive effect to light beams at 385 nm and 405 nm and an electron-donating methoxy group was contained to shift the absorption wavelength of D to a longer wavelength. D-55 to D-58 (Examples 140 to 143) each having a cyclic epoxy group exhibited photosensitivity to a photoionic polymerization initiator and could be simultaneously incorporated into a cured product due to photoionic polymerization, to allow a curable composition to be high in curability, thereby providing a cured product high

in durability. Examples 144, 145, and 147 to 151 each corresponded to a type of photohybrid polymerization of photoradical polymerization and photoionic polymerization, in which a photoionic polymerization initiator and D were used in combination, and these Examples also each provided a curable composition high in curability and allowed the resulting cured product to be favorable in light yellowing resistance and durability. In other words, it was confirmed that D had not only the effect in terms of a photoradical polymerization initiator, but also the effect in terms of a photosensitizer of photoionic polymerization. On the other hand, Comparative Examples 4 to 8, Comparative Example 11, and Comparative Example 16, in which no photosensitizer was used, each exhibited low sensitivity to light beams at wavelengths of 385 nm and 405 nm and provided a curable composition low in curability. In the case of Comparative Examples 9 and 10 in which isopropylthioxanthone (I-3) and 2-ethylanthraquinone (I-4) were respectively used as the photosensitizers, the curable compositions were favorable in curability, but the resulting cured products were low in both light yellowing resistance and durability. The same results as those of the radical type were also confirmed in Comparative Examples corresponding to the ion type and the hybrid type. In each of Comparative Example 15 and Comparative Example 18 in which high-molecular thioxanthone (I-5) was used, low photosensitivity was exhibited, a curable composition low in curability was provided, yellowing was observed in the resulting cured product, and the durability of the cured product was low, as compared with the case of use of low-molecular I-3.

Examples 152 to 161 and Comparatives Example 19 to 21 (active energy ray-curable ink compositions and evaluation thereof)

The benzoylformic acid amide derivative (D), curable composition (F) containing D, commercially available photopolymerization initiator (E), curable composition (G) containing E, monofunctionally unsaturated compound (h1), polyfunctionally unsaturated compound (h2) and other component (k) were weighed according to each proportion (in terms of solid content) described in Table 7, and mixed at 25°C for 30 minutes, to obtain each active energy ray-curable ink composition (hereinafter, also referred to as "ink composition".). The viscosity and the curability of the ink composition were evaluated by the following methods. The ink composition was used to perform ink-jet printing, and the ink ejection stability as printing suitability, and the blocking resistance, the clarity and the bleed-out resistance of a print product were evaluated by the following methods. Furthermore, the ink composition was used to produce a cured product for evaluation of the content rate of the low-molecular-weight component, and the content rate of the low-molecular-weight component in the cured product of the ink composition was evaluated by the same method as in evaluation of the content rate of the low-molecular-weight component in the cured product of the curable composition. These evaluation results are shown in Table 7.

<Viscosity>

The viscosity of the ink composition was measured with a cone plate type viscometer (RE550 type viscometer manufactured by Toki Sangyo Co., Ltd.), according to ISO 2884-1. The viscosity of the ink composition for ink-jet printing was evaluated on a 4-point scale as follows.

++: a viscosity of 5 mPa·s or more and less than 50 mPa·s.
+: a viscosity of 50 mPa·s or more and less than 100 mPa·s.
±: a viscosity of 100 mPa·s or more and less than 200 mPa·s.
-: a viscosity of 200 mPa·s or more.

<Method for producing print product by ultraviolet light irradiation>

The ink composition was applied by a bar coater to produce a coating film having a thickness of 20 μm on a PET film, and the coating film was cured by ultraviolet light irradiation (UV-LED lamp: wavelength 395 nm, illuminance 1,000 mW/cm$^2$), to produce a print product.

<Curability>

The cumulative amount of light, taken until complete curing (a state of no tackiness) of the ink composition in production of the print product, was measured, and the curability of the ink composition was evaluated according to the following criteria.

++: complete curing in a cumulative amount of light of less than 1,000 mJ/cm$^2$.
+: complete curing in a cumulative amount of light of 1,000 mJ/cm$^2$ or more and less than 2,000 mJ/cm$^2$
±: complete curing in a cumulative amount of light of 2,000 mJ/cm$^2$ or more and less than 5,000 mJ/cm$^2$.

-: a cumulative amount of light of 5,000 mJ/$cm^2$ or more needed for complete curing.

<Printing suitability>

The ink composition obtained was packed in an ink-jet printer (LuxelJet UV 350 GTW manufactured by FUJIFILM Holdings Corporation), a solid image was printed on coated paper , and the ejection stability of ink etc. was evaluated as printing suitability.

<Ejection stability>

A print state of the print product was visually observed, and the ejection stability was evaluated according to the following criteria.

++: no nozzle missing and favorable printing.
+: slight nozzle missing.
-: nozzle missing in a wide range.

<Blocking resistance>

The print product was left to stand still in an environment at a room temperature of 23°C and a relative humidity of 50% for 5 minutes, woodfree paper was overlapped on a printing surface, a load of 1 kg/$cm^2$ was applied for 1 minute, the transfer state of ink onto the paper was visually observed, and blocking resistance was evaluated according to the following criteria.

++: ink dried, and no transfer to the paper observed at all.
+: ink dried, and slight transfer to the paper observed.
±: ink substantially dried, and transfer to the paper observed.
-: almost no ink dried, and much transfer to the paper observed.

<Clarity>

The clarity of an image of a print product obtained from an ink composition with a pigment compounded was visually observed, and the clarity was evaluated according to the following criteria.

++: no ink bleeding observed at all, and a clear image.
+: almost no ink bleeding observed, and a favorable image.
-: ink bleeding observed.

<Bleed-out resistance>

The print product was left to stand still in a constant temperature and humidity chamber set to a temperature of 40°C and a relative humidity of 50%, for 168 hours, a surface of the print product was visually observed, and bleed-out resistance was evaluated according to the following criteria.

++: no bleed-out observed at all.
+: bleed-out slightly observed.
-: bleed-out remarkably observed.

As clear from the results in Table 7, the ink composition of each Example had high curability, the resulting cured film (print product) contained no low-molecular-weight component, the cured film was good in drying properties and less caused bleed-out, and a print product high in durability could be produced. On the other hand, the ink composition of Comparative Example 19 was low in curability. In Comparative Examples 20 and 21, curing could be made, but both the print products were high in content of the low-molecular-weight component, the cured films were inferior in blocking resistance and bleed-out resistance, and the print products were inferior in clarity. In Comparative Example 20 in which Esacure KIP 150 (E-1) was used, ink ejection stability was low and printing suitability was low. In each Example in which the pigment was compounded, a clear print product could be obtained. The reason for this was considered because the pigment was uniformly dispersed or uniformly dissolved in the ink composition of each Example. Furthermore, the ink composition of each Example was low in viscosity and was high in ejection stability, and was suitable for ink-jet printing.

[Table 7]

| Active energy ray-curable ink composition | | Curable composition (F) or Photopolymerization initiator (D), (E) | Mass(%) | Mono-functionally unsaturated compound(h1) | Mass(%) | Poly-functionally unsaturated compound(h2) | Mass(%) | Other(k) | Mass(%) | Viscosity | Curability | Content rate of low-molecular-weight component | Blocking resistance | Ejection stability | Clarity | Bleed-out resistance |
|---|---|---|---|---|---|---|---|---|---|---|---|---|---|---|---|---|
| Example 152 | F-94 | F-12 | 100 | | | | | | | ++ | + | + | + | ++ | *4 | + |
| Example 153 | F-95 | D-28 | 20 | h1-2 | 25 | h2-1 | 52.5 | k-3<br>k-4 | 0.8<br>1.7 | + | + | + | + | ++ | + | + |
| Example 154 | F-96 | F-19 | 97 | | | | | k-3<br>k-5 | 0.8<br>2.2 | + | ++ | ++ | ++ | + | ++ | ++ |
| Example 155 | F-97 | D-34 | 20 | h1-2 | 25 | h2-1 | 52.5 | k-3<br>k-4 | 0.8<br>1.7 | + | ++ | ++ | ++ | + | ++ | ++ |
| Example 156 | F-98 | F-27 | 90 | | | | | k-6 | 10 | ++ | + | + | + | + | ++ | + |
| Example 157 | F-99 | D-40 | 50 | h1-10 | 40 | | | k-6 | 10 | + | ++ | ++ | ++ | + | ++ | ++ |
| Example 158 | F-100 | D-48<br>E-3 | 0.5<br>0.5 | h1-1<br>h1-9 | 30<br>40 | h2-10<br>h2-16 | 15<br>11 | k-3<br>k-5 | 0.8<br>2.2 | ++ | + | + | + | ++ | + | + |
| Example 159 | F-101 | D-49 | 10 | h1-1<br>h1-16 | 10<br>40 | h2-13 | 30 | k-6 | 10 | ++ | ++ | ++ | ++ | ++ | ++ | ++ |
| Example 160 | F-102 | F-41 | 50 | | | h2-1 | 50 | | | + | ++ | ++ | + | + | *4 | ++ |
| Example 161 | F-103 | F-42 | 100 | | | | | k-7 | 0.1 | ++ | ++ | ++ | ++ | ++ | *4 | ++ |
| Comparative Example 19 | G-19 | G-1 | 60 | h1-10 | 40 | | | | | ++ | - | - | - | ++ | *4 | - |
| Comparative Example 20 | G-20 | E-1 | 20 | h1-2 | 25 | h2-1 | 52.5 | k-3<br>k-4 | 0.8<br>1.7 | + | + | - | - | - | - | - |
| Comparative Example 21 | G-21 | E-2 | 10 | h1-1<br>h1-16 | 10<br>40 | h2-13 | 30 | k-6 | 10 | ++ | + | - | ± | + | - | - |

(*4) Neither pigment, nor dye contained, and no evaluation carried out.

Examples 162 to 170 and Comparative Examples 22, 23 (preparation and evaluation of active energy ray-curable pressure-sensitive adhesive compositions)

The benzoylformic acid amide derivative (D), curable composition (F) containing D, commercially available photopolymerization initiator (E), monofunctionally unsaturated compound (h1), polyfunctionally unsaturated compound (h2) and other component (k) were weighed according to each proportion (in terms of solid content) described in Table 8, and mixed at 25°C for 30 minutes, to prepare each active energy ray-curable pressure-sensitive adhesive composition (hereinafter, also referred to as "pressure-sensitive adhesive composition".). The pressure-sensitive adhesive composition was used to produce a pressure-sensitive adhesive sheet having a pressure-sensitive adhesive layer by the following method, and the curability and the pressure-sensitive adhesive ability (pressure-sensitive adhesive force) to various substrates, of the pressure-sensitive adhesive composition, were evaluated. The content rate of the low-molecular-weight component, the transparency, the bleed-out resistance, the reworkability and the light yellowing resistance (the same method as for evaluating the light yellowing resistance of the curable composition) of the pressure-sensitive adhesive layer obtained (cured product of the pressure-sensitive adhesive composition) were evaluated. These evaluation results are shown in Table 8.

<Curability>

A heavy release film was closely contacted with a glass plate horizontally disposed, a spacer having a thickness of 1 mm and an interior of 60 mm × 100 mm was disposed, and the pressure-sensitive adhesive composition prepared in each of Examples and Comparative Examples was packed in the inside of the spacer. A light release film was overlapped on the composition packed, and irradiated by an UV-LED lamp at a wavelength of 405 nm and an illuminance of 100 mW/cm$^2$ so that the cumulative amount of light was 1,000 mJ/cm$^2$, to cure the pressure-sensitive adhesive composition. Thereafter, the light release film was released, to obtain a pressure-sensitive adhesive sheet including a cured product (pressure-sensitive adhesive layer) of the pressure-sensitive adhesive composition, and the heavy release film. Touch with the pressure-sensitive adhesive layer was performed, and the curability of the pressure-sensitive adhesive composition was evaluated according to the following criteria.

++: form-retainable cured product obtained, and no liquid residues observed at all.
+: form-retainable cured product obtained, and slight liquid residues observed.
±: form-retainable cured product obtained, and liquid attachment observed.
-: insufficient curing, and no form-retainable cured product obtained.

<Content rate of low-molecular-weight component>

The content rate of the low-molecular-weight component in the pressure-sensitive adhesive layer obtained was evaluated in the same manner as in evaluation of the content rate of the low-molecular-weight component in the cured product of the curable composition.

<Transparency>

The pressure-sensitive adhesive layer was transferred from the pressure-sensitive adhesive sheet obtained, to a glass plate, under an environment of a temperature of 23°C and a relative humidity of 50%, and the total light transmittance of the glass plate and the pressure-sensitive adhesive layer was measured with a haze meter (NDH-8000 manufactured by Nippon Denshoku Industries Co., Ltd.) according to ISO 14782. Thereafter, the transmittance of a glass plate was measured in the same manner and subtracted from the total light transmittance of the glass plate and the pressure-sensitive adhesive layer, to calculate the transmittance of the pressure-sensitive adhesive layer itself, and the transparency of the pressure-sensitive adhesive layer was evaluated according to the following criteria.

++: a transmittance of 90% or more.
+: a transmittance of 85% or more and less than 90%.
±: a transmittance of 50% or more and less than 85%.
-: a transmittance of less than 50%.

<Bleed-out resistance>

The pressure-sensitive adhesive sheet obtained was left to stand still in a constant temperature and humidity chamber at a temperature of 40°C and a relative humidity of 50% for 168 hours. Thereafter, the sheet was left to stand under an environment of a temperature of 23°C and a relative humidity of 50% for 30 minutes, and touch with the pressure-sensitive adhesive layer on a surface of the pressure-sensitive adhesive sheet was performed, and the bleed-out resistance of the pressure-sensitive adhesive layer was evaluated according to the following criteria.

++: no liquid residues observed at all, and no bleed-out observed at all.
+: extremely slight liquid residues observed, and bleed-out extremely slightly observed.
±: slight liquid attachment observed, and bleed-out slightly observed.
-: liquid attachment observed, and bleed-out observed.

<Pressure-sensitive adhesive force>

The pressure-sensitive adhesive layer was transferred from the pressure-sensitive adhesive sheet obtained, to the following substrate film or plate, under an environment of a temperature of 23°C and a relative humidity of 50%, pressure-bonded by two reciprocation operations with a pressing roller having a weight of 2 kg, and left to stand under the same environment for 30 minutes. Thereafter, a tensile tester (Tensilon RTA-100 manufactured by ORIENTEC, hereinafter, also referred to as "universal tester".) was used to measure the 180-degree peeling strength (N/25 mm) (peeling

rate 300 mm/min) according to ISO 29862, and the pressure-sensitive adhesive force was evaluated according to the following criteria.

PET2: polyethylene terephthalate film (Cosmoshine A4160, corona-treated, manufactured by Toyobo Co., Ltd.)
PC: polycarbonate (plate) (PC1600, manufactured by C.I. TAKIRON Corporation)
GL: glass (plate) (Eagle XG, manufactured by Corning Incorporated)
++: a peeling strength of 20 (N/25 mm) or more.
+: a peeling strength of 10 (N/25 mm) or more and less than 20 (N/25 mm).
±: a peeling strength of 5 (N/25 mm) or more and less than 10 (N/25 mm).
-: a peeling strength of less than 5 (N/25 mm).

<Reworkability>

In the same manner as in the evaluation of pressure-sensitive adhesive force, the pressure-sensitive adhesive layer was transferred to a film or plate made of a different material, and the films or plates were bonded together by pressure, and then left for 24 hours in a constant temperature chamber at 80°C. Thereafter, the resultant was left to stand under an environment of a temperature of 23°C and a relative humidity of 50% for 30 minutes, the pressure-sensitive adhesive layer was released, the residue state of the pressure-sensitive adhesive layer (glue) on a surface of the substrate was visually observed, and the reworkability of the pressure-sensitive adhesive layer was evaluated according to the following criteria.

++: no glue residue.
+: extremely slight glue residue.
±: slight glue residue.
-: glue residue.

<Durability>

The pressure-sensitive adhesive layer of the pressure-sensitive adhesive sheet was transferred to a glass plate, and left to stand still in a constant temperature and humidity chamber at a temperature of 85°C and a relative humidity of 85% for 100 hours. Thereafter, the resultant was left to stand under an environment of a temperature of 23°C and a relative humidity of 50% for 30 minutes, the state of the pressure-sensitive adhesive layer was visually observed, and durability was evaluated according to the following criteria.

++: transparent pressure-sensitive adhesive layer, and neither floating, nor air bubbles observed.
+: slightly clouded pressure-sensitive adhesive layer, but neither floating, nor air bubbles observed.
-: clouded pressure-sensitive adhesive layer, or floating and air bubbles observed.

[Table 8]

| Active energy ray-curable pressure-sensitive adhesive composition | | Curable composition (F) or Photopolymerization initiator (D), (E) | Mass(%) | Mono-functionally unsaturated compound(h1) | Mass(%) | Poly-functionally unsaturated compound(h2) | Mass(%) | Other(k) | Mass(%) | Curability | Content rate of low-molecular-weight component | Transparency | Bleed-out resistance | Pressure-sensitive adhesion force | | | Reworkability | | | Durability | Light yellowing resistance |
|---|---|---|---|---|---|---|---|---|---|---|---|---|---|---|---|---|---|---|---|---|---|
| | | | | | | | | | | | | | | PET2 | PC | GL | PET2 | PC | GL | | |
| Example 162 | F-104 | D-24 | 30 | h1-2<br>h1-4<br>h1-10 | 20<br>10<br>20 | h2-5 | 20 | | | + | + | + | + | + | + | + | + | + | + | + | + |
| Example 163 | F-105 | D-26 | 10 | h1-1<br>h1-12<br>h1-15 | 30<br>25<br>5 | h2-3 | 30 | | | + | + | + | + | + | + | + | + | + | + | + | + |
| Example 164 | F-106 | D-27 | 10 | h1-1<br>h1-12<br>h1-15 | 30<br>25<br>5 | h2-3 | 30 | | | ++ | ++ | + | ++ | ++ | + | + | ++ | + | + | + | + |
| Example 165 | F-107 | D-35 | 10 | h1-1<br>h1-7<br>h1-8<br>h1-10 | 10<br>25<br>5<br>40 | h2-6 | 10 | | | + | + | + | ++ | + | + | + | + | + | + | + | + |
| Example 166 | F-108 | D-36 | 20 | h1-4<br>h1-10 | 5<br>50 | h2-4 | 25 | | | ++ | ++ | ++ | ++ | ++ | ++ | ++ | + | + | + | ++ | + |
| Example 167 | F-109 | F-29 | 100 | | | | | | | ++ | + | + | + | ++ | + | + | ++ | + | + | + | + |
| Example 168 | F-110 | D-46 | 5 | h1-2<br>h1-8<br>h1-17 | 10<br>5<br>40 | h2-11 | 5 | k-8 | 35 | ++ | + | + | ++ | ++ | ++ | ++ | ++ | ++ | ++ | + | + |
| Example 169 | F-111 | D-53 | 50 | h1-4<br>h1-9<br>h1-10 | 5<br>15<br>30 | | | | | ++ | ++ | + | ++ | ++ | ++ | ++ | ++ | ++ | + | ++ | + |
| Example 170 | F-112 | D-54<br>E-5 | 0.5<br>1.5 | h1-4<br>h1-9<br>h1-10 | 5<br>15<br>30 | h2-6 | 48 | | | + | + | + | + | + | + | + | + | + | + | + | + |
| Comparative Example 22 | G-22 | E-1 | 20 | h1-4<br>h1-10 | 5<br>50 | h2-4 | 25 | | | ± | - | + | - | - | ± | - | - | ± | - | - | ± |
| Comparative Example 23 | G-23 | E-2 | 5 | h1-2<br>h1-8<br>h1-17 | 10<br>5<br>40 | h2-11 | 5 | k-8 | 35 | ± | - | + | - | ± | + | - | ± | ± | - | - | + |

As clear from the results in Table 8, the pressure-sensitive adhesive composition of each Example had high curability, and a pressure-sensitive adhesive layer obtained by curing the pressure-sensitive adhesive composition was high in transparency, and high in pressure-sensitive adhesive ability (pressure-sensitive adhesive force) to various substrates. The cured product obtained in each Example (pressure-sensitive adhesive layer) was low in content rate of the low-molecular-weight component, high in bleed-out resistance, durability and light yellowing resistance, and also favorable in reworkability in release of the cured product from the plate. On the other hand, the pressure-sensitive adhesive composition of each Comparative Example was low in curability, the content of the low-molecular-weight component in the resulting cured product was high, the pressure-sensitive adhesive layer was low in pressure-sensitive adhesive force, and all of bleed-out resistance, durability, light yellowing resistance and reworkability were low.

Examples 171 to 177, and Comparative Examples 24 and 25 (preparation and evaluation of active energy ray-curable adhesive compositions)

The benzoylformic acid amide derivative (D), curable composition (F) containing D, commercially available photopolymerization initiator (E), monofunctionally unsaturated compound (h1), polyfunctionally unsaturated compound (h2) and other component (k) were weighed according to each proportion (in terms of solid content) described in Table 9, and mixed at 25°C for 30 minutes, to prepare each active energy ray-curable adhesive composition (hereinafter, also referred to as "adhesive composition".). The curability of the adhesive composition, and the content rate of the low-molecular-weight component in the resulting cured product were evaluated. The adhesive composition was used for adhesive to various substrates, to produce a laminate, and the adhesive force and the durability of the laminate were evaluated. These evaluation results are shown in Table 9.

<Curability>

A PET film was closely contacted on a glass plate horizontally disposed, the adhesive composition of each of Examples and Comparative Examples was applied thereto at a thickness of 20 μm, by a bar coater, and a light release film was overlapped thereon and irradiated with ultraviolet light from an UV-LED lamp at a wavelength of 405 nm and an illuminance of 50 mW/cm$^2$, to cure the adhesive composition. Thereafter, the light release film was removed, the presence of tack on a surface of the cured film was confirmed, and the curability of the adhesive composition was evaluated based on the cumulative amount of light, taken until disappearance of tack, according to the following criteria.

++: tack disappeared in a cumulative amount of light of less than 500 mJ/cm$^2$.
+: tack disappeared in a cumulative amount of light of 500 mJ/cm$^2$ or more and less than 1,000 mJ/cm$^2$.
±: tack disappeared in a cumulative amount of light of 1,000 mJ/cm$^2$ or more and less than 5,000 mJ/cm$^2$.
-: tack remained even in a cumulative amount of light of 5,000 mJ/cm$^2$.

<Production of laminate>

The adhesive composition was applied onto various film-shaped or plate-shaped substrates (films or plates) described below, and the resultant was bonded with a PET film so that the thickness of an adhesive layer was 20 μm, by use of a desktop type roll laminator machine (RSL-382S) so that air bubbles were not entrained, and irradiated with ultraviolet light (UV-LED lamp of a wavelength 405 nm and an illuminance of 50 mW/cm$^2$, cumulative amount of light: 2,000 mJ/cm$^2$), to produce a laminate.

Substrate (film or plate)
PET3: polyethylene terephthalate film (Cosmoshine E5100, corona-treated, manufactured by Toyobo Co., Ltd.)
PMMA: polymethyl methacrylate (plate) (Comoglas P, manufactured by Kuraray Co., Ltd.)
PC: polycarbonate (plate) (PC1600, manufactured by C.I. TAKIRON Corporation)

<Adhesive force>

The universal tester was used to measure the 180-degree peeling strength (N/25 mm) (peeling rate 300 mm/min) of the laminate according to ISO 29862, and the adhesive force was evaluated according to the following criteria.

++: a peeling strength of 20 (N/25 mm) or more.
+: a peeling strength of 10 (N/25 mm) or more and less than 20 (N/25 mm).
±: a peeling strength of 5 (N/25 mm) or more and less than 10 (N/25 mm).
-: a peeling strength of less than 5 (N/25 mm).

<Durability>

A cured product (adhesive layer) was produced on a glass plate (cumulative amount of light 2,000 mJ/cm$^2$) in the same manner as in curability evaluation of the adhesive composition, and left to stand still in a constant temperature and humidity chamber at a temperature of 85°C and a relative humidity of 85% for 100 hours. Thereafter, the resultant was left to stand under an environment of a temperature of 23°C and a relative humidity of 50% for 30 minutes, the state of the laminate was visually observed, and durability was evaluated according to the following criteria.

++: transparent laminate, and neither peeling, nor air bubbles caused.
+: extremely slightly clouded laminate, but neither peeling, nor air bubbles caused.
±: slightly clouded laminate, or peeling and air bubbles caused.
-: extremely clouded laminate, or peeling and air bubbles caused.

<Content rate of low-molecular-weight component>

A cured product (adhesive layer) was produced on a PET film in the same manner as in curability evaluation of the adhesive composition (UV-LED at a wavelength 405 nm and an illuminance of 50 mW/cm$^2$, cumulative amount of light: 2,000 mJ/cm$^2$), the resulting PET film having the adhesive layer was cut out to a test piece of a size of 5 cm$^2$, and the content rate of the low-molecular-weight component in the adhesive layer was evaluated in the same manner as in evaluation of the content rate of the low-molecular-weight component in the cured product of the curable composition.

[Table 9]

| Active energy ray-curable adhesive composition | | Curable composition (F) or Photopolymerization initiator (D), (E) | Mass(%) | Mono-functionally unsaturated compound(h1) | Mass(%) | Poly-functionally unsaturated compound(h2) | Mass(%) | Photosensitizer(I) or Other(k) | Mass(%) | Curability | Adhesion force | | | Durability | Content rate of low-molecular-weight component |
|---|---|---|---|---|---|---|---|---|---|---|---|---|---|---|---|
| | | | | | | | | | | | PET3 /PET | PMMA /PET | PC /PET | | |
| Example 171 | F-113 | F-10 | 50 | h1-4 | 20 | h2-9 | 25 | I-1 | 5 | + | + | + | + | + | + |
| Example 172 | F-114 | D-29 | 35 | h1-1<br>h1-4 | 45<br>10 | h2-4 | 5 | k-9 | 5 | + | + | + | + | + | + |
| Example 173 | F-115 | D-30 | 20 | h1-2<br>h1-10 | 40<br>20 | h2-7 | 20 | | | + | + | + | + | + | + |
| Example 174 | F-116 | D-35<br>E-4 | 0.5<br>19.5 | h1-4<br>h1-14 | 10<br>60 | h2-10 | 10 | | | + | + | + | + | ++ | ++ |
| Example 175 | F-117 | D-38 | 20 | h1-1<br>h1-2 | 20<br>40 | h2-2 | 20 | | | ++ | ++ | ++ | ++ | ++ | ++ |
| Example 176 | F-118 | D-39 | 45 | h1-2 | 55 | | | | | + | ++ | ++ | ++ | + | + |
| Example 177 | F-119 | D-51 | 5 | h1-1<br>h1-4 | 50<br>20 | h2-9 | 25 | | | + | ++ | ++ | ++ | ++ | ++ |
| Comparative Example 24 | G-24 | E-1 | 20 | h1-1<br>h1-2 | 20<br>40 | h2-2 | 20 | | | ± | ± | ± | ± | - | - |
| Comparative Example 25 | G-25 | E-2 | 5 | h1-1<br>h1-4 | 50<br>20 | h2-9 | 25 | | | ± | + | ± | ± | ± | - |

As clear from the results in Table 9, the adhesive composition of each Example had high curability, and a laminate (adhesive product) obtained by curing the adhesive composition was high in adhesive force to not only the same type of substrate, but also a different type of substrate. The content rate of the low-molecular-weight component in the cured product (adhesive layer) was low, and the durability of the laminate was favorable. Such an adhesive composition exhibited properties suited to an adhesive. On the other hand, the adhesive composition of each Comparative Example was low in curability, the low-molecular-weight component considerably remained in the adhesive layer, and also the adhesive force of the adhesive product was also low in durability.

Examples 178 to 184, and Comparative Examples 26 and 27 (preparation and evaluation of active energy ray-curable sealant compositions)

The benzoylformic acid amide derivative (D), commercially available photopolymerization initiator (E), monofunctionally unsaturated compound (h1), polyfunctionally unsaturated compound (h2) and other component (k) were weighed according to each proportion (in terms of solid content) described in Table 10, and mixed at 25°C for 30 minutes, to prepare each active energy ray-curable sealant composition (hereinafter, also referred to as "sealant composition".). A cured product of the sealant composition was produced, and the curability of the sealant composition, and the transparency, the moist heat yellowing resistance, the water resistance, the outgas resistance, the heat cycle resistance and the corrosion resistance of the cured product obtained were evaluated. The content rate of the low-molecular-weight component in the cured product of the sealant composition was evaluated in the same manner as in that in the cured product of the curable composition. These evaluation results are shown in Table 10.

<Production of cured product of sealant composition>

A spacer (30 mm × 15 mm × 3 mm) was mounted on a glass plate, copper foil (5 mm length × 50 mm width × 80 μm thickness) was placed in the spacer, and the sealant composition prepared was injected thereinto. After sufficient degassing, ultraviolet light (UV-LED lamp at a wavelength 405 nm and an illuminance of 500 mW/cm$^2$, cumulative amount of light: 1,000 mJ/cm$^2$) was radiated, to obtain a cured product of the sealant composition.

<Curability>

The cured product was subjected to evaluation according to the following criteria, and curability thereof was thus evaluated.

++: form-retainable cured product obtained, and no tack observed during touch with cured product.
+: form-retainable cured product obtained, and tack observed during touch with cured product.
±: form-retainable cured product obtained, but liquid residues observed during touch with cured product.
-: insufficiently cured, and no form-retainable cured product obtained.

<Transparency>

After the cured product was allowed to stand still under an environment of a temperature of 23°C and a relative humidity of 50% for 24 hours, the transmittance of the cured product was measured with the same haze meter as that described above, and transparency was evaluated according to the following criteria.

++: a transmittance of 90% or more.
+: a transmittance of 85% or more and less than 90%.
±: a transmittance of 50% or more and less than 85%.
-: a transmittance of less than 50%.

<Moist heat yellowing resistance>

After the cured product was left to stand still under an environment of a temperature of 23°C and a relative humidity of 50% for 24 hours, a transmission spectrum of the cured product was measured with a specialized machine for Transmission Colormeter (TZ-6000 manufactured by Nippon Denshoku Industries Co., Ltd.), and defined as the initial b value. Thereafter, the cured product was left to stand still in a constant temperature and humidity machine set to a temperature of 85°C and a relative humidity of 85% for 500 hours, and an acceleration test of moist heat yellowing resistance was performed. The cured product after the test was left to stand still under an environment of a temperature of 23°C and a relative humidity of 50% for 24 hours, transmission color measurement was made, and the post-moist-heat b value was determined. The difference between the post-moist-heat b value and the initial b value was defined as the changed value Δb (Δb = post-moist-heat b value - initial b value). The moist heat yellowing resistance of the cured product was evaluated according to the following criteria.

++: both initial b value and post-moist-heat b value being 0.2 or less, and Δb being 0.1 or less.
+: any one or more of initial b value and post-moist-heat b value being more than 0.2, but both the values being 0.5 or less and Δb being 0.2 or less.
±: any one or more of initial b value and post-moist-heat b value being more than 0.5, but both the values being 1.0 or less and Δb being 0.3 or less.
-: any one or more of initial b value and post-moist-heat b value being more than 1.0, or Δb being more than 0.3.

<Water resistance>

A test piece was obtained by cutting out 1 g of the cured product and left to stand still in a constant temperature and humidity machine at a temperature of 85°C and a relative humidity of 95% for 48 hours, and thereafter the weight of the test piece was again measured. The rate of water absorption was calculated by the following expression, and water resistance was evaluated according to the following criteria. As the rate of water absorption is lower, the water resistance of the cured product is higher.

Rate of water absorption (%) = (Weight after water absorption - Weight before water absorption)/Weight before water absorption × 100%

++: a rate of water absorption of less than 1.0%.
+: a rate of water absorption of 1.0% or more and less than 2.0%.
±: a rate of water absorption of 2.0% or more and less than 3.0%.
-: a rate of water absorption of 3.0% or more.

<Outgas resistance>

A test piece was obtained by cutting out 1 g of the cured product and left to stand still in a constant temperature chamber at a set temperature of 100°C, a dry nitrogen stream was allowed to flow for 24 hours, and thereafter the weight of the test piece was again measured. The rate of outgas generation was calculated by the following expression, and outgas resistance was evaluated according to the following criteria. As the rate of outgas generation is lower, outgas resistance is higher.

Rate of outgas generation (%) = (Weight after test - Weight before test)/Weight before test × 100%

++: a rate of outgas generation of less than 0.1%.
+: a rate of outgas generation of 0.1% or more and less than 0.2%.
±: a rate of outgas generation of 0.2% or more and less than 0.3%.
-: a rate of outgas generation of 0.3% or more.

<Heat cycle resistance>

The cured product was treated repeatedly for 100 cycles with, as one heat cycle, a treatment where it was left to stand at -40°C for 30 minutes and then left to stand at 100°C for 30 minutes. Thereafter, the cured product was visually observed, and heat cycle resistance was evaluated according to the following criteria.

++: no change observed at all.
+: slight generation of air bubbles observed, but neither clouding, nor generation of cracking observed.
±: modest generation of air bubbles or cracking observed, and slight clouding observed.
-: air bubbles or cracking entirely generated, and translucence state.

<Corrosion resistance>

After the moist heat yellowing resistance test, a surface of the copper foil was visually observed, and the corrosion resistance of the cured product was evaluated according to the following criteria. As the corrosion of the copper foil is less, the cured product is lower in metal corrosivity and the cured product is higher in corrosion resistance.

++: no corrosion on copper foil in cured product.
+: slight corrosion on copper foil in cured product.
±: small corrosion on copper foil in cured product.
-: extreme corrosion on copper foil in cured product.

[Table 10]

| Active energy ray-curable sealant composition | | Photopolymerization initiator(D), (E) | Mass(%) | Mono-functionally unsaturated compound(h1) | Mass(%) | Poly-functionally unsaturated compound(h2) | Mass(%) | Other(k) | Mass(%) | Curability | Transparency | Moist heat yellowing resistance | Water resistance | Outgas resistance | Heat cycle resistance | Corrosion resistance | Content rate of low-molecular-weight component |
|---|---|---|---|---|---|---|---|---|---|---|---|---|---|---|---|---|---|
| Example 178 | F-120 | D-24 | 20 | h1-9<br>h1-10 | 30<br>10 | h2-3 | 40 | | | ++ | + | + | + | + | + | + | + |
| Example 179 | F-121 | D-38 | 30 | h1-1<br>h1-2 | 10<br>30 | h2-15 | 30 | | | ++ | ++ | ++ | + | ++ | ++ | ++ | ++ |
| Example 180 | F-122 | D-39<br>E-3 | 0.5<br>0.5 | h1-6<br>h1-9 | 30<br>20 | h2-7<br>h2-15 | 5<br>40 | k-12 | 4 | + | ++ | + | + | + | + | + | + |
| Example 181 | F-123 | D-40 | 5 | h1-3<br>h1-4<br>h1-6 | 15<br>10<br>50 | h2-8 | 20 | | | + | ++ | ++ | ++ | + | + | + | + |
| Example 182 | F-124 | D-42 | 10 | h1-2<br>h1-4 | 20<br>20 | h2-6<br>h2-15 | 30<br>20 | | | + | ++ | + | + | + | + | + | + |
| Example 183 | F-125 | D-50 | 20 | h1-6<br>h1-8 | 30<br>20 | h2-1 | 20 | k-11 | 10 | ++ | + | ++ | ++ | + | ++ | ++ | ++ |
| Example 184 | F-126 | D-54 | 50 | h1-12 | 20 | h2-7<br>h2-13 | 10<br>20 | | | ++ | + | + | ++ | ++ | ++ | ++ | ++ |
| Comparative Example 26 | G-26 | E-1 | 20 | h1-2<br>h1-4 | 20<br>20 | h2-6<br>h2-15 | 20<br>20 | | | ± | - | ± | - | - | - | - | - |
| Comparative Example 27 | G-27 | E-3 | 10 | h1-6<br>h1-8 | 30<br>20 | h2-1 | 30 | k-11 | 10 | ++ | + | - | + | ± | ± | - | - |

As clear from the results in Table 10, the sealant composition of each Example had high curability, the content rate of the low-molecular-weight component in the resulting cured product (sealant) was low, the cured product was high in transparency, moist heat yellowing resistance, and water resistance, less caused outgas generation, and was favorable in heat cycle resistance and corrosion resistance. On the other hand, the sealant composition of each Comparative Example was low in curability, the low-molecular-weight component considerably remained in the sealant, and the sealant could not satisfy any two or more of physical properties including transparency, moist heat yellowing resistance, water resistance, outgas resistance, heat cycle resistance and corrosion resistance.

Examples 185 to 192, and Comparative Examples 28 and 29 (preparation and evaluation of active energy ray-curable coating agent compositions)

The benzoylformic acid amide derivative (D), commercially available photopolymerization initiator (E), monofunctionally unsaturated compound (h1), polyfunctionally unsaturated compound (h2) and other component (k) were weighed according to each proportion (in terms of solid content) described in Table 11, and mixed at 25°C for 30 minutes, to prepare each active energy ray-curable coating agent composition (hereinafter, also referred to as "coating agent composition".). The coating agent composition was used to produce a coat layer by the following method, and the curability of the coating agent composition, the adhesion, the light yellowing resistance, the bending resistance, the chemical resistance, the scratch resistance and the durability of the coat layer obtained, and the content rate of the low-molecular-weight component in the coat layer were evaluated. The results are shown in Table 11.

<Curability>

The curability of the coating agent composition was evaluated by the same method and evaluation criteria as in the adhesive composition.

<Production of coat layer>

A PET film was closely contacted onto a glass plate horizontally disposed, and the coating agent composition of each of Examples and Comparative Examples was applied at a thickness of 5 μm by a bar coater and irradiated at an illuminance of 500 mW/cm$^2$ and in a cumulative amount of light 2,000 mJ/cm$^2$ with ultraviolet light from an UV-LED lamp at a wavelength of 385 nm under a nitrogen atmosphere, to produce a coat layer on the PET film.

<Adhesion evaluation>

A surface of the coat layer was nicked by a cutter knife according to the crosscut method described in ISO 2409, to produce 100 cells of 1 mm × 1 mm, thereby providing a test piece. A commercially available cellophane tape was pasted on and then released from the test piece, the number of cells remaining on the test piece was counted, and adhesion was evaluated according to the following criteria.

++: 100 cells remained.
+: 90 or more and 99 or less cells remained.
-: 89 or less cells remained.

<Light yellowing resistance>

The light yellowing resistance of the coat layer was evaluated by irradiating the coat layer with ultraviolet light by use of an UV-LED (wavelength 405 nm, illuminance 100 mW/cm$^2$, cumulative amount of light 20,000 mJ/cm$^2$), in the same manner as in light yellowing resistance evaluation of the cured product of the curable composition.

<Bending resistance>

According to the cylindrical mandrel method described in ISO 1519, bending was made under contact with a mandrel (10 mmφ) so that the coat layer was located outside. Thereafter, the coat layer was visually observed, and bending resistance was evaluated according to the following criteria.

++: bending portion not whitened and broken.
+: bending portion partially whitened.
±: bending portion partially broken.
-: bending portion broken.

<Chemical resistance>

The coat layer produced was used, oleic acid was applied to a surface of the coat layer so that the diameter was about 1 cm, the resultant was retained at 23°C for 1 hour and then washed off with a neutral detergent, the state of the surface was visually observed, and chemical resistance was evaluated according to the following criteria.

++: no oleic acid mark observed at all.
+: extremely light whitened mark observed on oleic acid-applied portion.
±: oleic acid-applied portion whitened, and swelling of surface observed.
-: tacky oleic acid-applied portion, and peeling of surface observed.

<Scratch resistance>

A surface of the coat layer was scratched by ten reciprocation operations with steel wool (#0000, a load of 100 g) under an environment at a room temperature of 23°C and a humidity of 50%, the surface of the coat layer was visually observed, and scratch resistance was evaluated according to the following criteria.

++: no generation of scratch of coat layer observed.
+: slightly fine scratch observed partially on coat layer.
±: streaky scratch observed fully on coat layer.
-: peeling of coat layer observed.

<Durability>

The durability of the coat layer was evaluated by the same method as in durability evaluation of the cured product of the adhesive composition.

<Content rate of low-molecular-weight component>

The content rate of the low-molecular-weight component in the coat layer was evaluated by the same method as in evaluation of the content rate of the low-molecular-weight component in the cured product of the curable composition except that the light source was an UV-LED lamp at a wavelength of 385 nm and an illuminance of 1,000 mW/cm$^2$ and the cumulative amount of light was 10,000 mJ/cm$^2$.

[Table 11]

| Active energy ray-curable coating agent composition | | Photopolymerization initiator(D), (E) | Mass(%) | Mono-functionally unsaturated compound (h1) | Mass(%) | Poly-functionally unsaturated compound (h2) | Mass(%) | Curability | Adhesion | Light yellowing resistance | Bending resistance | Chemical resistance | Scratchresistance | Durability | Content rate of low-molecular-weight component |
|---|---|---|---|---|---|---|---|---|---|---|---|---|---|---|---|
| Example 185 | F-127 | D-29 | 50 | h1-1 | 20 | h2-10 | 30 | + | + | + | + | + | + | + | + |
| Example 186 | F-128 | D-37 | 10 | h1-1 | 30 | h2-1<br>h2-8 | 40<br>20 | + | + | ++ | ++ | + | ++ | + | + |
| Example 187 | F-129 | D-38 | 20 | h1-1 | 40 | h2-8 | 40 | ++ | ++ | + | + | ++ | ++ | ++ | ++ |
| Example 188 | F-130 | D-39 | 20 | h1-1 | 20 | h2-1<br>h2-8 | 40<br>20 | ++ | + | + | + | + | ++ | ++ | + |
| Example 189 | F-131 | D-48<br>E-2 | 0.5<br>0.5 | h1-6<br>h1-10 | 19<br>10 | h2-1<br>h2-11 | 40<br>30 | + | + | ++ | + | + | + | + | + |
| Example 190 | F-132 | D-50 | 20 | h1-1<br>h1-16 | 30<br>40 | h2-2 | 10 | ++ | ++ | + | ++ | ++ | ++ | ++ | ++ |
| Example 191 | F-133 | D-51 | 40 | h1-4<br>h1-14 | 10<br>35 | h2-10 | 15 | ++ | ++ | ++ | ++ | ++ | ++ | ++ | ++ |
| Example 192 | F-134 | D-52 | 50 | h1-1 | 20 | h2-1<br>h2-8 | 15<br>15 | ++ | ++ | + | + | ++ | ++ | ++ | ++ |
| Comparative Example 28 | G-28 | E-1 | 20 | h1-1<br>h1-16 | 30<br>40 | h2-2 | 10 | ± | - | ± | ++ | ± | - | - | - |
| Comparative Example 29 | G-29 | E-2 | 10 | h1-1 | 30 | h2-1<br>h2-8 | 40<br>20 | ± | + | + | + | - | ± | ± | - |

As clear from the results in Table 11, the coating agent composition of each Example had high curability with a long-wavelength light beam, and the resulting cured product (coat layer) had favorable adhesion, light yellowing resistance, bending resistance, chemical resistance and durability. The coating agent composition had properties suited to coat agents for vehicles, and indoor and outdoor, and coat agents for decoration. On the other hand, the coating agent composition of each Comparative Example was low in curability with long-wavelength light beam, the low-molecular-weight component considerably remained in the coat layer obtained, and the coat layer was inferior in various physical properties.

Examples 193 to 202, and Comparative Examples 30 and 31 (preparation and evaluation of active energy ray-curable three-dimensional modeling ink compositions)

The benzoylformic acid amide derivative (D), curable composition (F) containing D, commercially available photopolymerization initiator (E), monofunctionally unsaturated compound (h1) and polyfunctionally unsaturated compound (h2) were weighed according to each proportion (in terms of solid content) described in Table 12, and mixed at 25°C for 30 minutes, to prepare each active energy ray-curable three-dimensional modeling ink composition (hereinafter, also referred to as "three-dimensional modeling ink composition".). The viscosity and the curability of the three-dimensional modeling ink composition were evaluated. A three-dimensionally modelled product was produced by the following modeling method, and curing shrinkage resistance and the content rate of the low-molecular-weight component in a modelled product were evaluated. The strength, the heat resistance, the modeling accuracy, the light yellowing resistance, and the bleed-out resistance of the modelled product obtained were evaluated. These evaluation results are shown in Table 12.

<Viscosity>

The viscosity of the three-dimensional modeling ink composition was measured with a cone plate type viscometer (RE550 type viscometer), according to ISO 2884-1, and was evaluated according to the following criteria.

++: a viscosity of 5 mPa·s or more and less than 500 mPa·s.
+: a viscosity of 500 mPa·s or more and less than 2,000 mPa·s.
-: a viscosity of 2,000 mPa·s or more.

<Curability>

The curability of the three-dimensional modeling ink composition was evaluated by the same method as in the curable composition except that the light source was an UV-LED lamp at a wavelength of 405 nm and an illuminance of 5 $mW/cm^2$.

<Production of modelled product>

A heavy release film was closely contacted with a glass plate horizontally disposed, and a spacer having an interior size of 6 mm × 60 mm × 60 mm was disposed thereon. The three-dimensional modeling ink composition of each of Examples and Comparative Examples was packed in the spacer so that a layer having a thickness of 0.3 mm was formed. The resultant was left to stand still in a constant temperature machine at a temperature of 60°C for 1 minute, and then irradiated with ultraviolet light (wavelength 405 nm, illuminance 5 $mW/cm^2$, cumulative amount of light 100 $mJ/cm^2$) by an UV-LED lamp and thus cured. The three-dimensional modeling ink composition was packed (thickness 0.3 mm) on the cured film (first layer) in the spacer and then cured in the same manner. The same operation was repeated to obtain a cured product (6 mm × 60 mm × 60 mm) of 20 layers in total. The cured product was irradiated with ultraviolet light (wavelength 405 nm, illuminance 100 $mW/cm^2$, cumulative amount of light 2,000 $mJ/cm^2$) by an UV-LED lamp, to obtain a modelled product after a post-curing treatment.

<Content rate of low-molecular-weight component>

A test piece having a thickness of 0.5 m was carved from the modelled product, and 0.5 g thereof was weighed. The content rate of the low-molecular-weight component in the modelled product was evaluated by the same method as in evaluation of the content rate of the low-molecular-weight component in the cured product of the curable composition.

<Curing shrinkage resistance>

The density of the three-dimensional modeling ink composition was measured with a Gay-Lussac type specific gravity bottle according to ISO 758. The density of the modelled product was measured with an electron hydrometer (MDS-300 manufactured by Alfa Mirage) according to ISO 1183-1. The rate of curing shrinkage was calculated from the density of the three-dimensional modeling ink composition and the density of the modelled product by the following expression, and the curing shrinkage resistance of the three-dimensional modeling ink composition was evaluated according to the following criteria. As the rate of curing shrinkage is lower, the curing shrinkage resistance is higher.

$$\text{Rate of curing shrinkage (\%)} = (\text{Ds-Dl})/\text{D1} \times 100\%$$

(wherein Ds represents the density of the modelled product and Dl represents the density of the three-dimensional modeling ink composition.)

++: a rate of curing shrinkage of less than 6%.
+: a rate of curing shrinkage of 6% or more and less than 7%.
±: a rate of curing shrinkage of 7% or more and less than 8%.
-: a rate of curing shrinkage of 8% or more.

<Strength>

The Shore D hardness of the modelled product was measured according to ISO 48, and the strength of the three-dimensionally modelled product was evaluated according to the following criteria.

++: a Shore D hardness of 60 or more.
+: a Shore D hardness of 40 or more and less than 60.
-: a Shore D hardness of less than 40.

<Heat resistance>

The glass transition temperature (Tg) of the modelled product was measured with a differential scanning calorimeter (DSC-60plus manufactured by Shimadzu Corporation), and the heat resistance of the modelled product was evaluated according to the following criteria.

++: a Tg of 60°C or more.
+: a Tg of 40°C or more and less than 60°C.
-: a Tg of less than 40°C.

<Modeling accuracy>

A side surface of the modelled product was visually observed, and the height of the modelled product was measured. These results were combined, and the modeling accuracy was evaluated according to the following criteria.

++: a height of 6 mm ± less than 0.1 mm, and no unevenness on the side surface.

+: a height of 6 mm ± 0.1 mm or more and less than ± 0.2 mm, or slight unevenness on the side surface.

±: a height of 6 mm ± 0.2 mm or more and less than ± 0.3 mm, or modest unevenness on the side surface.

-: a height of 6 mm ± 0.3 mm or more, or clear unevenness on the side surface.

<Light yellowing resistance>

The modelled product was further irradiated with ultraviolet light (UV-LED lamp, wavelength 405 nm, 100 mW/$cm^2$, cumulative amount of light 20,000 mJ/$cm^2$), and the light yellowing resistance of the modelled product was evaluated in the same manner as in evaluation of the light yellowing resistance of the cured product of the curable composition.

<Bleed-out resistance>

After the modelled product was left to stand still in a constant temperature and humidity chamber at a temperature of 40°C and a relative humidity of 50% for 168 hours, a side surface of the modelled product was visually observed, and the bleed-out resistance of the modelled product was evaluated in the same manner as in evaluation of the bleed-out resistance of the ink composition cured film.

[Table 12]

| Active energy ray-curable three-dimensional modeling ink composition | | Curable composition (F) or Photopolymerization initiator (D), (E) | Mass(%) | Mono-functionally unsaturated compound (h1) | Mass(%) | Poly-functionally unsaturated compound (h2) | Mass(%) | Viscosity | Curability | Content rate of low-molecular-weight component | Curing shrinkage resistance | Strength | Heat resistance | Modeling accuracy | Light yellowing resistance | Bleed-out resistance |
|---|---|---|---|---|---|---|---|---|---|---|---|---|---|---|---|---|
| Example 193 | F-135 | D-31 | 25 | h1-10 | 25 | h2-7<br>h2-11 | 10<br>40 | ++ | ++ | ++ | + | ++ | ++ | + | ++ | ++ |
| Example 194 | F-136 | F-18 | 100 | | | | | + | ++ | ++ | ++ | ++ | ++ | ++ | ++ | ++ |
| Example 195 | F-137 | D-33 | 30 | h1-1 | 30 | h2-16 | 40 | ++ | ++ | + | ++ | ++ | ++ | ++ | + | + |
| Example 196 | F-138 | D-42 | 20 | h1-1<br>h1-3 | 30<br>5 | h2-2<br>h2-3<br>h2-16 | 5<br>20<br>20 | ++ | ++ | ++ | ++ | + | + | ++ | ++ | ++ |
| Example 197 | F-139 | D-43 | 20 | h1-1<br>h1-3 | 30<br>5 | h2-2<br>h2-3<br>h2-16 | 5<br>20<br>20 | + | ++ | ++ | ++ | ++ | ++ | ++ | ++ | ++ |
| Example 198 | F-140 | D-44 | 20 | h1-1<br>h1-3 | 30<br>5 | h2-2<br>h2-3<br>h2-16 | 5<br>20<br>20 | ++ | + | ++ | ++ | + | + | ++ | ++ | ++ |
| Example 199 | F-141 | D-47 | 50 | h1-1<br>h1-16 | 30<br>20 | | | ++ | + | ++ | ++ | + | + | ++ | ++ | ++ |
| Example 200 | F-142 | D-48<br>E-3 | 1<br>1 | h1-1 | 30 | h2-1<br>h2-12 | 18<br>50 | ++ | + | + | ++ | + | + | ++ | + | ++ |
| Example 201 | F-143 | D-50 | 30 | h1-1<br>h1-18 | 30<br>10 | h2-3<br>h2-10 | 20<br>10 | + | ++ | ++ | ++ | ++ | ++ | ++ | + | ++ |
| Example 202 | F-144 | D-53 | 5 | h1-1<br>h1-9 | 30<br>10 | h2-1<br>h2-11<br>h2-12 | 10<br>15<br>30 | ++ | + | ++ | + | ++ | + | + | ++ | ++ |
| Comparative Example 30 | G-30 | E-1 | 25 | h1-10 | 25 | h2-7<br>h2-11 | 10<br>40 | ++ | ± | - | ± | - | - | - | ± | - |
| Comparative Example 31 | G-31 | E-2 | 5 | h1-1<br>h1-9 | 30<br>10 | h2-1<br>h2-11<br>h2-12 | 10<br>15<br>30 | ++ | + | - | ± | ± | ± | ± | + | - |

As clear from Table 12, the three-dimensional modeling ink composition of each Example was high in curability with a long-wavelength light beam and was less shrunk when cured, and the resulting modelled product was high in modeling accuracy. The modelled product obtained in each Example was high also in strength and heat resistance, and favorable in bleed-out resistance and light yellowing resistance. On the other hand, the three-dimensional modeling ink composition of each Comparative Example was low in curability, and the resulting modelled product was low in modeling accuracy. The modelled product of each Comparative Example had a high content of the low-molecular-weight component, and the modelled product did not achieve satisfiable strength, heat resistance and light yellowing resistance, and was particularly low in bleed-out resistance.

Examples 203 to 208, and Comparative Examples 32 and 33 (preparation and evaluation of active energy ray-curable nail cosmetic compositions)

The benzoylformic acid amide derivative (D), commercially available photopolymerization initiator (E), monofunctionally unsaturated compound (h1), polyfunctionally unsaturated compound (h2), photosensitizer (I), and other component (k) were weighed according to each proportion (in terms of solid content) described in Table 13, and mixed at 25°C for 30 minutes, to prepare each active energy ray-curable nail cosmetic composition (hereinafter, also referred to as "nail cosmetic composition".). The curability and the adhesion with a nylon substrate, of the nail cosmetic composition, the surface hardness, the surface glossiness and the light yellowing resistance of the resulting cured film, and the content rate of the low-molecular-weight component in the cured film were evaluated. The results are shown in Table 13.

<Curability>

The nail-cosmetic composition was applied onto a test piece of nylon 6 (SHT-N6 (NC) manufactured by Toray Plastics Precision Co., Ltd.) with a separator so that the thickness was 100 μm. A cured film was produced by performing ultraviolet light irradiation with an UV-LED lamp for gel nail (manufactured by Beauty Nailer, wavelength 405 nm, output 48 W). The time until disappearance of tack during touch with a surface of the cured film was measured, and curability was evaluated according to the following criteria. As the time necessary until tack disappears is shorter, the curability is higher.

++: tack disappeared at less than 1 minute.
+: tack disappeared at 1 minute or more and less than 3 minutes.
±: tack disappeared at 3 minutes or more and less than 10 minutes.
-: no tack disappeared at 10 minutes or more.

<Adhesion>

The nail-cosmetic composition was used and applied onto a nylon substrate in the same manner as in curability evaluation, and irradiated in the same manner by use of the UV-LED lamp for gel nail, for 3 minutes, to produce a cured film. The adhesion of the cured film obtained was evaluated in the same manner as in adhesion evaluation of the coat layer of the coating agent composition, according to ISO 2409.

<Surface hardness>

A cured film was produced in the same manner as in adhesion evaluation, a pencil having a hardness of HB was used for drawing by application of a load of 750 g at an angle of 45°, onto a surface of the film, the change of the surface of the film was visually confirmed, and the hardness of the surface was evaluated according to the following criteria. As the surface of the film is less scratched and peeled, the hardness of the surface is higher.

+: neither scratch, nor peeling generated.
±: no peeling generated, but scratch generated.
-: peeling generated.

<Surface glossiness>

A cured film was produced in the same manner as in adhesion evaluation, and left to stand still in a constant temperature and humidity chamber at a temperature of 40°C and a relative humidity of 50% for 24 hours. Thereafter, the gloss of a surface of the film was visually observed, and the surface glossiness of the cured film was evaluated according to the following criteria.

+: gloss observed.
±: light reflection confirmable, but clouding section observed.
-: no light reflection confirmable, and no gloss observed.

<Content rate of low-molecular-weight component>

A cured film was produced in the same manner as in adhesion evaluation, a test piece of nylon 6, having the cured film obtained, was used, and the content rate of the low-molecular-weight component in the cured film was evaluated in the same manner as in evaluation of the content rate of the low-molecular-weight component in the cured product (adhesive

layer) of the adhesive composition.

<Light yellowing resistance>

A cured film was produced in the same manner as in adhesion evaluation, mounted on a xenon fade meter, and irradiated with ultraviolet light having an intensity of 70 mW/cm$^2$ for 120 hours. Thereafter, the change in color of the cured film was visually observed, and light yellowing resistance was evaluated according to the following criteria.

++: no yellowing observed at all.
+: yellowing extremely slightly observed.
±: yellowing observed.
-: clear yellowing observed.

[Table 13]

| Active energy ray-curable nail cosmetic composition | | Photopolymerization initiator(D), (E) | Mass(%) | Mono-functionally unsaturated compound (h1) | Mass(%) | Poly-functionally unsaturated compound (h2) | Mass(%) | Photosensitizer(I) Other(k) | Mass(%) | Curability | Adhesion | Surface hardness | Surface glossiness | Content rate of low-molecular-weight component | Light yellowing resistance |
|---|---|---|---|---|---|---|---|---|---|---|---|---|---|---|---|
| Example 203 | F-145 | D-28<br>E-3 | 1<br>1 | h1-6 | 18 | h2-3<br>h2-16 | 40<br>40 | | | + | + | + | + | + | + |
| Example 204 | F-146 | D-40 | 10 | h1-1<br>h1-2<br>h1-4 | 20<br>20<br>10 | h2-3 | 35 | k-1 | 5 | + | + | + | + | + | ++ |
| Example 205 | F-147 | D-45 | 40 | h1-1<br>h1-15 | 20<br>15 | h2-5 | 15 | I-1 | 10 | ++ | ++ | + | + | ++ | + |
| Example 206 | F-148 | D-46 | 45 | h1-1<br>h1-2<br>h1-4 | 20<br>20<br>10 | | | k-1 | 5 | ++ | ++ | + | + | ++ | + |
| Example 207 | F-149 | D-53 | 10 | h1-2 | 25 | h2-11<br>h2-16 | 40<br>20 | k-1 | 5 | ++ | ++ | + | + | ++ | ++ |
| Example 208 | F-150 | D-54 | 50 | h1-1<br>h1-15 | 20<br>15 | h2-5 | 15 | | | + | ++ | + | + | ++ | + |
| Comparative Example 32 | G-32 | E-1 | 10 | h1-1<br>h1-2<br>h1-4 | 20<br>20<br>10 | h2-3 | 35 | k-1 | 5 | ± | ± | - | ± | - | ± |
| Comparative Example 33 | G-33 | E-2 | 10 | h1-2 | 25 | h2-11<br>h2-16 | 40<br>20 | k-1 | 5 | ± | + | ± | - | - | + |

As clear from the results in Table 13, the nail cosmetic composition of each Example had high curability with an UV lamp for gel nail, and the resulting cured film was high in adhesion with a nylon substrate (material having many amide bonds as in nails mainly made of proteins). The nail cosmetic composition was found to be suitably usable in a gel nail for base gel, for direct application to nails. The cured film was low in content rate of the low-molecular-weight component, and could ensure safety. The cured film was favorable in surface glossiness, surface hardness and light yellowing resistance, and was suitably usable in a gel nail for topcoat. On the other hand, the nail cosmetic composition of each Comparative Example was low in curability, the cured film obtained had a high content of the low-molecular-weight component, and the

cured film was low in adhesion, surface hardness, surface glossiness and light yellowing resistance.

Examples 209 to 214 and Comparative Examples 34 to 36 (preparation and evaluation of active energy ray-curable dental material compositions)

The benzoylformic acid amide derivative (D) and commercially available photopolymerization initiator (E), monofunctionally unsaturated compound (h1), polyfunctionally unsaturated compound (h2) and other component (k) were weighed according to each proportion (in terms of solid content) described in Table 14, and mixed at 25°C for 30 minutes, to prepare each active energy ray-curable dental material composition (hereinafter, also referred to as "dental material composition".). The solubility (dispersibility), the storage stability and the curability of the dental material composition were evaluated. The dental material composition was cured to obtain a cured product. The content rate of the low-molecular-weight component in the cured product, and the hardness, the surface smoothness and bending strength of the cured product were evaluated. The results are shown in Table 14.

<Solubility (dispersibility)>

The state of the dental material composition was visually observed, and solubility (dispersibility) was evaluated according to the following criteria.

+: composition obtained in uniform state.
±: composition obtained in slight ununiform state.
-: composition obtained in ununiform state.

<Storage stability>

The dental material composition was placed in a light-shielding screw tube, covered with a lid, and stored in two conditions of 40°C and one month and 80°C and two weeks. The dissolution or dispersion state of the composition after storage was confirmed, and storage stability was evaluated according to the following criteria.

+: no change in state after storage confirmed in two conditions of 40°C and one month and 80°C and two weeks.
±: change in state after storage confirmed in any one condition of 40°C and one month or 80°C and two weeks.
-: change in state after storage confirmed in two conditions of 40°C and one month and 80°C and two weeks.

<Curability>

The dental material composition was packed in a polytetrafluoroethylene mold having a hole of 6 mm in diameter at the center (20 mm × 20 mm × 10 mm), and pressure-contacted with a polypropylene film. The resultant was irradiated with ultraviolet light (wavelength 405 nm, illuminance 50 mW/cm $^2$) for 30 seconds, the polypropylene film was released, the cured product was touched with a hand, and curability was evaluated according to the following criteria.

++: no tackiness at all.
+: modest tackiness, but no finger mark remaining on surface.
±: tackiness, and finger mark remaining on surface.
-: extreme tackiness, and finger sticking to surface.

<Content rate of low-molecular-weight component>

The cured product obtained in curability evaluation was used, and the content rate of the low-molecular-weight component in the cured product of the dental material composition was evaluated in the same manner as in evaluation of the content rate of the low-molecular-weight component in the curable composition.

<Hardness>

A surface of the cured product obtained in curability evaluation was buffed, the Knoop hardness was measured with a micro hardness meter (DMH-2 manufactured by Matsuzawa Co., Ltd.) in conditions of a temperature of 23°C, 100 gf, and loading application for 20 seconds, and the hardness was evaluated according to the following criteria.

++: a Knoop hardness of 200 KHN or more.

+: a Knoop hardness of 70 KHN or more, and less than 200 KHN.
-: a Knoop hardness of less than 70 KHN.

<Surface smoothness>

A surface of the cured product obtained in curability evaluation was visually observed, and surface smoothness was evaluated according to the following criteria.

++: smooth and glossy surface.
+: almost smooth surface, and slight clouding or unevenness observed.
±: surface entirely clouded, and unevenness or grain-like object observed somewhat.
-: surface clouded entirely and covered with grain-like object.

<Bending strength>

A heavy release film was closely contacted with a glass plate horizontally disposed, a polytetrafluoroethylene spacer (2 mm × 2 mm × 25 mm) was disposed thereon, and the dental material composition was packed. A liquid surface of the spacer was covered with a light release film so that air bubbles were not entrained, and irradiated with ultraviolet light by an UV-LED lamp (wavelength 405 nm, illuminance 50 mW/cm$^2$, cumulative amount of light 1,500 mJ/cm$^2$). Thereafter, the release film on each of both surfaces was released, and the cured product was taken out from the spacer and adopted as a specimen. The specimen was immersed in water at 37°C for 24 hours, and thereafter subjected to a bending test with a universal tester. Test conditions were according to ISO 4049, and the distance between fulcrums was 20 mm and the crosshead speed was 1 mm/min. The bending strength was evaluated according to the following criteria.

++: a bending strength of 100 MPa or more.
+: a bending strength of 90 MPa or more and less than 100 MPa.
±: a bending strength of 80 MPa or more and less than 90 MPa.
-: a bending strength of less than 80 MPa.

[Table 14]

| Active energy ray-curable dental material composition | | Photopolymerization initiator(D), (E) | Mass(%) | Mono-functionally unsaturated compound (h1) | Mass(%) | Poly-functionally unsaturated compound (h2) | Mass(%) | Other(k) | Mass(%) | Solubility(dispersibility) | Storage stability | Curability | Content rate of low-molecular-weight component | Hardness | Surface smoothness | Bending strength |
|---|---|---|---|---|---|---|---|---|---|---|---|---|---|---|---|---|
| Example 209 | F-151 | D-23<br>E-4 | 0.5<br>9.5 | h1-6<br>h1-19 | 20<br>10 | h2-17 | 50 | k-11 | 10 | + | + | + | ++ | ++ | + | ++ |
| Example 210 | F-152 | D-38 | 40 | h1-3<br>h1-8 | 20<br>10 | h2-15 | 20 | k-11 | 10 | + | + | ++ | ++ | + | ++ | + |
| Example 211 | F-153 | D-43 | 10 | h1-8<br>h1-14<br>h1-19 | 10<br>35<br>10 | h2-18 | 30 | k-12 | 5 | + | + | ++ | ++ | ++ | + | + |
| Example 212 | F-154 | D-39<br>D-52 | 5<br>5 | h1-3 | 20 | h2-6<br>h2-15 | 30<br>30 | k-11 | 10 | + | + | + | ++ | ++ | + | + |
| Example 213 | F-155 | D-48 | 50 | | | h2-3<br>h2-19 | 25<br>20 | k-12 | 5 | + | + | ++ | ++ | ++ | + | ++ |
| Example 214 | F-156 | D-54 | 20 | | | h2-17<br>h2-19 | 50<br>27 | k-9 | 3 | + | + | ++ | ++ | ++ | + | ++ |
| Comparative Example 34 | G-34 | E-1 | 20 | | | h2-17<br>h2-19 | 50<br>20 | k-12 | 10 | - | - | ± | - | - | - | - |
| Comparative Example 35 | G-35 | E-2 | 10 | h1-8<br>h1-14<br>h1-19 | 10<br>35<br>10 | h2-18 | 30 | k-12 | 5 | ± | ± | ± | - | + | ± | - |
| Comparative Example 36 | G-36 | E-3 | 10 | h1-3 | 20 | h2-6<br>h2-15 | 30<br>30 | k-11 | 10 | - | - | ++ | - | + | + | ± |

As clear from the results in Table 14, the dental material composition of each of Examples had high solubility or dispersibility, and was high in curability and storage stability. The cured product obtained was low in content rate of the low-molecular-weight component, and was excellent in safety of a dental material. The cured product was high in both hardness and bending strength, and favorable in surface smoothness. On the other hand, the dental material composition of each of Comparative Examples was low in curability, and insufficient in both solubility and storage stability. The cured product was high in content rate of the low-molecular-weight component, and had a concern about safety. The cured product was low in surface smoothness, hardness and bending strength. The dental material composition of the present disclosure can be suitably used in a dental restoration material (composite resin for tooth crowns, composite resin for dental caries cavity filling, composite resin for support base construction, composite resin for filling/restoring), a denture base resin, a cementing resin, a cementing material (resin cement, resin-addition-type glass ionomer cement), a dental adhesive material (adhesive material for orthodontics, adhesive material for cavity covering), a denture base liner, an impression material, a dental temporary sealing material, a dental fissure sealant, a resin block for CAD/CAM, a temporary crown, and an artificial tooth material.

Examples 215 to 220, and Comparative Examples 37 and 38 (preparation and evaluation of active energy ray-curing photosensitive compositions)

The benzoylformic acid amide derivative (D), commercially available photopolymerization initiator (E), monofunctionally unsaturated compound (h1), polyfunctionally unsaturated compound (h2), thermal polymerization initiator (J), solvent (c) and other component (k) were weighed according to each proportion (in terms of solid content) described in Table 15, and mixed at 25°C for 30 minutes, to prepare each active energy ray-curing photosensitive composition (hereinafter, also referred to as "photosensitive composition".). The photosensitive composition was used to produce a photosensitive resin by the following method, and the sensitivity (curability) and the storage stability of the photosensitive

resin obtained were evaluated. A patterned cured product was produced from the photosensitive composition, and the pattern formability of the cured product obtained, and the content rate of the low-molecular-weight component in the cured product were evaluated. These results are shown in Table 15.

<Production of photosensitive resin>

The photosensitive composition of each of Examples and Comparative Examples was adopted, and applied by a rotatory applicator so that the thickness was 15 μm, and dried in an oven for 3 minutes. Thereafter, irradiation with ultraviolet light for 3 minutes (wavelength 405 nm, illuminance 0.5 mW/$cm^2$, cumulative amount of light 90 mJ/$cm^2$) was performed, to obtain a photosensitive resin (cured product). A photosensitive composition containing no thermal polymerization initiator (J) was dried at 80°C. A photosensitive composition containing thermal polymerization initiator (J) was dried at 40°C, and irradiated with ultraviolet light and then heat-treated with an oven at 130°C for 30 minutes.

<Sensitivity >

The photosensitive resin was touched with a hand, and sensitivity was evaluated according to the following criteria.

++: no tackiness at all.
+: modest tackiness, but no finger mark remaining on surface.
±: tackiness, and finger mark remaining on surface.
-: extreme tackiness, and finger sticking to surface.

<Storage stability>

The photosensitive resin was left to stand still in a constant temperature and humidity chamber at a temperature of 40°C and a relative humidity of 50% for 168 hours, a surface of the photosensitive resin was visually observed, and storage stability was evaluated according to the following criteria. As bleed-out is less observed, storage stability is higher.

++: no bleed-out observed at all.
+: bleed-out slightly observed.
-: bleed-out remarkably observed.

<Content rate of low-molecular-weight component>

Three 5-$cm^2$-sized test pieces were cut out from the photosensitive resin, and dried at 130°C for 30 minutes. Thereafter, the content rate of the low-molecular-weight component in the cured product of the photosensitive resin (cured product of photosensitive composition) was evaluated in the same manner as in evaluation of the content rate of the low-molecular-weight component in the cured product of the curable composition.

<Production of patterned cured product>

A negative-type photomask (pattern mask) was used, and a cured product of the photosensitive composition of each of Examples and Comparative Examples was produced in the same manner as in production of the photosensitive resin. Thereafter, the negative-type photomask was detached from the cured product, and an unexposed section was removed with cyclopentanone, to obtain a patterned cured product.

<Pattern formability>

The pattern formability of the patterned cured product was evaluated according to the following criteria.

++: neither pattern distortion, nor edge portion chipping.
+: no pattern distortion, and slight edge portion chipping.
±: slight pattern distortion, and edge portion chipping.
-: not only pattern distortion, but also edge portion chipping.

[Table 15]

| Active energy ray-curable photosensitive composition | | Photopolymerization initiator(D), (E) | Mass(%) | Thermal polymerization initiator(J) | Mass(%) | Polyfunctionally unsaturated compound (h2) | Mass(%) | Solvent(c) | Mass(%) | Other(k) | Mass(%) | Sensitivity | Pattern formability | Content rate of low-molecular-weight component | Storage stability |
|---|---|---|---|---|---|---|---|---|---|---|---|---|---|---|---|
| Example 215 | F-157 | D-37 | 12 | | | h2-2 | 27 | c-5 | 55 | k-13 | 6 | + | + | ++ | ++ |
| Example 216 | F-158 | D-38 | 30 | J-1 | 2 | h2-7 | 18 | c-1 | 45 | k-10 | 5 | + | + | ++ | ++ |
| Example 217 | F-159 | D-43<br>E-5 | 1<br>1 | | | h2-7<br>h2-15 | 30<br>10 | c-5 | 55 | k-10 | 3 | + | + | + | + |
| Example 218 | F-160 | D-45 | 8 | J-1 | 5 | h2-10 | 13 | c-3 | 70 | k-13 | 4 | ++ | ++ | ++ | ++ |
| Example 219 | F-161 | D-48 | 0.5 | J-1 | 2.5 | h2-8<br>h2-15 | 17<br>20 | c-1 | 60 | | | ++ | ++ | ++ | ++ |
| Example 220 | F-162 | D-52 | 50 | | | | | c-5 | 50 | | | ++ | + | ++ | ++ |
| Comparative Example 37 | G-37 | E-1 | 12 | | | h2-2 | 27 | c-5 | 55 | k-13 | 6 | + | + | - | - |
| Comparative Example 38 | G-38 | E-2 | 8 | J-1 | 5 | h2-10 | 13 | c-3 | 70 | k-13 | 4 | ++ | ++ | - | - |

As clear from the results in Table 15, the photosensitive composition of each of Examples had high curability (sensitivity), and a cured product (photosensitive resin) obtained by curing the photosensitive composition was high in storage stability, and low in content rate of the low-molecular-weight component. The patterned cured product of each of Examples, obtained with a pattern mask, had excellent pattern formability. On the other hand, the photosensitive composition of each of Comparative Examples was low in sensitivity, a cured product obtained therefrom was high in content of the low-molecular-weight component and was low in storage stability. The patterned cured product of each of Comparative Examples, obtained with a pattern mask, was inferior in pattern formability.

Examples 221 to 227, and Comparative Examples 39 and 40 (preparation and evaluation of active energy ray-curable hydrogel compositions)

The benzoylformic acid amide derivative (D), commercially available photopolymerization initiator (E), monofunctionally unsaturated compound (h1), polyfunctionally unsaturated compound (h2), ion-exchange water and other component (k) were weighed according to each proportion (in terms of solid content) described in Table 16, and mixed at 25°C for 30 minutes, to prepare each active energy ray-curable hydrogel composition (hereinafter, also referred to as "hydrogel composition".). The compatibility and the curability of the hydrogel composition were evaluated, the appearance of the cured product (hydrogel) obtained and the content rate of the low-molecular-weight component in the cured product were evaluated. The results are shown in Table 16.

<Compatibility>

The hydrogel composition prepared was evaluated by the same method and evaluation criteria as in compatibility evaluation of the active energy ray-curable composition.

<Curability>

The hydrogel composition was applied onto a PET film with a bar coater so that the thickness was 20 μm. A coating film was cured by irradiation with ultraviolet light in the following conditions 7) to 9), and touch with the cured product

was performed to evaluate the curability according to the following criteria.

7) High-pressure mercury lamp: wavelength 200 to 450 nm, illuminance 100 mW/cm$^2$, 1,000 mJ/cm$^2$
8) UV-LED lamp: wavelength 385 nm, illuminance 100 mW/cm$^2$, 1,000 mJ/cm$^2$
9) UV-LED lamp: wavelength 405 nm, illuminance 100 mW/cm$^2$, 1,000 mJ/cm$^2$
++: gel entirely formed, and slight hard state.
+: gel entirely formed, and slight soft state.
±: gel partially formed.
-: no gel formed.

<Appearance of cured product>

The appearance of the cured product obtained in curability evaluation in ultraviolet light irradiation condition 9) was visually observed, and was evaluated according to the following criteria.

+: neither clouding, nor phase separation observed.
±: no phase separation, but clouding observed.
-: both clouding and phase separation observed.

<Content rate of low-molecular-weight component>

The cured product obtained in curability evaluation of ultraviolet light irradiation condition 9) was used and the content rate of the low-molecular-weight component (except for water) in the cured product (hydrogel) was evaluated in the same manner as in evaluation of the content rate of the low-molecular-weight component in the cured product of the curable composition.

[Table 16]

| Active energy ray-curable hydrogel composition | | Photopolymerization initiator(D), (E) | Mass(%) | Mono-functionally unsaturated compound(h1) | Mass(%) | Poly-functionally unsaturated compound(h2) | Mass(%) | Other(k) | Mass(%) | Mass (%) of ion-exchange water | Compatibility | Curability (wavelength | | | Appearance of cured product | Content rate of low-molecular-weight component |
|---|---|---|---|---|---|---|---|---|---|---|---|---|---|---|---|---|
| | | | | | | | | | | | | High-pressure mercury | LED 385 | LED 405 | | |
| Example 221 | F-163 | D-9<br>D-15 | 4<br>1 | h1-1 | 52 | h2-5 | 5 | k-14 | 3 | 35 | + | ++ | ++ | + | + | ++ |
| Example 222 | F-164 | D-10 | 0.5 | h1-8 | 40 | h2-18 | 9.5 | | | 50 | + | + | + | + | + | + |
| Example 223 | F-165 | D-20 | 10 | h1-1<br>h1-11 | 30<br>15 | h2-5 | 5 | | | 40 | + | ++ | ++ | ++ | + | ++ |
| Example 224 | F-166 | D-4<br>E-7 | 1<br>1 | h1-8 | 25 | h2-18 | 3 | | | 70 | + | ++ | ++ | ++ | + | + |
| Example 225 | F-167 | D-26 | 50 | h1-1 | 29 | h2-12 | 1 | | | 20 | + | ++ | ++ | ++ | ++ | ++ |
| Example 226 | F-168 | D-27 | 5 | h1-1 | 60 | h2-5 | 5 | k-14 | 3 | 27 | + | ++ | ++ | + | + | ++ |
| Example 227 | F-169 | D-40 | 10 | h1-1 | 60 | h2-5 | 5 | k-14 | 3 | 22 | + | ++ | ++ | ++ | + | ++ |
| Comparative Example 39 | G-39 | E-2 | 5 | h1-1 | 52 | h2-5 | 5 | k-14 | 3 | 35 | ± | ++ | + | + | - | - |
| Comparative Example 40 | G-40 | E-7 | 10 | h1-1<br>h1-11 | 30<br>15 | h2-5 | 5 | | | 40 | ± | ++ | ± | - | *3 | *3 |

(*3) No cured product obtained and no evaluation carried out.

As clear from the results in Table 16, the hydrogel composition of each of Examples contained water-soluble or hydrophilic acryloylmorpholine (h1-1), N-(2-hydroxyethyl)acrylamide (h1-7) or N-vinylpyrrolidone (h1-11) and water, exhibited favorable compatibility, and was in an aqueous solution state. The hydrogel composition of each of Examples

had high curability, and gel was entirely formed (hydrogel) in a cured product obtained by curing the hydrogel composition. The cured product (hydrogel) was low in content rate of the low-molecular-weight component, and high in safety. Such hydrogel can be suitably used as a health material or a medical material. On the other hand, the hydrogel composition of each of Comparative Examples was low in both compatibility and curability, and could not form any homogeneous hydrogel even by irradiation with ultraviolet light, and the content rate of the low-molecular-weight component could not be evaluated. In Comparative Example in which hydrogel was partially formed, the hydrogel was high in content rate of the low-molecular-weight component.

Examples 228 to 234, and Comparative Examples 41 and 42 (preparation and evaluation of active energy ray-curable aqueous compositions)

The benzoylformic acid amide derivative (D), commercially available photopolymerization initiator (E), monofunctionally unsaturated compound (h1), polyfunctionally unsaturated compound (h2), ion-exchange water and other component (k) were weighed according to each proportion (in terms of solid content) described in Table 17, and mixed at 25°C for 30 minutes, to prepare each active energy ray-curable aqueous composition (hereinafter, also referred to as "aqueous composition".). The dispersibility and the curability of the aqueous composition were evaluated, and the appearance of the cured product obtained and the content rate of the low-molecular-weight component in the cured product were evaluated. The results are shown in Table 17.

<Dispersibility>

The aqueous composition was left to stand still in a constant temperature chamber at 40°C for 24 hours, thereafter the state of the aqueous composition was visually observed, and dispersibility was evaluated according to the following criteria. +: aqueous composition as stable and homogeneous emulsion.

±: partial aggregation observed in aqueous composition, and heterogeneous emulsion.
-: phase separation observed in aqueous composition.

<Curability>

A coating film of the aqueous composition was produced by the same method as in curability evaluation of the curable composition, dried at 80°C for 5 minutes, and then irradiated with ultraviolet light, and the curability thereof was evaluated.

<Appearance of cured product>

The cured product obtained in curability evaluation in ultraviolet light irradiation condition 3) was visually observed, and the appearance of the cured product was evaluated according to the following criteria.

+: neither clouding, nor phase separation observed in cured product.
±: clouding observed in cured product.
-: phase separation observed in cured product.

<Content rate of low-molecular-weight component>

The cured product obtained in curability evaluation in ultraviolet light irradiation condition 3) was used, and the content rate of the low-molecular-weight component in the cured product of the aqueous composition was evaluated in the same manner as in evaluation of the content rate of the low-molecular-weight component in the curable composition.

[Table 17]

| Active energy ray-curable aqueous composition | | Photo-polymerization initiator(D), (E) | Mass(%) | Mono-functionally unsaturated compound(h1) | Mass(%) | Poly-functionally unsaturated compound(h2) | Mass(%) | Other(k) | Mass(%) | Mass (%) of ion-exchange water | Dispersibility | Curability (wavelength | | | Appearance of cured product | Content rate of low-molecular-weight component |
|---|---|---|---|---|---|---|---|---|---|---|---|---|---|---|---|---|
| | | | | | | | | | | | | High-pressure mercury | LED 385 | LED 405 | | |
| Example 228 | F-170 | D-9 | 2 | h1-12 | 40 | h2-5 | 17 | k-14 | 1 | 40 | + | ++ | ++ | + | + | + |
| Example 229 | F-171 | D-24 | 5 | h1-2<br>h1-7 | 20<br>20 | h2-1 | 5 | | | 50 | + | ++ | ++ | ++ | + | ++ |
| Example 230 | F-172 | D-40 | 10 | h1-10 | 20 | h2-8 | 5 | k-15 | 5 | 60 | + | ++ | + | + | + | + |
| Example 231 | F-173 | D-43 | 10 | h1-6<br>h1-9 | 15<br>20 | h2-8 | 3 | k-14 | 2 | 50 | + | ++ | ++ | ++ | + | ++ |
| Example 232 | F-174 | D-48<br>E-7 | 0.5<br>1.5 | h1-10<br>h1-12 | 30<br>10 | h2-10 | 8 | | | 50 | + | + | + | + | + | + |
| Example 233 | F-175 | D-35 | 15 | h1-13 | 15 | | | k-15 | 0.5 | 70 | + | ++ | ++ | ++ | + | ++ |
| Example 234 | F-176 | D-49 | 40 | h1-10 | 10 | | | k-14 | 2 | 48 | + | ++ | ++ | ++ | + | ++ |
| Comparative Example 41 | G-41 | E-1 | 10 | h1-10 | 20 | h2-8 | 5 | k-15 | 5 | 60 | ± | + | + | - | *3 | *3 |
| Comparative Example 42 | G-42 | E-2 | 2 | h1-12 | 40 | h2-5 | 17 | k-14 | 1 | 40 | + | ++ | + | ± | ± | - |

(*3) No cured product obtained and no evaluation carried out.

As clear from the results in Table 17, the aqueous composition in each Example was good in dispersibility, could keep a favorable emulsion state, and was high in curability even in use of a long-wavelength light beam. The cured product obtained was low in content rate of the low-molecular-weight component. On the other hand, the aqueous composition of each of Comparative Examples was low in both dispersibility and curability, and the cured product obtained was high in content of the low-molecular-weight component.

The present disclosure includes the following content.

(1) A benzoylformic acid amide derivative having a benzoylformic acid amide group represented by general formula (1):

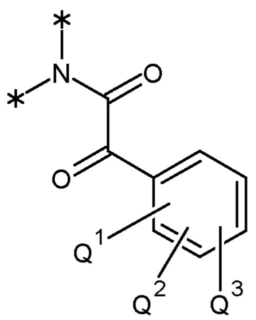

General formula (1)

wherein $Q^1$ to $Q^3$ independently of each other represent a hydrogen atom, a substituent represented by formulas (Formula 2) to (Formula 8), a halogen group, or a nitrile group, and are each bound to any position of the 2-position to the 6-position,

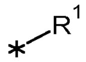

(Formula 2)

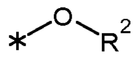
(Formula 3)

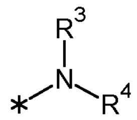
(Formula 4)

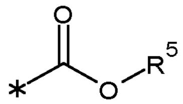
(Formula 5)

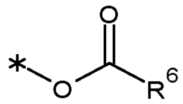
(Formula 6)

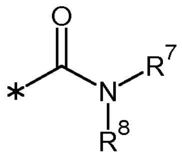
(Formula 7)

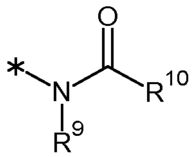
(Formula 8)

$R^1$ to $R^{10}$ each independently represents a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, or a cyclic alkenyl group having 3 to 18 carbon atoms, and

* is a binding position.

(2) The benzoylformic acid amide derivative according to (1), wherein the benzoylformic acid amide derivative is at least one compound represented by any one general formula of general formula (2) to general formula (4):

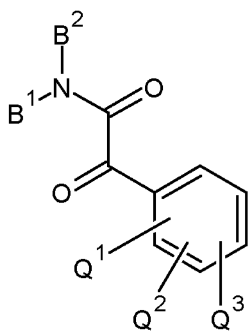
General formula (2)

wherein $Q^1$ to $Q^3$ have the same meanings as the definitions described in the general formula (1),

$B^1$ represents a hydrogen atom, or a monovalent organic group optionally having a hydroxyl group, an amino group, a thiol group, an ether group, a thioether group, an ester group, a carbonate group, a urethane group, a thiourethane group, a urea group, a siloxane group, an amide group, an imide group, an ethylenically unsaturated group or a benzoylformic acid amide group,

$B^2$ represents a monovalent organic group optionally having a hydroxyl group, an amino group, a thiol group, an ether group, a thioether group, an ester group, a carbonate group, a urethane group, a thiourethane group, a urea group, an amide group, an imide group, a siloxane group, an ethylenically unsaturated group or a benzoylformic acid amide group,

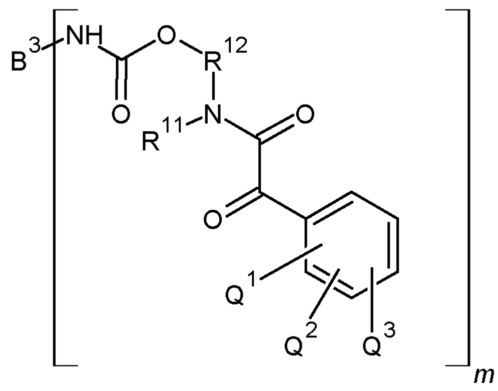

General formula (3)

wherein $Q^1$ to $Q^3$ have the same meanings as the definitions described in the general formula (1),
$B^3$ represents an m-valent organic group optionally having an ethylenically unsaturated group, an ether group, a thioether group, an ester group, a carbonate group, a urethane group, a thiourethane group, an isocyanurate group, an allophanate group, a urea group, a siloxane group, an amide group or an imide group,
$R^{11}$ represents a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, a cyclic alkenyl group having 3 to 18 carbon atoms, or an aryl group having 6 to 8 carbon atoms,
$R^{12}$ represents a linear saturated divalent hydrocarbon group having 1 to 18 carbon atoms, a linear unsaturated divalent hydrocarbon group having 2 to 18 carbon atoms, a branched saturated or unsaturated divalent hydrocarbon group having 3 to 18 carbon atoms, an alicyclic saturated or unsaturated divalent hydrocarbon group having 3 to 8 carbon atoms, a divalent aromatic hydrocarbon group having 6 to 8 carbon atoms, or a divalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group, and m represents an integer of 1 to 10,

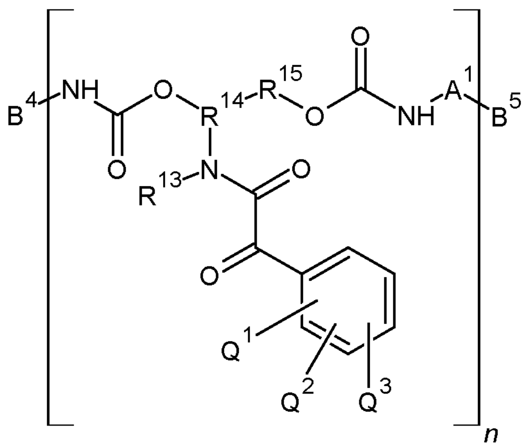

General formula (4)

wherein $Q^1$ to $Q^3$ have the same meanings as the definitions described in the general formula (1),
$A^1$ represents a divalent organic group optionally having an ether group, a thioether group, an ester group, a carbonate group, a urethane group, a thiourethane group, a urea group, a siloxane group, an amide group or an imide group,
$B^4$ and $B^5$ independently of each other optionally have an ethylenically unsaturated group, an ether group, a thioether group, an ester group, a carbonate group, a urethane group, a thiourethane group, an isocyanurate group, an allophanate group, a urea group, a siloxane group, an amide group or an imide group, and any one or both of $B^4$ and $B^5$ represent a monovalent organic group having one or more ethylenically unsaturated bonds,

$R^{13}$ represents a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, a cyclic alkenyl group having 3 to 18 carbon atoms, or an aryl group having 6 to 8 carbon atoms,

$R^{14}$ represents a linear saturated trivalent hydrocarbon group having 1 to 8 carbon atoms, a linear unsaturated trivalent hydrocarbon group having 2 to 8 carbon atoms, a branched saturated or unsaturated trivalent hydrocarbon group having 3 to 8 carbon atoms, an alicyclic saturated or unsaturated trivalent hydrocarbon group having 3 to 8 carbon atoms, a trivalent aromatic hydrocarbon group having 6 to 8 carbon atoms, or a trivalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group,

$R^{15}$ represents a linear saturated divalent hydrocarbon group having 1 to 18 carbon atoms, a linear unsaturated divalent hydrocarbon group having 2 to 18 carbon atoms, a branched saturated or unsaturated divalent hydrocarbon group having 3 to 8 carbon atoms, an alicyclic saturated or unsaturated divalent hydrocarbon group having 3 to 8 carbon atoms, a divalent aromatic hydrocarbon group having 6 to 8 carbon atoms, or a divalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group, and n represents an integer of 1 to 100.

(3) The benzoylformic acid amide derivative according to (1) or (2), wherein $Q^1$ to $Q^3$ in the benzoylformic acid amide group represented by general formula (1) are independently of each other a hydrogen atom, a substituent represented by formula (Formula 3), or a substituent represented by formula (Formula 6).

(4) The benzoylformic acid amide derivative according to any one of (1) to (3), having one or more ethylenically unsaturated bonds in a molecule, wherein the ethylenically unsaturated bonds are included in one or more groups selected from a (meth)acrylate group, a (meth)acrylamide group, a vinyl group, a vinyl ether group, an alkyl vinyl ether group, an allyl group, a (meth)allyl ether group, a styryl group and a maleimide group.

(5) The benzoylformic acid amide derivative according to any one of (2) to (4), wherein the ethylenically unsaturated bonds are included in each an acrylate group or an acrylamide group.

(6) The benzoylformic acid amide derivative according to any one of (2) to (5), having one or more urethane groups in a molecule, wherein the number of atoms directly linking a nitrogen atom of the benzoylformic acid amide group and a nitrogen atom of a proximate urethane group is 3 to 20.

(7) The benzoylformic acid amide derivative according to any one of (2) to (6), having one or more urethane groups in a molecule, wherein the number of atoms directly linking a nitrogen atom of the benzoylformic acid amide group and a nitrogen atom of a proximate urethane group is 4 to 10.

(8) The benzoylformic acid amide derivative according to any one of (2) to (7), wherein m in the benzoylformic acid amide derivative represented by general formula (3) is an integer of 1 to 4.

(9) The benzoylformic acid amide derivative according to any one of (2) to (8), wherein n in the benzoylformic acid amide derivative represented by general formula (4) is an integer of 2 to 50.

(10) The benzoylformic acid amide derivative according to any one of (2) to (9), wherein a ratio of the number of urethane groups (total) and the number of benzoylformic acid amide groups (total) in the benzoylformic acid amide derivative represented by general formula (3) is 0.5 to 10.0.

(11) The benzoylformic acid amide derivative according to any one of (2) to (10), wherein a ratio of the number of urethane groups (total) and the number of benzoylformic acid amide groups (total) in the benzoylformic acid amide derivative represented by general formula (4) is 2.0 to 15.0.

(12) The benzoylformic acid amide derivative according to any one of (1) to (11), wherein the benzoylformic acid amide derivative is a photopolymerization initiator.

(13) The photopolymerization initiator according to (12), wherein the benzoylformic acid amide group represented by general formula (1) is represented by benzoylformic acid monosubstituted-amide.

(14) The benzoylformic acid amide derivative according to any one of (1) to (11), wherein the benzoylformic acid amide derivative is a photosensitizer of photoradical polymerization and/or a photosensitizer of photoionic polymerization.

(15) An active energy ray-curable composition comprising the benzoylformic acid amide derivative according to any one of (1) to (12) and (14).

(16) An active energy ray-curable ink composition comprising the benzoylformic acid amide derivative according to any one of (1) to (12) and (14).

(17) An active energy ray-curable pressure-sensitive adhesive composition comprising the benzoylformic acid amide derivative according to any one of (1) to (12) and (14).

(18) An active energy ray-curable adhesive composition comprising the benzoylformic acid amide derivative according to any one of (1) to (12) and (14).

(19) An active energy ray-curable sealant composition comprising the benzoylformic acid amide derivative according to any one of (1) to (12) and (14).

(20) An active energy ray-curing photosensitive composition comprising the benzoylformic acid amide derivative according to any one of (1) to (12) and (14).

(21) An active energy ray-curable nail cosmetic composition comprising the benzoylformic acid amide derivative according to any one of (1) to (12) and (14).

(22) An active energy ray-curable dental material composition comprising the benzoylformic acid amide derivative according to any one of (1) to (12) and (14).

(23) An active energy ray-curable coating agent composition comprising the benzoylformic acid amide derivative according to any one of (1) to (12) and (14).

(24) An active energy ray-curable aqueous composition comprising the benzoylformic acid amide derivative according to any one of (1) to (12) and (14).

(25) An active energy ray-curable ink-jet ink composition comprising the benzoylformic acid amide derivative according to any one of (1) to (12) and (14).

(26) An active energy ray-curable elastomer composition comprising the benzoylformic acid amide derivative according to any one of (1) to (12) and (14).

(27) An active energy ray-curable decorative sheet resin composition comprising the benzoylformic acid amide derivative according to any one of (1) to (12) and (14).

(28) An active energy ray-curable architectural-paint composition comprising the benzoylformic acid amide derivative according to any one of (1) to (12) and (14).

(29) An active energy ray-curable medical device surface coating agent composition comprising the benzoylformic acid amide derivative according to any one of (1) to (12) and (14).

(30) An active energy ray-curable three-dimensional modeling ink composition comprising the benzoylformic acid amide derivative according to any one of (1) to (12) and (14).

(31) An active energy ray-curable flexographic ink composition comprising the benzoylformic acid amide derivative according to any one of (1) to (12) and (14).

(32) An active energy ray-curable offset ink composition comprising the benzoylformic acid amide derivative according to any one of (1) to (12) and (14).

(33) An active energy ray-curable screen ink composition comprising the benzoylformic acid amide derivative according to any one of (1) to (12) and (14).

(34) An active energy ray-curable self-repairing-material resin composition comprising the benzoylformic acid amide derivative according to any one of (1) to (12) and (14).

Industrial Applicability

As described above, the benzoylformic acid amide derivative (D) of the present disclosure exhibits high curability with ultraviolet light at various wavelengths, including long-wavelength ultraviolet light at wavelengths of 360 to 420 nm, and in particular exhibits high photopolymerization initiation ability, photosensitivity and curability even in use of UV-LED lamps at 385 nm, 395 nm, and 405 nm. D contains a urethane group and an ethylenically unsaturated group in its molecule and thus the performance and effects due to such each group are further enhanced. In particular, D having an ethylenically unsaturated group is contained to allow the resulting cured product to be extremely low in content of the low-molecular-weight component, high in not only safety, but also adhesion with various materials, and also favorable in various physical properties such as surface hardness, light yellowing resistance, durability, and transparency. The benzoylformic acid amide derivative (D) of the present disclosure can be suitably used in an active energy ray-curable ink composition, an active energy ray-curable ink-jet ink composition, an active energy ray-curable flexographic ink composition, an active energy ray-curable offset ink composition, an active energy ray-curable screen ink composition, an active energy ray-curable nail cosmetic composition, an active energy ray-curable pressure-sensitive adhesive composition, an active energy ray-curable adhesive composition, an active energy ray-curable sealant composition, an active energy ray-curable coating agent composition, an active energy ray-curable decorative sheet resin composition, an active energy ray-curable elastomer composition, an active energy ray-curable three-dimensional modeling ink composition, an active energy ray-curable vehicular coating agent composition, an active energy ray-curable self-repairing-material resin composition, an active energy ray-curable architectural-paint composition, an active energy ray-curable composition used in fields of various coatings such as ship bottom paint, an anti-fogging material, and antifouling paint, an active energy ray-curable composition used in the medical device surface coating field, an active energy ray-curable dental material composition, an active energy ray-curing photosensitive composition, an active energy ray-curable hydrogel composition, and an active energy ray-curable water-dispersion composition. The resulting hydrogel composition and aqueous composition can also be each suitably used as a material for a wide variety of fields, for example, the health field of high water-absorption resins, paper diapers, soft contact lenses, and the like, the medical field of medical device surface coatings, artificial organs, and

the like, the civil engineering and construction field of soil amendments and the like, the agricultural field of water retention agents and the like, and the energy-absorbing material field.

## Claims

**1.** A benzoylformic acid amide derivative having a benzoylformic acid amide group represented by general formula (1):

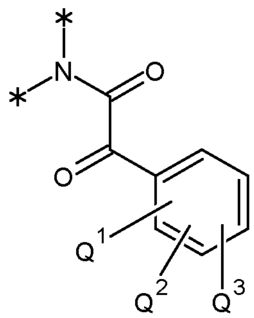

General formula (1)

$Q^1$ to $Q^3$ independently of each other represent a hydrogen atom, a substituent represented by formulas (Formula 2) to (Formula 8), a halogen group, or a nitrile group, and are each bound to any position of the 2-position to the 6-position,

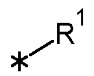

(Formula 2)

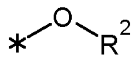

(Formula 3)

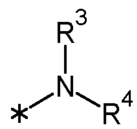

(Formula 4)

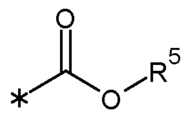

(Formula 5)

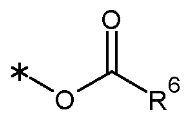

(Formula 6)

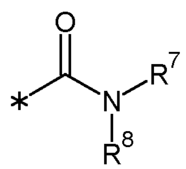

(Formula 7)

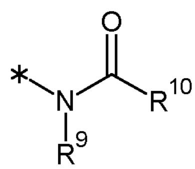

(Formula 8)

$R^1$ to $R^{10}$ each independently represent a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, or a cyclic alkenyl group having 3 to 18 carbon atoms, and

* is a binding position.

**2.** The benzoylformic acid amide derivative according to claim 1, wherein the benzoylformic acid amide derivative is at least one compound represented by any one general formula of general formula (2) to general formula (4):

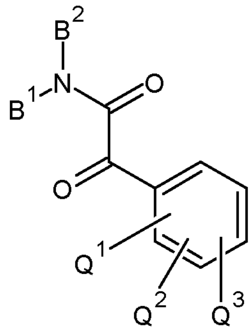

General formula (2)

wherein $Q^1$ to $Q^3$ have the same meanings as the definitions described in the general formula (1),

$B^1$ represents a hydrogen atom, or a monovalent organic group optionally having a hydroxyl group, an amino group, a thiol group, an ether group, a thioether group, an ester group, a carbonate group, a urethane group, a thiourethane group, a urea group, a siloxane group, an amide group, an imide group, an ethylenically unsaturated group or a benzoylformic acid amide group,
$B^2$ represents a monovalent organic group optionally having a hydroxyl group, an amino group, a thiol group, an ether group, a thioether group, an ester group, a carbonate group, a urethane group, a thiourethane group, a urea group, an amide group, an imide group, a siloxane group, an ethylenically unsaturated group or a benzoylformic acid amide group,

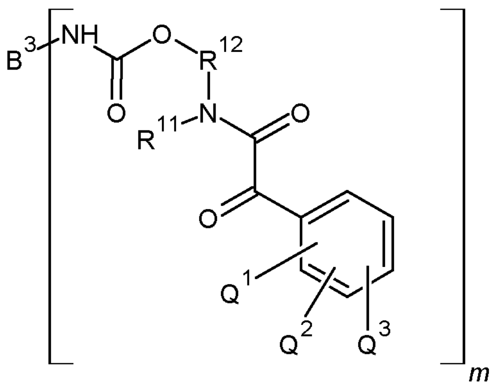

General formula (3)

wherein $Q^1$ to $Q^3$ have the same meanings as the definitions described in the general formula (1),
$B^3$ represents an m-valent organic group optionally having an ethylenically unsaturated group, an ether group, a thioether group, an ester group, a carbonate group, a urethane group, a thiourethane group, an isocyanurate group, an allophanate group, a urea group, a siloxane group, an amide group or an imide group,
$R^{11}$ represents a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, a cyclic alkenyl group having 3 to 18 carbon atoms, or an aryl group having 6 to 8 carbon atoms,
$R^{12}$ represents a linear saturated divalent hydrocarbon group having 1 to 18 carbon atoms, a linear unsaturated divalent hydrocarbon group having 2 to 18 carbon atoms, a branched saturated or unsaturated divalent hydrocarbon group having 3 to 18 carbon atoms, an alicyclic saturated or unsaturated divalent hydrocarbon group having 3 to 8 carbon atoms, a divalent aromatic hydrocarbon group having 6 to 8 carbon atoms, or a divalent

organic group formed by replacing at least one atom of carbon atoms or hydrogen atoms in any of these hydrocarbon groups by an oxygen atom, a nitrogen atom, a sulfur atom, a hydroxyl group, a thiol group, or an amine group, and
m represents an integer of 1 to 10,

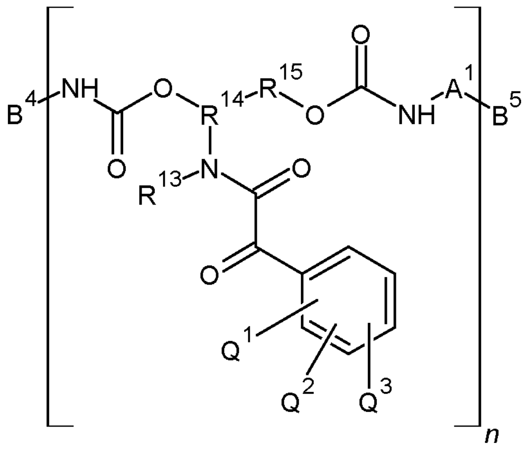

General formula (4)

wherein $Q^1$ to $Q^3$ have the same meanings as the definitions described in the general formula (1),
$A^1$ represents a divalent organic group optionally having an ether group, a thioether group, an ester group, a carbonate group, a urethane group, a thiourethane group, a urea group, a siloxane group, an amide group or an imide group,
$B^4$ and $B^5$ independently of each other optionally have an ethylenically unsaturated group, an ether group, a thioether group, an ester group, a carbonate group, a urethane group, a thiourethane group, an isocyanurate group, an allophanate group, a urea group,
a siloxane group, an amide group or an imide group, and any one or both of $B^4$ and $B^5$ represent a monovalent organic group having one or more ethylenically unsaturated bonds,
$R^{13}$ represents a hydrogen atom, a linear alkyl group having 1 to 18 carbon atoms, a linear alkenyl group having 2 to 18 carbon atoms, a branched alkyl group having 3 to 18 carbon atoms, a branched alkenyl group having 3 to 18 carbon atoms, a cyclic alkyl group having 3 to 18 carbon atoms, a cyclic alkenyl group having 3 to 18 carbon atoms, or an aryl group having 6 to 8 carbon atoms,
$R^{14}$ represents a linear saturated trivalent hydrocarbon group having 1 to 8 carbon atoms, a linear unsaturated trivalent hydrocarbon group having 2 to 8 carbon atoms, a branched saturated or unsaturated trivalent hydrocarbon group having 3 to 8 carbon atoms, an alicyclic saturated or unsaturated trivalent hydrocarbon group having 3 to 8 carbon atoms, a trivalent aromatic hydrocarbon group having 6 to 8 carbon atoms, or a trivalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group,
$R^{15}$ represents a linear saturated divalent hydrocarbon group having 1 to 18 carbon atoms, a linear unsaturated divalent hydrocarbon group having 2 to 18 carbon atoms, a branched saturated or unsaturated divalent hydrocarbon group having 3 to 8 carbon atoms, an alicyclic saturated or unsaturated divalent hydrocarbon group having 3 to 8 carbon atoms, a divalent aromatic hydrocarbon group having 6 to 8 carbon atoms, or a divalent organic group formed by replacing at least one atom of carbon atoms or hydrogen atoms in any of these hydrocarbon groups by an oxygen atom, a nitrogen atom, a sulfur atom, a hydroxyl group, a thiol group, or an amine group, and
n represents an integer of 1 to 100.

**3.** The benzoylformic acid amide derivative according to claim 1 or claim 2, having one or more ethylenically unsaturated bonds in a molecule, wherein the ethylenically unsaturated bonds are included in one or more groups selected from a (meth)acrylate group, a (meth)acrylamide group, a vinyl group, a vinyl ether group, an alkyl vinyl ether group, an allyl group, a (meth)allyl ether group, a styryl group and a maleimide group.

**4.** The benzoylformic acid amide derivative according to any one of claims 1 to 3, having one or more urethane groups in a molecule, wherein the number of atoms directly linking a nitrogen atom of the benzoylformic acid amide group and a nitrogen atom of a proximate urethane group is 3 to 20.

5. The benzoylformic acid amide derivative according to any one of claims 1 to 4, wherein the benzoylformic acid amide derivative is a photopolymerization initiator.

6. The benzoylformic acid amide derivative according to any one of claims 1 to 4, wherein the benzoylformic acid amide derivative is a photosensitizer of photoradical polymerization and/or a photosensitizer of photoionic polymerization.

7. An active energy ray-curable composition comprising the benzoylformic acid amide derivative according to any one of claims 1 to 6.

8. An active energy ray-curable ink composition comprising the benzoylformic acid amide derivative according to any one of claims 1 to 6.

9. An active energy ray-curable pressure-sensitive adhesive composition comprising the benzoylformic acid amide derivative according to any one of claims 1 to 6.

10. An active energy ray-curable adhesive composition comprising the benzoylformic acid amide derivative according to any one of claims 1 to 6.

11. An active energy ray-curable sealant composition comprising the benzoylformic acid amide derivative according to any one of claims 1 to 6.

12. An active energy ray-curing photosensitive composition comprising the benzoylformic acid amide derivative according to any one of claims 1 to 6.

13. An active energy ray-curable nail cosmetic composition comprising the benzoylformic acid amide derivative according to any one of claims 1 to 6.

14. An active energy ray-curable dental material composition comprising the benzoylformic acid amide derivative according to any one of claims 1 to 6.

15. An active energy ray-curable coating agent composition comprising the benzoylformic acid amide derivative according to any one of claims 1 to 6.

16. An active energy ray-curable aqueous composition comprising the benzoylformic acid amide derivative according to any one of claims 1 to 6.

## INTERNATIONAL SEARCH REPORT

International application No.
**PCT/JP2024/013398**

### A. CLASSIFICATION OF SUBJECT MATTER

***C07C 235/78***(2006.01)i; ***C07D 251/34***(2006.01)i; ***C07D 403/14***(2006.01)i; ***C08F 2/50***(2006.01)i; ***C08F 4/00***(2006.01)i; ***C08F 20/60***(2006.01)i; ***C08G 65/10***(2006.01)i; ***C09D 7/63***(2018.01)i; ***C09D 11/38***(2014.01)i; ***C09D 201/00***(2006.01)i; ***C09J 11/06***(2006.01)i; ***C09J 201/00***(2006.01)i; ***G03F 7/031***(2006.01)i
FI: C07C235/78 CSP; C08F20/60; C09D201/00; C09D7/63; C09D11/38; C09J201/00; C09J11/06; C07D403/14; C07D251/34 E; G03F7/031; C08F4/00; C08G65/10; C08F2/50

According to International Patent Classification (IPC) or to both national classification and IPC

### B. FIELDS SEARCHED

Minimum documentation searched (classification system followed by classification symbols)

C07C235/78; C07D251/34; C07D403/14; C08F2/50; C08F4/00; C08F20/60; C08G65/10; C09D7/63; C09D11/38; C09D201/00; C09J11/06; C09J201/00; G03F7/031

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | US 4118298 A (STAUFFER CHEMICAL COMPANY) 03 October 1978 (1978-10-03) claims, examples, columns 3, 6 | 1, 2, 5-16 |
| X | JP 10-182781 A (BASF AKTIENGESELLSCHAFT) 07 July 1998 (1998-07-07) claims, examples, paragraph [0126] | 1, 2, 5-16 |
| X | HE, Minghui et al. Benzoylformamides as new photocaged bases for photo-latent anion polymerization. CHINESE CHEMICAL LETTERS. 2015, 26(1), 21-25 p. 21, abstract, p. 22, scheme 1 | 1, 2, 5-16 |
| X | CN 101735133 A (CHINESE ACAD INST CHEMISTRY) 16 June 2010 (2010-06-16) paragraph [0016], examples | 1-3 |
| X | CN 104892547 A (UNIV NANJING NORMAL) 09 September 2015 (2015-09-09) examples | 1-3 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 June 2024** | **25 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/013398**

**C. DOCUMENTS CONSIDERED TO BE RELEVANT**

| Category* | Citation of document, with indication, where appropriate, of the relevant passages | Relevant to claim No. |
|---|---|---|
| X | JP 2008-523987 A (CIBA HOLDING INC.) 10 July 2008 (2008-07-10) examples 19, 20 | 1-3 |
| X | US 2020/0255578 A1 (COVESTRO LLC) 13 August 2020 (2020-08-13) pp. 23-25 | 1, 2, 4 |
| X | JP 2002-529449 A (BRISTOL-MYERS SQUIBB COMPANY) 10 September 2002 (2002-09-10) examples 7-21 | 1, 2, 4 |

Form PCT/ISA/210 (second sheet) (July 2022)

**INTERNATIONAL SEARCH REPORT**
**Information on patent family members**

International application No.
**PCT/JP2024/013398**

| Patent document cited in search report | Publication date (day/month/year) | Patent family member(s) | Publication date (day/month/year) |
|---|---|---|---|
| US 4118298 A | 03 October 1978 | (Family: none) | |
| JP 10-182781 A | 07 July 1998 | US 6031044 A<br>claims, examples<br>EP 849300 A1<br>KR 10-1998-0064354 A<br>CA 2222922 A1 | |
| CN 101735133 A | 16 June 2010 | (Family: none) | |
| CN 104892547 A | 09 September 2015 | (Family: none) | |
| JP 2008-523987 A | 10 July 2008 | US 2009/0092768 A1<br>examples 19, 20<br>WO 2006/067061 A2<br>EP 1836002 A2<br>TW 200632057 A | |
| US 2020/0255578 A1 | 13 August 2020 | (Family: none) | |
| JP 2002-529449 A | 10 September 2002 | US 6228872 B1<br>examples 7-21<br>WO 2000/027811 A1<br>EP 1129070 A1 | |

Form PCT/ISA/210 (patent family annex) (July 2022)